# EUROPEAN PATENT APPLICATION

(11) **EP 4 571 309 A1**
(43) Date of publication of application: **18.06.2025**
(21) Application number: 23852568.7
(22) Date of filing: 08.08.2023
(51) Int. Cl.: G01N 33/50, A61K 45/00, A61P 35/00, G01N 33/92

(54) **SAMPLE TEST METHOD, TEST KIT FOR GYNECOLOGICAL CANCER AND PRECANCEROUS LESION THEREOF, AND MEDICINE**

(30) Priority: 10.08.2022 JP 2022128403; 21.02.2023 JP 2023025389
(71) Applicant: Nihon University, Tokyo 102-8275 (JP); Keio University, Tokyo, 108-8345 (JP)
(72) Inventor: KATOH Yuki, Tokyo 102-8275 (JP); KAWANA Kei, Tokyo 102-8275 (JP); KUBO Akiko, Tokyo 160-8582 (JP)
(74) Representative: Eisenführ Speiser
(86) International application number: PCT/JP2023/028921
(87) International publication number: WO 2024/034606

(57) **Abstract**

Provided are a simple and highly accurate sample test method, which is capable of being applied to an evaluation of the presence or absence of one or more selected from the group consisting of a gynecological cancer and a precancerous lesion thereof, an evaluation of prognosis of a gynecological cancer patient, or an evaluation of a degree of malignancy of the gynecological cancer, a test kit capable of being used for the test method, and a medicine for treating or preventing a gynecological cancer, which is to be administered to a subject detected by the test method. A sample test method including a step (A) of measuring a concentration of free fatty acids in a sample derived from a subject and a step (B) of evaluating a possibility that the subject has a gynecological cancer based on the concentration of the free fatty acids obtained in the step (A) is employed.

## Description

### TECHNICAL FIELD

The present invention relates to a sample test method, a test kit for a gynecological cancer and a precancerous lesion thereof, and a medicine. Priority is claimed on Japanese Patent Application No. 2022-128403, filed August 10, 2022 and Japanese Patent Application No. 2023-025389, filed February 21, 2023, the contents of which are incorporated herein by reference.

### BACKGROUND ART

Since ovarian cancer is a tumor that occurs in the abdominal cavity, it is difficult to exhibit symptoms and it cannot be pathologically diagnosed by biopsy, and thus it is extremely difficult to detect ovarian cancer at an early stage. In addition, there is no established serum tumor marker with which other gynecological cancers such as endometrial cancer and cervical cancer can be also diagnosed at an early stage, and currently, there are many cases in which these cancers are accidentally found because of irregular bleeding or the like.

As an ovarian cancer tumor marker, for example, CA125 is known (for example, see Patent Document 1). However, the blood concentration of CA125 is not always high in the early ovarian cancer, the mucinous ovarian cancer, and the ovarian clear cell cancer, and thus these cancers may not be determined to be positive. In addition, since the blood concentration of CA125 may be also high in benign diseases and inflammatory diseases, the determination based on the blood concentration of CA125 has a high false positive rate. Therefore, CA125 is insufficient as a tumor marker for ovarian cancer.

In recent years, as a cancer diagnosis method, a method of detecting a specific exosome or microRNA in peripheral blood has been proposed (for example, see Non Patent Document 1). However, in a case of analyzing an exosome and microRNA from a biological sample, operations such as ultracentrifugation and affinity separation are required. Therefore, the test using these as tumor markers is complicated and takes time. In addition, there is also an object that a certain number of false positives exist, and thus the practical application has not been achieved.

### Citation List

### Patent Document

Patent Document 1: Japanese Patent No. 6538887

### Non Patent Document

Non Patent Document 1: Yu Zhang et al., Emerging Functions and Clinical Applications of Exosomal ncRNAs in Ovarian Cancer. Front. Oncol., 05 November 2021, https://doi.org/10.3389/fonc.2021.765458

### SUMMARY OF INVENTION

### Technical Problem

As described above, the conventional cancer screening method has objects in terms of simplicity and accuracy. Therefore, there is a demand for a method capable of accurately detecting a gynecological cancer at an early stage by a simple method.

Therefore, an object of the present invention is to provide a simple and highly accurate sample test method, which is capable of being applied to an evaluation of the presence or absence of one or more selected from the group consisting of a gynecological cancer and a precancerous lesion thereof, an evaluation of prognosis of a gynecological cancer patient, or an evaluation of a degree of malignancy of the gynecological cancer, a test kit capable of being used for the test method, and a medicine for treating or preventing a gynecological cancer, which is to be administered to a subject detected by the test method.

### Solution to Problem

The present invention includes the following aspects.
[1] A sample test method including a step (A1) of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid, in a sample derived from a subject and a step (B 1) of evaluating a possibility that the subject has one or more selected from the group consisting of a gynecological cancer and a precancerous lesion of the gynecological cancer, based on the concentration of the free fatty acids in the sample, which is obtained in the step (A1), in which in the step (B1), the possibility that the subject has one or more selected from the group consisting of a gynecological cancer and a precancerous lesion of the gynecological cancer is evaluated to be high in a case corresponding to at least one selected from the group consisting of the following (i1), (ii1), and (iii1),
   (i1) a concentration of at least one free fatty acid selected from the group consisting of palmitoleic acid, linoleic acid, α-linolenic acid, oleic acid, palmitic acid, and arachidonic acid in the sample is high as compared with a predetermined threshold value,
   (ii1) a concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value, and
   (iii1) a concentration ratio of docosapentaenoic acid to oleic acid is low as compared with a predetermined threshold value.
[2] A sample test method including a step (A2) of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid, in a sample derived from a subject having a gynecological cancer and a step (B2) of evaluating a possibility that the subject has poor prognosis based on the concentration of the free fatty acids in the sample, which is obtained in the step (A2), in which in the step (B2), the possibility that the subject has poor prognosis is evaluated to be high in a case corresponding to at least one selected from the group consisting of the following (i2) and (ii2),
   (i2) a concentration of at least one free fatty acid selected from the group consisting of palmitoleic acid, linoleic acid, α-linolenic acid, oleic acid, and palmitic acid in the sample is high as compared with a predetermined threshold value, and
   (ii2) a concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.
[3] A sample test method including a step (A3) of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid, in a sample derived from a subject having a gynecological cancer and a step (B3) of evaluating whether the gynecological cancer of the subject is a malignant tumor or a borderline malignant tumor based on the concentration of the free fatty acids in the sample, which is obtained in the step (A3), in which in the step (B3), a possibility that the gynecological cancer is a malignant tumor is evaluated to be high in a case corresponding to at least one selected from the group consisting of the following (i3) and (ii3),
   (i3) a concentration of at least one free fatty acid selected from the group consisting of palmitoleic acid, linoleic acid, α-linolenic acid, oleic acid, and palmitic acid in the sample is high as compared with a predetermined threshold value, and
   (ii3) a concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.
[4] The sample test method according to [1], further including a step (C1) of evaluating whether the subject determined to have a high possibility of having one or more selected from the group consisting of a gynecological cancer and a gynecological precancerous lesion by the step (B 1) has any of a gynecological cancer or a gynecological cancer precancerous lesion based on the concentration of the free fatty acid in the sample, which is obtained in the step (A1), in which in the step (C1), a possibility that the subject has a gynecological cancer is evaluated to be high in a case corresponding to the following (Ci),
   (Ci) a concentration of at least one free fatty acid selected from the group consisting of dihomo-γ-linolenic acid, docosahexaenoic acid, adrenic acid, and docosapentaenoic acid in the sample is low as compared with a predetermined threshold value.
[5] The sample test method according to any one of [1] to [4], in which the gynecological cancer includes at least one selected from the group consisting of ovarian cancer, endometrial cancer, and cervical cancer.
[6] The sample test method according to [1], in which the gynecological cancer is a Stage I or Stage II gynecological malignant tumor.
[7] The sample test method according to [6], in which the step (A1) is a step of measuring a concentration of one or more free fatty acids selected from the group consisting of palmitoleic acid, linoleic acid, α-linolenic acid, oleic acid, palmitic acid, vaccenic acid, arachidic acid, and stearic acid, and the free fatty acid in (i1) of the step (B 1) is at least one selected from the group consisting of palmitoleic acid, linoleic acid, α-linolenic acid, oleic acid, and palmitic acid, and the free fatty acid in (ii1) of the step (B1) is at least one selected from the group consisting of vaccenic acid, arachidic acid, and stearic acid.
[8] The sample test method according to any one of [1] to [4], in which the sample is a blood sample.
[9] A test kit for a gynecological cancer and a precancerous lesion of the gynecological cancer, the test kit including a reagent for measuring one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid, in which the test kit is used for at least one purpose selected from the group consisting of the following (a) to (d),
   (a) evaluation of a possibility that a subject has one or more selected from the consisting of a gynecological cancer and a precancerous lesion of the gynecological cancer,
   (b) evaluation of a possibility that a subject having a gynecological cancer has poor prognosis,
   (c) evaluation of whether a gynecological cancer of a subject is a malignant tumor or a borderline malignant tumor, and
   (d) evaluation of whether a subject having a high possibility of having one or more selected from the group consisting of a gynecological cancer and a gynecological precancerous lesion has any of a gynecological cancer and a gynecological precancerous lesion.
[10] The test kit for a gynecological cancer according to [9], in which the test kit is used in the sample test method according to any one of [1] to [4].
[11] A medicine for treating or preventing a gynecological cancer, which is administered to a subject evaluated to have a gynecological cancer by the test method according to [1].

### Advantageous Effects of Invention

According to the present invention, it is possible to provide a simple and highly accurate sample test method, which is capable of being applied to an evaluation of the presence or absence of a gynecological cancer, an evaluation of prognosis of a gynecological cancer patient, or an evaluation of a degree of malignancy of the gynecological cancer, a test kit capable of being used for the test method, and a medicine for treating or preventing a gynecological cancer, which is to be administered to a subject specified by the test method.

### BRIEF DESCRIPTION OF DRAWINGS

[FIG. 1A] A diagram showing the analysis results of the expression levels of the fatty acid metabolic enzymes in normal ovarian tissue (Normal tissue) and ovarian cancer tissue (OV tumor tissue) in Experimental Example 1 by a heat map.
[FIG. 1B] A graph showing the analysis results of the expression levels of FASN and SCD1 in normal ovarian tissue (NT) and ovarian cancer tissue (Tu) in Experimental Example 1.
[FIG. 1C] A graph showing the analysis results of the expression levels of ELOVL1, ELOVL2, ELOVL3, ELOVL4, ELOVL5, ELOVL6, and FADS1 in normal ovarian tissue (NT) and ovarian cancer tissue (Tu) in Experimental Example 1.
[FIG. 1D] A graph showing the analysis results of the expression levels of ACACA and FADS2 in normal ovarian tissue (NT) and ovarian cancer tissue (Tu) in Experimental Example 1.
[FIG. 2A] A diagram showing the analysis results of the free fatty acid concentrations in the blood serum of a healthy female subject (Healthy donor) and an ovarian cancer patient (OV ca patients) in Experimental Example 2 by a heat map.
[FIG. 2B] A graph showing the analysis results of the concentrations of palmitoleic acid, palmitic acid, γ-linolenic acid, stearidonic acid, linoleic acid, α-linolenic acid, oleic acid, vaccenic acid, and stearic acid in the blood serum of a healthy female subject (HD) and an ovarian cancer patient (OV) in Experimental Example 2.
[FIG. 2C] A graph showing the analysis results of the concentrations of arachidic acid, arachidonic acid, eicosapentaenoic acid, dihomo-γ-linolenic acid, docosahexaenoic acid, adrenic acid, docosapentaenoic acid, nervonic acid, and lignoceric acid in the blood serum of a healthy female subject (HD) and an ovarian cancer patient (OV) in Experimental Example 2.
[FIG. 2D] A diagram showing the analysis results of the free fatty acid concentration in the blood serum of an ovarian cancer patient in Experimental Example 2 by a volcano plot. In the figure, "OV ca" and "healthy" represent an ovarian cancer patient and a healthy female subject.
[FIG. 3A] A diagram showing a correlation between a change in expression of a fatty acid metabolic enzyme in Experimental Example 1 and a change in a fatty acid composition in Experimental Example 2.
[FIG. 3B] A diagram showing a correlation between a change in expression of a fatty acid metabolic enzyme in Experimental Example 1 and a change in a fatty acid composition in Experimental Example 2.
[FIG. 3C] A graph showing a correlation between the expression level of the SCD1 gene in the ovarian tumor tissue and the fatty acid ratio (ratio of produced fatty acid/substrate fatty acid (ratio of palmitoleic acid/palmitic acid)) corresponding to the SCD1 activity in the blood serum of the ovarian cancer patient in Experimental Example 3.
[FIG. 3D] A graph showing a correlation between the expression level of the SCD1 gene in the ovarian tumor tissue and the fatty acid ratio (ratio of produced fatty acid/substrate fatty acid (ratio of oleic acid/stearic acid)) corresponding to the SCD1 activity in the blood serum of the ovarian cancer patient in Experimental Example 3.
[FIG. 4A] A diagram showing the ROC analysis results based on concentrations of palmitoleic acid, linoleic acid, α-linolenic acid, palmitic acid, and oleic acid in the blood serum for a healthy female subject and an ovarian cancer patient in Experimental Example 4.
[FIG. 4B] A diagram showing the ROC analysis results based on concentrations of vaccenic acid, arachidic acid, docosahexaenoic acid, adrenic acid, nervonic acid, and lignoceric acid in the blood serum for a healthy female subject and an ovarian cancer patient in Experimental Example 4.
[FIG. 5A] A diagram showing a result of determining whether or not concentrations of ten types of free fatty acids in the blood serum are abnormal values for a healthy female subject in Experimental Example 5 based on a threshold value.
[FIG. 5B] A diagram showing a result of determining whether or not concentrations of ten types of free fatty acids in the blood serum are abnormal values for an ovarian malignant tumor patient in Experimental Example 5 based on a threshold value.
[FIG. 5C] A graph showing the ROC analysis result based on the FFA score for a healthy female subject and an ovarian malignant tumor patient in Experimental Example 5.
[FIG. 6A] A diagram showing the analysis results of concentrations of palmitoleic acid, linoleic acid, α-linolenic acid, and oleic acid in the blood serum for a healthy female subject (HD), Stages I and II ovarian cancer patients, and Stages III and IV ovarian cancer patients in Experimental Example 6.
[FIG. 6B] A diagram showing the analysis results of concentrations of vaccenic acid, arachidic acid, docosahexaenoic acid, and lignoceric acid in the blood serum for a healthy female subject (HD), Stages I and II ovarian cancer patients, and Stages III and IV ovarian cancer patients in Experimental Example 6.
[FIG. 7A] A diagram showing the ROC analysis results based on concentrations of palmitoleic acid, linoleic acid, α-linolenic acid, and oleic acid in the blood serum for a healthy female subject and a Stage I or Stage II ovarian cancer patient in Experimental Example 7.
[FIG. 7B] A diagram showing the ROC analysis results based on concentrations of vaccenic acid, arachidic acid, docosahexaenoic acid, and lignoceric acid in the blood serum for a healthy female subject and a Stage I or Stage II ovarian cancer patient in Experimental Example 7.
[FIG. 8A] A diagram showing a result of determining whether or not concentrations of eight types of free fatty acids in the blood serum are abnormal values for a healthy female subject in Experimental Example 8 based on a threshold value.
[FIG. 8B] A diagram showing a result of determining whether or not concentrations of eight types of free fatty acids in the blood serum are abnormal values for an ovarian cancer patient in Experimental Example 8 based on a threshold value.
[FIG. 8C] A diagram showing an ROC analysis result by FFA score for a healthy female subject (HD) and early ovarian cancer patients (Stage I and II) in Experimental Example 8.
[FIG. 8D] A diagram showing the distribution of the FFA score and the blood concentration of CA125 of early ovarian cancer patients (Stage I and II) in Experimental Example 8. In the figure, "Clear cell" and "Others" represent ovarian clear cell cancer patients and ovarian cancer patients with tissue types other than clear cell cancer.
[FIG. 9] A diagram showing the transition of progression-free survival (PFS) of the FFA score high group and the FFA score low group in Experimental Example 9.
[FIG. 10A] A diagram showing a result of determining whether or not concentrations of ten types of free fatty acids in the blood serum are abnormal values for an ovarian borderline malignant tumor patient in Experimental Example 10 based on a threshold value.
[FIG. 10B] A diagram showing an ROC analysis result based on FFA score for ovarian borderline malignant tumor patients and ovarian cancer patients (ovarian malignant tumor patients) in Experimental Example 10.
[FIG. 11A] A graph showing the analysis results of the concentrations of palmitoleic acid, palmitic acid, linoleic acid, α-linolenic acid, and oleic acid in the blood serum of a healthy female subject, an endometrial cancer patient, and a cervical cancer patient in Experimental Example 11.
[FIG. 11B] A graph showing the analysis results of the concentrations of vaccenic acid, arachidic acid, and stearic acid in the blood serum of a healthy female subject, an endometrial cancer patient, and a cervical cancer patient in Experimental Example 11.
[FIG. 12A] A diagram showing the analysis results of the concentrations of free fatty acids such as palmitoleic acid, linoleic acid, α-linolenic acid, and oleic acid in blood serum and the ROC analysis results based on the blood serum concentration for a healthy female subject and a Stage I or Stage II or III cervical cancer patient in Experimental Example 12.
[FIG. 12B] A diagram showing the analysis results of the concentrations of free fatty acids such as vaccenic acid, arachidic acid, dihomo-γ-linolenic acid, and docosahexaenoic acid in blood serum and the ROC analysis results based on the blood serum concentration for a healthy female subject and a Stage I or Stage II or III cervical cancer patient in Experimental Example 12.
[FIG. 12C] A diagram showing the analysis results of the concentrations of free fatty acids such as adrenic acid and docosapentaenoic acid in blood serum and the ROC analysis results based on the blood serum concentration for a healthy female subject and a Stage I or Stage II or III cervical cancer patient in Experimental Example 12.
[FIG. 13A] A diagram showing the analysis results of the concentration ratio (docosapentaenoic acid/oleic acid) in blood serum and the ROC analysis results for a healthy female subject and a Stage I or Stage II or III cervical cancer patient in Experimental Example 13.
[FIG. 13B] A diagram showing a distribution of a concentration ratio (docosapentaenoic acid/oleic acid), an SCC marker, and a CEA blood concentration for a healthy female subject and a Stage I or Stage II or III cervical cancer patient in Experimental Example 13.
[FIG. 14A] A diagram showing the analysis results of the concentrations of free fatty acids such as palmitoleic acid, linoleic acid, α-linolenic acid, and oleic acid in blood serum and the ROC analysis results based on the blood serum concentration for a healthy female subject and a cervical precancerous lesion patient in Experimental Example 14.
[FIG. 14B] A diagram showing the analysis results of the concentrations of free fatty acids such as arachidonic acid and arachidic acid in blood serum and the ROC analysis results based on the blood serum concentration for a healthy female subject and a cervical precancerous lesion patient in Experimental Example 14.
[FIG. 15] A diagram showing the analysis results of the concentrations of free fatty acids such as dihomo-γ-linolenic acid, docosahexaenoic acid, adrenic acid, and docosapentaenoic acid in blood serum and the ROC analysis results based on the concentration in the blood serum for a cervical precancerous lesion (CIN) patient and a cervical cancer patient in Experimental Example 15.

### DESCRIPTION OF EMBODIMENTS

### [Sample test method (1)]

The sample test method according to the first aspect can be used for evaluating the possibility that the subject has a gynecological cancer. The test method includes the following step (A1) and step (B1).

The step (A1): a step of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid in a sample derived from a subject

The step (B1): a step of evaluating the possibility that the subject has one or more selected from the group consisting of a gynecological cancer and a precancerous lesion thereof, based on the concentration of the free fatty acid in the sample, which is obtained in the step (A1)

The "subject" from which the sample to be subjected to test is derived is a human female. In the present specification, the gynecological cancer means a cancer that is not suffered by a human male but can be suffered by a human female. In other words, the gynecological cancer means a cancer that can occur in a female-specific organ (an organ that a human male does not have and a human female has). Examples of the female-specific organ include an ovary, a uterus, and the like.

Examples of the gynecological cancer include ovarian cancer, endometrial cancer, and cervical cancer. In the present specification, the term "cancer" includes a malignant tumor and a borderline malignant tumor. The ovarian cancer may be an ovarian malignant tumor or may be an ovarian borderline malignant tumor. The endometrial cancer may be a corpus uteri malignant tumor or a corpus uteri borderline malignant tumor. The cervical cancer may be a uterine cervix malignant tumor or a uterine cervix borderline malignant tumor. The gynecological cancer detected by the test method according to the present embodiment is preferably a malignant tumor, and more preferably an ovarian malignant tumor, a corpus uteri malignant tumor, or a uterine cervix malignant tumor. Hereinafter, the malignant tumor in gynecological cancer is also referred to as a "gynecological malignant tumor". The "borderline malignant tumor" in the gynecological cancer is also referred to as a "gynecological borderline malignant tumor".

The stage of the gynecological cancer to be detected by the test method according to the present embodiment is not particularly limited. It is noted that the cancer stage described in the present specification is based on the TNM classification. In the test method according to the present embodiment, it is possible to widely detect a cancer from an early gynecological cancer (for example, Stage I or II) to a cancer at an advanced stage (for example, Stage III or IV). The gynecological cancer to be detected may be an early gynecological malignant tumor. The early gynecological malignant tumor means a Stage 0, Stage I, or Stage II gynecological malignant tumor. The early gynecological malignant tumor is preferably a Stage I or Stage II malignant tumor. As the early gynecological malignant tumor to be detected, an early ovarian malignant tumor is preferable. The gynecological cancer to be detected may be a Stage III or Stage IV malignant tumor. The gynecological cancer to be detected by the test method according to the present embodiment may be a gynecological malignant tumor having a degree of progress of Stage I or higher. The gynecological malignant tumor having a degree of progress of Stage I or more includes a Stage I gynecological malignant tumor, a Stage II gynecological malignant tumor, a Stage III gynecological malignant tumor, and a Stage IV gynecological malignant tumor.

In the present specification, the term "precancerous lesion" means a tissue that has changed to a state in which cancer is likely to occur as compared with a normal tissue. That is, the "precancerous lesion" is a tissue that has a high probability of developing into cancer. The "precancerous lesion of gynecological cancer" means a tissue that is highly likely to progress to the "gynecological cancer". The precancerous lesion is also called an intraepithelial tumor.

### (Step (A1))

In the step (A1), a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid in a sample derived from a subject is measured. The measurement may be performed in vitro.

Hereinafter, palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid are collectively referred to as "free fatty acids of group A1".

The sample derived from the subject is not particularly limited as long as the effect of the present invention is exhibited, examples thereof include a biological sample such as urine, blood, saliva, sweat, and a tissue piece, and the blood is preferable. Examples of the blood sample include whole blood, blood plasma, blood serum, and the like.

A method of measuring the concentration of the free fatty acid in the sample is not particularly limited. For example, the concentration of the free fatty acid in the sample can be measured using a method known to those skilled in the art. Examples of such a method include gas chromatography mass spectrometry, liquid chromatography mass spectrometry, gas chromatography, and the like.

In the step (A1), the number of types of free fatty acids to be measured is only required to be 1 or more selected from the free fatty acids of the group A1, and may be 2 or more, may be 3 or more, may be 4 or more, may be 5 or more, may be 6 or more, may be 7 or more, may be 8 or more, may be 9 or more, may be 10 or more, may be 11 or more, may be 12 or more, may be 13 or more, may be 14 or more, or may be 15 selected from the free fatty acids of the group A1. The free fatty acid to be measured can be arbitrarily selected from the free fatty acids of the group A1.

The free fatty acid to be measured may be one or more (for example, two or more, three or more, four or more, five or more, six or more, seven or more, eight or more, nine or more, ten or more, eleven or more, twelve or more, thirteen or more, or fourteen) selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid.

Alternatively, the free fatty acid to be measured may be one or more (for example, two or more, three or more, four or more, five or more, six or more, seven or more, eight or more, nine or more, or ten) selected from the group consisting of palmitoleic acid, linoleic acid, α-linolenic acid, oleic acid, vaccenic acid, arachidic acid, docosahexaenoic acid, adrenic acid, nervonic acid, and lignoceric acid.

Alternatively, the free fatty acid to be measured may be one or more (for example, two or more, three or more, four or more, five or more, six or more, seven or more, or eight) selected from the group consisting of palmitoleic acid, linoleic acid, α-linolenic acid, oleic acid, vaccenic acid, arachidic acid, docosahexaenoic acid, and lignoceric acid.

Alternatively, the free fatty acid to be measured may be one or more (for example, two or more, three or more, four or more, five or more, six or more, seven or more, eight or more, nine or more, ten or more, eleven or more, or twelve) selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid.

Alternatively, the free fatty acid to be measured may be one or more (for example, two or more, three or more, four or more, five or more, six or more, seven or more, or eight) selected from the group consisting of palmitic acid, palmitoleic acid, linoleic acid, α-linolenic acid, oleic acid, vaccenic acid, stearic acid, and arachidic acid.

Alternatively, the free fatty acid to be measured may be one or more (for example, two or more, three or more, four or more, five or more, six or more, seven or more, eight or more, nine or more, or ten) selected from the group consisting of palmitoleic acid, linoleic acid, α-linolenic acid, oleic acid, vaccenic acid, arachidic acid, dihomo-γ-linolenic acid, docosahexaenoic acid, adrenic acid, and docosapentaenoic acid.

Alternatively, the free fatty acid to be measured may be one or more (for example, two or more, three or more, four or more, five or more, or six) selected from the group consisting of palmitoleic acid, linoleic acid, α-linolenic acid, oleic acid, arachidonic acid, and arachidic acid.

In a case where a gynecological cancer is to be detected, in the step (A1), one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid may be to be measured. Among the free fatty acids, two or more types, three or more types, four or more types, five or more types, six or more types, seven or more types, eight or more types, nine or more types, ten or more types, eleven or more types, twelve or more types, thirteen or more types, or fourteen or more types of the free fatty acids can be measured. The free fatty acid to be measured can be arbitrarily selected from these fourteen types of fatty acids. In a case where the gynecological cancer is to be detected, it is preferable that, in the step (A1), the concentrations of the fourteen types of free fatty acids are measured.

In a case where the gynecological cancer which may be to be detected is ovarian cancer, in the step (A1), one or more types of free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid may be to be detected. Among the free fatty acids, two or more types, three or more types, four or more types, five or more types, six or more types, seven or more types, eight or more types, nine or more types, or ten types of the free fatty acids can be measured. The free fatty acid to be measured can be arbitrarily selected from these ten types of fatty acids. In a case where the gynecological cancer is ovarian cancer, it is preferable that, in the step (A1), the concentrations of the ten types of free fatty acids are measured.

To increase the detection sensitivity of early gynecological malignant tumors (for example, Stage I and II), in the step (A1), one or more free fatty acids selected from the group consisting of palmitoleic acid, linoleic acid, α-linolenic acid, oleic acid, vaccenic acid, arachidic acid, docosahexaenoic acid, and lignoceric acid may be to be measured. Among the free fatty acids, two or more types, three or more types, four or more types, five or more types, six or more types, seven or more types, or eight types of the free fatty acids can be measured. The free fatty acid to be measured can be arbitrarily selected from these eight types of fatty acids. In a case of intending to detect an early gynecological malignant tumor, it is preferable that, in the step (A1), concentrations of the eight types of free fatty acids are measured. Examples of the early gynecological malignant tumor include an early ovarian malignant tumor.

In a case where the gynecological cancer which may be to be detected is endometrial cancer or cervical cancer, in the step (A1), one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid may be to be measured. Among the free fatty acids, two or more types, three or more types, four or more types, five or more types, six or more types, seven or more types, eight or more types, nine or more types, ten or more types, eleven or more types, or twelve types of the free fatty acids can be measured. The free fatty acid to be measured can be arbitrarily selected from these twelve types of fatty acids. In a case where the gynecological cancer is endometrial cancer or cervical cancer, it is preferable that, in the step (A1), the concentrations of the twelve types of free fatty acids are measured.

In a case where the gynecological cancer which may be to be detected is endometrial cancer, in the step (A1), one or more free fatty acids selected from the group consisting of palmitic acid, palmitoleic acid, linoleic acid, α-linolenic acid, oleic acid, vaccenic acid, stearic acid, and arachidic acid may be to be measured. Among the free fatty acids, two or more types, three or more types, four or more types, five or more types, six or more types, seven or more types, or eight types of the free fatty acids can be measured. The free fatty acid to be measured can be arbitrarily selected from these eight types of fatty acids. In a case where the gynecological cancer is endometrial cancer, it is preferable that, in the step (A1), the concentrations of the eight types of free fatty acids are measured.

In a case where the gynecological cancer which may be to be detected is cervical cancer, in the step (A1), one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid may be to be measured. Among the free fatty acids, two or more types, three or more types, four or more types, five or more types, six or more types, seven or more types, eight or more types, nine or more types, ten or more types, eleven or more types, or twelve types of the free fatty acids can be measured. The free fatty acid to be measured can be arbitrarily selected from these twelve types of fatty acids. In a case where the gynecological cancer is cervical cancer, it is preferable that, in the step (A1), the concentrations of the twelve types of free fatty acids are measured.

In a case of intending to detect, including a gynecological precancerous lesion (for example, a cervical precancerous lesion), in the step (A1), as the free fatty acid to be used for the evaluation, one or more free fatty acids selected from the group consisting of palmitoleic acid, linoleic acid, α-linolenic acid, oleic acid, arachidonic acid, and arachidic acid may be to be measured. Among the free fatty acids, two or more types, three or more types, four or more types, five or more types, or six types of the free fatty acids can be measured.

### (Step (B1))

In the step (B1), the possibility that the subject has a gynecological cancer is evaluated based on the concentration of the free fatty acid in the sample, which is obtained in the step (A1).

In the step (B1), in a case corresponding to at least one selected from the group consisting of the following (i1) and (ii1), the possibility that the subject has one or more selected from the group consisting of a gynecological cancer and a precancerous lesion thereof is evaluated to be high.

(i1) A concentration of at least one free fatty acid selected from the group consisting of palmitoleic acid, linoleic acid, α-linolenic acid, oleic acid, palmitic acid, and arachidonic acid in the sample is high as compared with a predetermined threshold value.
(ii1) A concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.
(iii1) A concentration ratio of docosapentaenoic acid to oleic acid is low as compared with a predetermined threshold value.

The free fatty acids (palmitoleic acid, linoleic acid, α-linolenic acid, oleic acid, palmitic acid, and arachidonic acid) described in the (i1) are also collectively referred to as "free fatty acids of B(i1) group". The free fatty acids (vaccenic acid, arachidic acid, docosahexaenoic acid, adrenic acid, nervonic acid, lignoceric acid, stearic acid, dihomo-γ-linolenic acid, and docosapentaenoic acid) described in the (ii1) are also collectively referred to as "free fatty acids of the B(ii1) group".

The "predetermined threshold value" in the (i1) and (ii1) is a value set for each free fatty acid.

In a case where the free fatty acid measured in the step (A1) is a free fatty acid of the group B(i1), in a case where the concentration of the free fatty acid measured in the step (A1) is high as compared with the predetermined threshold value set for the free fatty acid, the possibility that the subject has one or more selected from the group consisting of a gynecological cancer and precancerous lesions thereof is evaluated to be high.

In a case where the free fatty acid measured in the step (A1) is a free fatty acid of the group B(ii1), in a case where the concentration of the free fatty acid measured in the step (A1) is low as compared with the predetermined threshold value set for the free fatty acid, the possibility that the subject has one or more selected from the group consisting of a gynecological cancer and precancerous lesions thereof is evaluated to be high.

In a case where two or more types of free fatty acids are measured in the step (A1), the free fatty acids are classified into free fatty acids of the group B(i1) and free fatty acids of the group B(ii1), and it can be confirmed whether each free fatty acid corresponds to (i1) or (ii1). As the proportion of the number of types of free fatty acids corresponding to (i1) or (ii1) is higher with respect to the number of types of the measured free fatty acids, the possibility that the subject has one or more selected from the group consisting of a gynecological cancer and precancerous lesions thereof can be evaluated to be higher.

The predetermined threshold value of the concentration of each free fatty acid in (i1) and (ii1) may be a cut-off value obtained by acquiring the concentration of each free fatty acid from a plurality of samples derived from healthy female subjects and a plurality of samples derived from patients having one or more selected from the group consisting of a gynecological cancer and precancerous lesions thereof, and statistically processing the concentration of each free fatty acid therefrom.

The predetermined threshold value of the concentration of the specific free fatty acid may be set, for example, such that the positive likelihood ratio for the specific free fatty acid is maximized. The threshold value at which the positive likelihood ratio is maximized can be set, for example, by performing ROC analysis. For example, concentrations of specific free fatty acids in the blood serum can be measured for a patient group having one or more selected from the group consisting of a gynecological cancer and precancerous lesions thereof and a healthy female subject group, which are constituted with any number of persons, and ROC analysis can be performed using the concentrations in the blood serum.

In a case where the sample derived from the subject, which is used in the step (A1), is blood serum, examples of the threshold value of the concentration of each free fatty acid in the (i1) and (ii1) include the following, but the threshold value is not limited thereto.

The threshold value of palmitoleic acid is preferably 20 µEQ/L or more and 40 µEQ/L or less, more preferably 23 µEQ/L or more and 35 µEQ/L or less, and still more preferably 25 µEQ/L or more and 33 µEQ/L or less. In a case where the gynecological cancer is an ovarian malignant tumor, specific examples of the threshold value of palmitoleic acid include 29.06 µEQ/L. In a case of intending to detect an early ovarian malignant tumor, specific examples of the threshold value include the same value as described above. In a case where the gynecological cancer is cervical cancer, specific examples of the threshold value of palmitoleic acid include 29.12 µEQ/L. In a case of intending to detect, including the cervical precancerous lesion, specific examples of the threshold value of palmitoleic acid include 36.63 µEQ/L.

The threshold value of linoleic acid is preferably 170 µEQ/L or more and 200 µEQ/L or less, more preferably 175 µEQ/L or more and 190 µEQ/L or less, and still more preferably 180 µEQ/L or more and 185 µEQ/L or less. In a case where the gynecological cancer is an ovarian malignant tumor, specific examples of the threshold value of linoleic acid include 183.1 µEQ/L. In a case of intending to detect an early ovarian malignant tumor, specific examples of the threshold value include the same value as described above. In a case where the gynecological cancer is cervical cancer, specific examples of the threshold value of linoleic acid include 84.63 µEQ/L. In a case of intending to detect, including the cervical precancerous lesion, specific examples of the threshold value of linoleic acid include 83.34 µEQ/L.

The threshold value of α-linolenic acid is preferably 20 µEQ/L or more and 40 µEQ/L or less, more preferably 23 µEQ/L or more and 35 µEQ/L or less, and still more preferably 25 µEQ/L or more and 33 µEQ/L or less. In a case where the gynecological cancer is an ovarian malignant tumor, specific examples of the threshold value of α-linolenic acid include 28.62 µEQ/L. In a case of intending to detect an early ovarian malignant tumor, specific examples of the threshold value include the same value as described above. In a case where the gynecological cancer is cervical cancer, specific examples of the threshold value of α-linolenic acid include 13.13 µEQ/L. In a case of intending to detect, including the cervical precancerous lesion, specific examples of the threshold value of α-linolenic acid include 8.334 µEQ/L.

The threshold value of oleic acid is preferably 300 µEQ/L or more and 35 µEQ/L or less, more preferably 310 µEQ/L or more and 340 µEQ/L or less, and still more preferably 320 µEQ/L or more and 330 µEQ/L or less. In a case where the gynecological cancer is an ovarian malignant tumor, specific examples of the threshold value of oleic acid include 326.0 µEQ/L. In a case of intending to detect an early ovarian malignant tumor, specific examples of the threshold value include the same value as described above. In a case where the gynecological cancer is cervical cancer, specific examples of the threshold value of oleic acid include 251.5 µEQ/L. In a case of intending to detect, including the cervical precancerous lesion, specific examples of the threshold value of oleic acid include 333.4 µEQ/L.

The threshold value of vaccenic acid is preferably 1.2 µEQ/L or more and 2.4 µEQ/L or less, more preferably 1.5 µEQ/L or more and 2.2 µEQ/L or less, and still more preferably 1.8 µEQ/L or more and 2.0 µEQ/L or less. In a case where the gynecological cancer is an ovarian malignant tumor, specific examples of the threshold value of vaccenic acid include 1.926 µEQ/L. In a case of intending to detect an early ovarian malignant tumor, specific examples of the threshold value of the vaccenic acid include 1.930 µEQ/L. In a case where the gynecological cancer is cervical cancer, a specific example of the threshold value of the vaccenic acid include 5.69 µEQ/L. In a case of intending to detect, including the cervical precancerous lesion, specific examples of the threshold value of arachidonic acid include 6.655 µEQ/L.

The threshold value of arachidic acid is preferably 1.2 µEQ/L or more and 2.4 µEQ/L or less, more preferably 1.5 µEQ/L or more and 2.2 µEQ/L or less, and still more preferably 1.8 µEQ/L or more and 2.0 µEQ/L or less. In a case where the gynecological cancer is an ovarian malignant tumor, specific examples of the threshold value of vaccenic acid include 1.924 µEQ/L. In a case of intending to detect an early ovarian malignant tumor, specific examples of the threshold value of the arachidic acid include 1.953 µEQ/L. In a case where the gynecological cancer is cervical cancer, specific examples of the threshold value of arachidic acid include 1.875 µEQ/L. In a case of intending to detect, including the cervical precancerous lesion, specific examples of the threshold value of arachidic acid include 1.781 µEQ/L.

The threshold value of docosahexaenoic acid is preferably 1.3 µEQ/L or more and 2.3 µEQ/L or less, more preferably 1.5 µEQ/L or more and 2.2 µEQ/L or less, and still more preferably 1.7 µEQ/L or more and 2.03 µEQ/L or less. In a case where the gynecological cancer is an ovarian malignant tumor, specific examples of the threshold value of docosahexaenoic acid include 1.847 µEQ/L. In a case of intending to detect an early ovarian malignant tumor, specific examples of the threshold value include the same value as described above. In a case where the gynecological cancer is cervical cancer, specific examples of the threshold value of docosahexaenoic acid include 2.670 µEQ/L.

The threshold value of adrenic acid is preferably 0.55 µEQ/L or more and 0.85 µEQ/L or less, more preferably 0.60 µEQ/L or more and 0.80 µEQ/L or less, and still more preferably 0.65 µEQ/L or more and 0.75 µEQ/L or less.

In a case where the gynecological cancer is an ovarian malignant tumor, specific examples of the threshold value of adrenic acid include 0.7085 µEQ/L. In a case where the gynecological cancer is cervical cancer, specific examples of the threshold value of adrenic acid include 0.6688 µEQ/L.

The threshold value of nervonic acid is preferably 0.06 µEQ/L or more and 0.11 µEQ/L or less, more preferably 0.07 µEQ/L or more and 0.10 µEQ/L or less, and still more preferably 0.08 µEQ/L or more and 0.09 µEQ/L or less.

In a case where the gynecological cancer is an ovarian malignant tumor, specific examples of the threshold value of nervonic acid include 0.0891 µEQ/L.

The threshold value of lignoceric acid is preferably 0.3 µEQ/L or more and 0.8 µEQ/L or less, more preferably 0.4 µEQ/L or more and 0.7 µEQ/L or less, and still more preferably 0.5 µEQ/L or more and 0.6 µEQ/L or less. In a case where the gynecological cancer is an ovarian malignant tumor, specific examples of the threshold value of lignoceric acid include 0.5773 µEQ/L. In a case of intending to detect an early ovarian malignant tumor, specific examples of the threshold value include the same value as described above.

The threshold value of the concentration of stearic acid is preferably 200 µEQ/L or more and 300 µEQ/L or less, more preferably 220 µEQ/L or more and 280 µEQ/L or less, and still more preferably 240 µEQ/L or more and 260 µEQ/L or less. Stearic acid can be suitably used for evaluation, for example, in a case where the gynecological cancer is endometrial cancer or cervical cancer.

The threshold value of palmitic acid is preferably 350 µEQ/L or more and 600 µEQ/L or less, more preferably 400 µEQ/L or more and 550 µEQ/L or less, and still more preferably 430 µEQ/L or more and 500 µEQ/L or less. In a case where the gynecological cancer is ovarian cancer, specific examples of the threshold value of palmitic acid include 452.9 EQ/L. Palmitic acid can be suitably used for evaluation, for example, in a case where the gynecological cancer is endometrial cancer or cervical cancer.

The threshold value of dihomo-γ-linolenic acid is preferably 0.8 µEQ/L or more and 1.0 µEQ/L or less, more preferably 0.85 µEQ/L or more and 0.95 µEQ/L or less, and still more preferably 0.90 µEQ/L or more and 0.95 µEQ/L or less. In a case where the gynecological cancer is cervical cancer, specific examples of the threshold value of dihomo-γ-linolenic acid include 0.9315 µEQ/L.

The threshold value of docosapentaenoic acid is preferably 0.6 µEQ/L or more and 1.5 µEQ/L or less, more preferably 0.65 µEQ/L or more and 1.2 µEQ/L or less, and still more preferably 0.7 µEQ/L or more and 1.0 µEQ/L or less. In a case where the gynecological cancer is cervical cancer, specific examples of the threshold value of docosapentaenoic acid include 0.7814 µEQ/L.

The "predetermined threshold value" in the (iii1) is a value set to the concentration ratio of docosapentaenoic acid to oleic acid.

In a case where the concentration ratio of docosapentaenoic acid to oleic acid, which is calculated based on the of free fatty acid concentrations measured in the step (A1), is low as compared with the threshold value set for the concentration ratio of docosapentaenoic acid to oleic acid, the possibility that the subject has one or more selected from the group consisting of a gynecological cancer and precancerous lesions thereof is evaluated to be high.

The predetermined threshold value for the concentration ratio in the (iii1) may be a cut-off value obtained by calculating the concentration ratio from a plurality of samples derived from healthy female subjects and a plurality of samples derived from patients having one or more selected from the group consisting of a gynecological cancer and precancerous lesions thereof, and statistically processing the concentration ratio thereof.

The predetermined threshold value for the concentration ratio in the (iii1) may be set, for example, such that the positive likelihood ratio for the concentration ratio is maximized. The threshold value at which the positive likelihood ratio is maximized can be set, for example, by performing ROC analysis. For example, the concentration ratio can be measured for a patient group having one or more selected from the group consisting of a gynecological cancer and precancerous lesions thereof and a healthy female subject group, which are constituted with any number of persons, and ROC analysis can be performed using the concentration ratio thereof.

In a case where the sample derived from the subject, which is used in the step (A1), is blood serum, examples of the predetermined threshold value for the concentration ratio of docosapentaenoic acid to oleic acid include 0.004433, but the threshold value is not limited thereto.

In the step (B 1), in a case corresponding to the (iii1), a possibility that the subject has cervical cancer is preferably evaluated to be high. In this case, the cervical cancer that can be detected may be cervical cancer having a degree of progress of Stage I or more.

In a case where a gynecological cancer is to be detected, the free fatty acid used for the evaluation is preferably one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid. The detection accuracy of the gynecological cancer is further improved by selecting a free fatty acid to be used for evaluation from the fourteen types of free fatty acids. Among the free fatty acids, the free fatty acid used for the evaluation may be two or more types, three or more types, four or more types, five or more types, six or more types, seven or more types, eight or more types, nine or more types, ten or more types, eleven or more types, twelve or more types, thirteen or more types, or fourteen types of the free fatty acids. The free fatty acid used for the evaluation can be arbitrarily selected from these fourteen types of fatty acids.

In a case where a gynecological cancer is to be detected, for example, in the step (B 1), the following evaluation may be performed.

In a case corresponding to at least one selected from the group consisting of the following (i1)-0 and (ii1)-0, a possibility that the subject has ovarian cancer is evaluated to be high.
(i1)-0 A concentration of at least one free fatty acid selected from the group consisting of palmitoleic acid, linoleic acid, α-linolenic acid, oleic acid, and palmitic acid in the sample is high as compared with a predetermined threshold value.
(ii1)-0 A concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.

In a case where the gynecological cancer which may be to be detected is ovarian cancer, the free fatty acid used for the evaluation is preferably one or more types of free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The detection accuracy of the ovarian cancer is further improved by selecting a free fatty acid to be used for evaluation from the ten types of free fatty acids. Among the free fatty acids, the free fatty acid used for the evaluation may be two or more types, three or more types, four or more types, five or more types, six or more types, seven or more types, eight or more types, nine or more types, or ten types of the free fatty acids. The free fatty acid used for the evaluation can be arbitrarily selected from these ten types of fatty acids.

In a case where the gynecological cancer is ovarian cancer, for example, in the step (B 1), the following evaluation may be performed.

In a case corresponding to at least one selected from the group consisting of the following (i1)-1 and (ii1)-1, the possibility that the subject has ovarian cancer is evaluated to be high.
(i1)-1 A concentration of at least one free fatty acid selected from the group consisting of palmitoleic acid, linoleic acid, α-linolenic acid, and oleic acid in the sample is high as compared with a predetermined threshold value.
(ii1)-1 A concentration of at least one free fatty acid selected from the group consisting of vaccenic acid, arachidic acid, docosahexaenoic acid, adrenic acid, nervonic acid, and lignoceric acid in the sample is low as compared with a predetermined threshold value.

To increase the detection sensitivity of early gynecological malignant tumors (for example, Stage I and II), the free fatty acid used for the evaluation may be one or more free fatty acids selected from the group consisting of palmitoleic acid, linoleic acid, α-linolenic acid, oleic acid, vaccenic acid, arachidic acid, docosahexaenoic acid, and lignoceric acid. The detection accuracy of the early gynecological malignant tumor (particularly, a Stage I or II ovarian malignant tumor) is further improved by selecting a free fatty acid to be used for evaluation from the eight types of free fatty acids. Among the free fatty acids, the free fatty acid used for the evaluation may be two or more types, three or more types, four or more types, five or more types, six or more types, seven or more types, or eight types of the free fatty acids. The free fatty acid used for the evaluation can be arbitrarily selected from these eight types of fatty acids.

In a case of intending to detect the early gynecological malignant tumor, for example, in the step (B 1), the following evaluation may be performed.

In a case corresponding to at least one selected from the group consisting of the following (i1)-2 and (ii1)-2, a possibility that the subject has a gynecological cancer (for example, an ovarian malignant tumor) having a degree of progress of Stage I or higher is evaluated to be high.
(i1)-2 A concentration of at least one free fatty acid selected from the group consisting of palmitoleic acid, linoleic acid, α-linolenic acid, and oleic acid in the sample is high as compared with a predetermined threshold value.
(ii1)-2 A concentration of at least one free fatty acid selected from the group consisting of vaccenic acid, arachidic acid, docosahexaenoic acid, and lignoceric acid in the sample is low as compared with a predetermined threshold value.

In a case where the gynecological cancer which may be to be detected is endometrial cancer or cervical cancer, the free fatty acid used for the evaluation is preferably one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The detection accuracy of the endometrial cancer or cervical cancer is further improved by selecting a free fatty acid to be used for evaluation from the twelve types of free fatty acids. Among the free fatty acids, the free fatty acid used for the evaluation may be two or more types, three or more types, four or more types, five or more types, six or more types, seven or more types, eight or more types, nine or more types, ten or more types, eleven or more types, or twelve types of the free fatty acids. The free fatty acid used for the evaluation can be arbitrarily selected from these twelve types of fatty acids.

In a case where the gynecological cancer is endometrial cancer or cervical cancer, for example, in the step (B 1), the following evaluation may be performed.

In a case corresponding to at least one selected from the group consisting of the following (i1)-3 and (ii1)-3, the possibility that the subject has ovarian cancer is evaluated to be high.
(i1)-3 A concentration of at least one free fatty acid selected from the group consisting of palmitic acid, palmitoleic acid, linoleic acid, α-linolenic acid, and oleic acid in the sample is high as compared with a predetermined threshold value.
(ii1)-3 A concentration of at least one free fatty acid selected from the group consisting of vaccenic acid, stearic acid, arachidic acid, dihomo-γ-linolenic acid, docosahexaenoic acid, adrenic acid, and docosapentaenoic acid in the sample is low as compared with a predetermined threshold value.

In a case where the gynecological cancer which may be to be detected is endometrial cancer, the free fatty acid used for the evaluation is preferably one or more free fatty acids selected from the group consisting of palmitic acid, palmitoleic acid, linoleic acid, α-linolenic acid, oleic acid, vaccenic acid, stearic acid, and arachidic acid. The detection accuracy of the endometrial cancer is further improved by selecting a free fatty acid to be used for evaluation from the eight types of free fatty acids. Among the free fatty acids, the free fatty acid used for the evaluation may be two or more types, three or more types, four or more types, five or more types, six or more types, seven or more types, or eight types of the free fatty acids. The free fatty acid used for the evaluation can be arbitrarily selected from these eight types of fatty acids.

In a case where the gynecological cancer is endometrial cancer, for example, in the step (B 1), the following evaluation may be performed.

In a case corresponding to at least one selected from the group consisting of the following (i1)-4 and (ii1)-4, the possibility that the subject has ovarian cancer is evaluated to be high.
(i1)-4 A concentration of at least one free fatty acid selected from the group consisting of palmitic acid, palmitoleic acid, linoleic acid, α-linolenic acid, and oleic acid in the sample is high as compared with a predetermined threshold value.
(ii1)-4 A concentration of at least one free fatty acid selected from the group consisting of vaccenic acid, arachidic acid, docosahexaenoic acid, and adrenic acid in the sample is low as compared with a predetermined threshold value.

In a case where the gynecological cancer which may be to be detected is cervical cancer, the free fatty acid used for the evaluation is preferably one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The detection accuracy of the cervical cancer is further improved by selecting a free fatty acid to be used for evaluation from the twelve types of free fatty acids. Among the free fatty acids, the free fatty acid used for the evaluation may be two or more types, three or more types, four or more types, five or more types, six or more types, seven or more types, eight or more types, nine or more types, ten or more types, eleven or more types, or twelve types of the free fatty acids. The free fatty acid used for the evaluation can be arbitrarily selected from these twelve types of fatty acids.

In a case where the gynecological cancer is cervical cancer, for example, in the step (B 1), the following evaluation may be performed.

In a case corresponding to at least one selected from the group consisting of the following (i1)-5 and (ii1)-5, the possibility that the subject has ovarian cancer is evaluated to be high.
(i1)-5 A concentration of at least one free fatty acid selected from the group consisting of palmitic acid, palmitoleic acid, linoleic acid, α-linolenic acid, and oleic acid in the sample is high as compared with a predetermined threshold value.
(ii1)-5 A concentration of at least one free fatty acid selected from the group consisting of vaccenic acid, stearic acid, arachidic acid, dihomo-γ-linolenic acid, docosahexaenoic acid, adrenic acid, and docosapentaenoic acid in the sample is low as compared with a predetermined threshold value.

In a case of intending to detect, including a gynecological precancerous lesion (for example, a cervical precancerous lesion), as the free fatty acid to be used for the evaluation, one or more free fatty acids selected from the group consisting of palmitoleic acid, linoleic acid, α-linolenic acid, oleic acid, arachidonic acid, and arachidic acid are preferable. Among the free fatty acids, the free fatty acid used for the evaluation may be two or more types, three or more types, four or more types, five or more types, or six types of the free fatty acids. The free fatty acid used for the evaluation can be arbitrarily selected from these six types of fatty acids.

In a case where a gynecological cancer and precancerous lesions thereof are to be detected, for example, in the step (B 1), the following evaluation may be performed.

In a case corresponding to at least one selected from the group consisting of the following (i1)-6 and (ii1)-6, a possibility that the subject has gynecological precancerous lesions and/or the gynecological cancer is evaluated to be high.
(i1)-6 A concentration of at least one free fatty acid selected from the group consisting of palmitoleic acid, linoleic acid, α-linolenic acid, and oleic acid in the sample is high as compared with a predetermined threshold value.
(ii1)-6 A concentration of the free fatty acid of arachidonic acid in the sample is low as compared with a predetermined threshold value.

In the step (B 1), the step of determining whether or not the subject corresponds to one or more selected from the group consisting of the (i1) and (ii1) may be a step in which the possibility that the subject has a gynecological cancer is determined to be high (hereinafter, also referred to as a "step (B 1-1)") in a case where a total value of the score (hereinafter, referred to as an "FFA score") of each free fatty acid set in the following (i-1) and (ii-1) is higher than a predetermined threshold value.

(i-1) In a case where the free fatty acid is a free fatty acid of the group B(i1), in a case where the concentration of the free fatty acid in the sample is high as compared with a predetermined threshold value, the score of the free fatty acid is set to "+1". In a case where the concentration of the free fatty acid in the sample is low as compared with a predetermined threshold value, the score of the free fatty acid is set to "0".
(ii-1) In a case where the free fatty acid is a free fatty acid of the group B(ii1), in a case where the concentration of the free fatty acid in the sample is low as compared with a predetermined threshold value, the score of the free fatty acid is set to "+1". In a case where the concentration of the free fatty acid in the sample is high as compared with a predetermined threshold value, the score of the free fatty acid is set to "0".

Examples of the predetermined threshold value for each free fatty acid used in (i-1) and (ii-1) include the same ones as those exemplified as the predetermined threshold value in (i1) and (ii1). The total value of the scores of the free fatty acids set by (i-1) and (ii-1) is calculated and defined as the FFA score.

The predetermined threshold value for the FFA score may be a cut-off value obtained by calculating the FFA score for a plurality of samples derived from healthy female subjects and a plurality of samples derived from patients having one or more selected from the group consisting of a gynecological cancer and precancerous lesions thereof, and statistically processing the FFA score thereof.

The predetermined threshold value for the FFA score may be set, for example, such that the positive likelihood ratio for the FFA score is maximized. The threshold value at which the positive likelihood ratio is maximized can be set, for example, by performing ROC analysis. For example, concentrations of specific free fatty acids in the blood serum can be measured for a patient group having one or more selected from the group consisting of a gynecological cancer and precancerous lesions thereof and a healthy female subject group, which are constituted with any number of persons, and ROC analysis can be performed using the concentrations in the blood serum.

The predetermined threshold value for the FFA score depends on the number of types of free fatty acids used for calculating the FFA score, and examples thereof include 1 or more and 3 or less, and more than 1 and less than 3 is preferable, more than 1 and 2 or less is more preferable, and more than 1 and less than 2 is still more preferable. Examples of the number of types of free fatty acids used for calculating the FFA score include 3 or more, 4 or more, 5 or more, 6 or more, 7 or more, 8 or more, 9 or more, 10 or more, 11 or more, 12 or more, 13 or more, 14 or more, and 15 of free fatty acids among the free fatty acids of the group A1. The free fatty acid used for calculating the FFA score can be arbitrarily selected from the fatty acids of the group A1.

In a case where the gynecological cancer is ovarian cancer, the free fatty acid used for calculating the FFA score is preferably one or more selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The free fatty acid used for calculating the FFA score may be three or more types, four or more types, five or more types, six or more types, seven or more types, eight or more types, nine or more types, or ten types of the free fatty acids. The free fatty acid used for calculating the FFA score can be arbitrarily selected from these fatty acids. In a case where the gynecological cancer is ovarian cancer, it is preferable to use the ten types of free fatty acids for calculating the FFA score. In a case of using the ten types of free fatty acids, examples of the predetermined threshold value of the FFA score include the same ones as described above, and the FFA score can be set to, for example, more than 1 and less than 2 (for example, 1.5).

To increase the detection sensitivity of early gynecological malignant tumors (for example, Stage I and II), the free fatty acid used for calculating the FFA score may be one or more selected from the group consisting of palmitoleic acid, linoleic acid, α-linolenic acid, oleic acid, vaccenic acid, arachidic acid, docosahexaenoic acid, and lignoceric acid. The free fatty acid used for calculating the FFA score may be three or more types, four or more types, five or more types, six or more types, seven or more types, or eight types of the free fatty acids. The free fatty acid used for calculating the FFA score can be arbitrarily selected from these fatty acids. From the viewpoint of detection sensitivity of early gynecological malignant tumors, it is preferable that the eight types of free fatty acids is used for calculating the FFA score.

In a case of using the eight types of free fatty acids, examples of the predetermined threshold value of the FFA score include the same ones as described above, and the FFA score can be set to, for example, more than 1 and less than 2 (for example, 1.5).

In a case where the gynecological cancer is endometrial cancer or cervical cancer, the free fatty acid used for calculating the FFA score is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for calculating the FFA score can be arbitrarily selected from these fatty acids. The number of types of free fatty acids used for calculating the FFA score and the predetermined threshold value of the FFA score can be appropriately set.

In a case where the gynecological cancer is endometrial cancer, the free fatty acid used for calculating the FFA score is preferably one or more selected from the group consisting of palmitic acid, palmitoleic acid, linoleic acid, α-linolenic acid, oleic acid, vaccenic acid, stearic acid, and arachidic acid. The free fatty acid used for calculating the FFA score may be three or more types, four or more types, five or more types, six or more types, seven or more types, or eight types of the free fatty acids. The free fatty acid used for calculating the FFA score can be arbitrarily selected from these fatty acids. The number of types of free fatty acids used for calculating the FFA score and the predetermined threshold value of the FFA score can be appropriately set.

In a case where the gynecological cancer is cervical cancer, the free fatty acid used for calculating the FFA score is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for calculating the FFA score can be arbitrarily selected from these fatty acids. The number of types of free fatty acids used for calculating the FFA score and the predetermined threshold value of the FFA score can be appropriately set.

In a case of intending to detect, including a gynecological precancerous lesion, as the free fatty acid to be used for calculating the FFA score, one or more selected from the group consisting of palmitoleic acid, linoleic acid, α-linolenic acid, oleic acid, arachidonic acid, and arachidic acid are preferable. The free fatty acid used for calculating the FFA score may be two or more types, three or more types, four or more types, five or more types, or six types of the free fatty acids. The free fatty acid used for calculating the FFA score can be arbitrarily selected from these fatty acids. The number of types of free fatty acids used for calculating the FFA score and the predetermined threshold value of the FFA score can be appropriately set.

In the test method according to the first aspect, in a case where the FFA score is used, specific examples of the step (A1) and the step (B1) include the following.

### (Example 1)

In the step (A1), the concentrations of ten types of free fatty acids consisting of palmitoleic acid, linoleic acid, α-linolenic acid, oleic acid, vaccenic acid, arachidic acid, docosahexaenoic acid, adrenic acid, nervonic acid, and lignoceric acid in the sample derived from the subject.

In the step (B1), the possibility that the subject has a gynecological cancer is determined to be high in a case where the FFA score calculated from ten types of free fatty acids consisting of palmitoleic acid, linoleic acid, α-linolenic acid, oleic acid, vaccenic acid, arachidic acid, docosahexaenoic acid, adrenic acid, nervonic acid, and lignoceric acid is higher than a predetermined threshold value.

The threshold value of the FFA score calculated from the ten types of free fatty acids is preferably 1 or more and 3 or less, more preferably 1.2 or more and 2.5 or less, still more preferably 1.3 or more and 2 or less, and particularly preferably 1.3 or more and 1.9 or less.

### (Example 2)

In the step (A1), the concentrations of eight types of free fatty acids consisting of palmitoleic acid, linoleic acid, α-linolenic acid, oleic acid, vaccenic acid, arachidic acid, docosahexaenoic acid, and lignoceric acid in the sample derived from the subject.

In the step (B1), the possibility that the subject has a gynecological cancer is determined to be high in a case where the FFA score calculated from eight types of free fatty acids consisting of palmitoleic acid, linoleic acid, α-linolenic acid, oleic acid, vaccenic acid, arachidic acid, docosahexaenoic acid, and lignoceric acid is higher than a predetermined threshold value.

The threshold value of the FFA score of the eight types of free fatty acids is preferably 0.5 or more and 2.5 or less, more preferably 1 or more and 2 or less, and still more preferably 1.1 or more and 1.9 or less.

By using the FFA score, it is possible to evaluate with higher accuracy the possibility that the subject has one or more selected from the group consisting of a gynecological cancer and precancerous lesions thereof. By using the FFA score, it is possible to accurately detect even an early gynecological malignant tumor (for example, an early ovarian malignant tumor), a gynecological clear cell malignant tumor (for example, an ovarian clear cell malignant tumor), or a gynecological borderline malignant tumor (for example, an ovarian borderline malignant tumor). In these tumors, the proportion of false negatives is high and the sensitivity is low in conventional tumor markers such as CA125. However, by using the FFA score, these tumors can also be detected with high sensitivity. For example, for the early gynecological malignant tumor, by using an FFA score, the Stage I or Stage II gynecological malignant tumor can be detected with high sensitivity.

In the step (B1), in the subject evaluated to have a high possibility of having one or more selected from the group consisting of a gynecological cancer and precancerous lesions thereof, has a high possibility of having one or more selected from the group consisting of any cancers among gynecological cancers, and precancerous lesions thereof. Therefore, to identify one or more selected from the group consisting of a gynecological cancer and precancerous lesions thereof, further detection may be performed by a computer tomography (CT) examination, a magnetic resonance image diagnosis (MRI), a positron emission tomography (PET) examination, an ultrasonography, or the like. Furthermore, a pathological examination (cytodiagnostic examination or histological examination) may be performed for definitive diagnosis.

The sample test method according to the first aspect may further include the following step (C1).

The step (C1): evaluating whether the subject determined to have a high possibility of having one or more selected from the group consisting of a gynecological cancer and a gynecological precancerous lesion by the step (B 1) has any of a gynecological cancer or a gynecological cancer precancerous lesion based on the concentration of the free fatty acid in the sample, which is obtained in the step (A1)

The subject in the step (C1) is a subject determined to have a high possibility of having one or more selected from the group consisting of a gynecological cancer and precancerous lesions thereof by the step (B1).

### (Step (C1))

In the step (B 1), based on the concentration of the free fatty acid in the sample, which is obtained in the step (A1), whether the subject has any of the gynecological cancer or the gynecological precancerous lesions is evaluated.

The gynecological cancer to be determined is preferably cervical cancer. The gynecological precancerous lesion to be determined is preferably a cervical precancerous lesion.

In the step (C1), the possibility that the subject has a gynecological cancer is determined to be high in a case corresponding to the following (Ci).

(Ci) A concentration of at least one free fatty acid selected from the group consisting of dihomo-γ-linolenic acid, docosahexaenoic acid, adrenic acid, and docosapentaenoic acid in the sample is low as compared with a predetermined threshold value.

Hereinafter, the dihomo-γ-linolenic acid, docosahexaenoic acid, adrenic acid, and docosapentaenoic acid are collectively referred to as "free fatty acids of C1 group".

In the step (C1), the number of types of free fatty acids to be determined is only required to be 1 or more selected from the free fatty acids of the C1 group, may be 2 or more, may be 3 or more, or 4, selected from the free fatty acids of the C1 group. The free fatty acid to be determined can be arbitrarily selected from the free fatty acids of the group C1.

The "predetermined threshold value" in the (Ci) is a value set for each free fatty acid.

The predetermined threshold value of the concentration of each free fatty acid in the (Ci) may be a cut-off value obtained by acquiring the concentration of each free fatty acid from a plurality of samples derived from patients having a gynecological cancer and a plurality of samples derived from patients having gynecological precancerous lesions, and statistically processing the concentrations of each free fatty acid thereof.

The predetermined threshold value of the concentration of the specific free fatty acid may be set, for example, such that the positive likelihood ratio for the specific free fatty acid is maximized. The threshold value at which the positive likelihood ratio is maximized can be set, for example, by performing ROC analysis. For example, concentrations of specific free fatty acids in the blood serum can be measured for a patient group having a gynecological cancer and a patient group having gynecological precancerous lesions, which are constituted with any number of persons, and ROC analysis can be performed using the concentrations in the blood serum.

Examples of the threshold value of the concentration of each free fatty acid in the (Ci) include the following, but the threshold value is not limited thereto.

The threshold value of dihomo-γ-linolenic acid is preferably 0.80 µEQ/L or more and 1.20 µEQ/L or less, more preferably 0.90 µEQ/L or more and 1.10 µEQ/L or less, and still more preferably 0.95 µEQ/L or more and 1.05 µEQ/L or less. In a case where the gynecological cancer is cervical cancer and the gynecological precancerous lesion is a cervical precancerous lesion, specific examples of the threshold value of the dihomo-γ-linolenic acid include 1.012 µEQ/L.

The threshold value of docosahexaenoic acid is preferably 2.0 µEQ/L or more and 5.0 µEQ/L or less, more preferably 2.5 µEQ/L or more and 4.5 µEQ/L or less, and still more preferably 3.0 µEQ/L or more and 4.0 µEQ/L or less. In a case where the gynecological cancer is cervical cancer and the gynecological precancerous lesion is a cervical precancerous lesion, specific examples of the threshold value of the docosahexaenoic acid include 3.507 µEQ/L.

The threshold value of adrenic acid is preferably 0.15 µEQ/L or more and 0.60 µEQ/L or less, more preferably 0.20 µEQ/L or more and 0.50 µEQ/L or less, and still more preferably 0.25 µEQ/L or more and 0.40 µEQ/L or less.

In a case where the gynecological cancer is cervical cancer and the gynecological precancerous lesion is a cervical precancerous lesion, specific examples of the threshold value of the docosahexaenoic acid include 0.312 µEQ/L.

The threshold value of docosapentaenoic acid is preferably 0.40 µEQ/L or more and 1.00 µEQ/L or less, more preferably 0.50 µEQ/L or more and 0.90 µEQ/L or less, and still more preferably 0.60 µEQ/L or more and 0.80 µEQ/L or less. In a case where the gynecological cancer is cervical cancer and the gynecological precancerous lesion is a cervical precancerous lesion, specific examples of the threshold value of the docosapentaenoic acid include 0.7231 µEQ/L.

The step (C1) may be a step of calculating an FFA score, and evaluating the possibility that the subject has a gynecological cancer to be high in a case where the FFA score is higher than a predetermined threshold value. The FFA score is calculated by the same method as the method described in the first aspect. That is, the FFA score is calculated as a total value of the scores of free fatty acids set in the same manner as in the (i-1) and (ii-1).

The predetermined threshold value for the FFA score may be a cut-off value obtained by calculating the FFA score for a plurality of samples derived from gynecological cancer patients and a plurality of samples derived from patients having the precancerous lesions, and statistically processing the FFA scores.

The predetermined threshold value for the FFA score depends on the number of types of free fatty acids used for calculating the FFA score, and examples thereof include 0.2n or more and 0.6n or less, 0.3n or more and 0.6n or less, 0.4n or more and 0.6n or less, and the like in a case where the number of types of free fatty acids is represented by n. Examples of the number of types of free fatty acids used for calculating the FFA score include 3 or more and 4 among the free fatty acids of the group A5.

For a subject confirmed to have one or more selected from the group consisting of a gynecological cancer and precancerous lesions thereof by the diagnosis as described above, treatments for one or more selected from the group consisting of a gynecological cancer and precancerous lesions thereof can be performed.

In one embodiment, the present invention provides a treatment method for one or more selected from the group consisting of a gynecological cancer and precancerous lesions thereof, the treatment method including a step (a1) of specifying a subject having a high possibility of having one or more selected from the group consisting of a gynecological cancer and precancerous lesions thereof by the sample test method according to the first aspect, a step (b1) of confirming that the subject has one or more selected from the group consisting of a gynecological cancer and precancerous lesions thereof, and a step (c1) of performing a treatment for one or more selected from the group consisting of a gynecological cancer and precancerous lesions thereof on the subject confirmed to have one or more selected from the group consisting of the gynecological cancer and the precancerous lesions thereof.

In the step (a1), the sample test method according to the first aspect is performed as described above to specify a subject having a high possibility of having one or more selected from the group consisting of a gynecological cancer and precancerous lesions thereof.

In the step (b 1), it is possible to confirm that the subject has one or more selected from the group consisting of a gynecological cancer and precancerous lesions thereof by performing a further test on the subject specified in the step (a1). Examples of the test for one or more selected from the group consisting of a gynecological cancer and precancerous lesions thereof include the above-described test.

In the step (c1), it is possible to perform the treatment for one or more selected from the group consisting of a gynecological cancer and precancerous lesions thereof on the subject confirmed to have one or more selected from the group consisting of a gynecological cancer and precancerous lesions thereof in the step (b 1). The treatment method for a gynecological cancer is not particularly limited, and examples thereof include surgical treatment, chemotherapy, radiation therapy, and the like. In a case where, for example, the gynecological cancer is an early gynecological cancer (for example, Stage I or Stage II), for example, a surgical treatment can be selected as the main treatment method. In a case of the early gynecological cancer, in a case where the gynecological cancer can be completely removed, for example, by surgical treatment or the like, the gynecological cancer can be completely cured. In a case of a gynecological cancer (for example, Stage III or Stage IV) in which the degree of progress is advanced, treatment may be performed by combining surgical treatment, chemotherapy (administration of an anticancer agent or the like), and radiation therapy. For example, chemotherapy and/or radiation therapy may be performed before surgical treatment to reduce the tumor. Alternatively, after the surgical treatment, chemotherapy and/or radiation therapy may be performed to reduce the tumor remaining after the surgical treatment. Examples of the anticancer agent used in chemotherapy include the anticancer agents described later in the section of [Medicine]. As a treatment method for a gynecological precancerous lesion, for example, a vaginal operation such as a cervical conization or a cervical laser vaporization may be performed.

According to the sample test method according to the first aspect described above, it is possible to simply determine with high specificity and high sensitivity the possibility that the subject has one or more selected from the group consisting of a gynecological cancer and precancerous lesions thereof.

In the diagnosis using CA125 as a marker, the detection sensitivity of early ovarian malignant tumor and ovarian clear cell malignant tumor is low. In the test method according to the first aspect, it is possible to perform detection with high specificity and high sensitivity even in a case where the subject has an early ovarian malignant tumor or an ovarian clear cell malignant tumor.

Advanced gynecological cancer has a high probability of recurrence even after treatment, and has poor prognosis in many cases. On the other hand, in a case where the gynecological cancer is detected at an early stage before the cancer progresses, it is possible to completely cure the gynecological cancer by treatment such as resection of the tumor. Or the prognosis can be improved. According to the sample test method according to the first aspect, it is possible to detect an early gynecological cancer, and thus it is possible to increase the probability of completely curing the gynecological cancer by treatment.

Since cervical cancer has a risk of metastasis, surgical treatment with high invasiveness such as modified radical hysterectomy, radical hysterectomy or lymphadenectomy, or treatment with radiation irradiation is performed. Therefore, in young people in their 30s and 40s who are at the peak of the disease, the fertility may be lost. On the other hand, since the cervical precancerous lesion is a tumor confined to the epithelium without invasion, there is no possibility of metastasis. Therefore, a treatment with less invasive such as cervical conization or cervical laser vaporization is performed, and the fertility is also preserved. Therefore, the detection of the cervical precancerous lesion is a great advantage for the subject.

The step (B1) may be a step of specifying a sample derived from the subject having a high possibility of having one or more selected from the group consisting of a gynecological cancer and precancerous lesions thereof in a case corresponding to at least one selected from the group consisting of the (i1) and (ii1).

The step (C1) may be a step of specifying a sample derived from the subject having a high possibility of having the gynecological cancer in a case corresponding to the (Ci).

### [Sample test method (2)]

The sample test method according to the second aspect is a method of evaluating a possibility that a subject having a gynecological cancer has poor prognosis. The test method includes a step (A2) and a step (B2).

The step (A2): a step of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid in a sample derived from a subject

The step (B2): a step of evaluating a possibility that the subject has poor prognosis based on the concentration of the free fatty acid in the sample, which is obtained in the step (A2)

In the sample test method according to the second aspect, the subject from which the sample to be subjected to the test is derived is a human female having a gynecological cancer. For example, the subject is a human female who has been diagnosed in advance to have a gynecological cancer. The subject may be before the start of the treatment of the gynecological cancer, may be during the treatment of the gynecological cancer, or may have received the treatment of the gynecological cancer in the past. Alternatively, the subject may be a subject determined to have a gynecological cancer by the test method according to the first aspect.

Examples of the gynecological cancer include the cancer described above in the first aspect. As the gynecological cancer, a gynecological malignant tumor is preferable, one or more selected from the group consisting of an ovarian malignant tumor, a corpus uteri malignant tumor, and a uterine cervix malignant tumor is more preferable, and an ovarian malignant tumor is still more preferable.

Palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid are collectively referred to as "free fatty acids of group A2".

The step (A2) can be performed in the same manner as the step (A1) in the first aspect, except that the sample derived from the subject having a gynecological cancer is used. The measurement may be performed in vitro.

In the step (A2), the number of types of free fatty acids to be measured is only required to be 1 or more selected from the free fatty acids of the group A2, and may be 2 or more, may be 3 or more, may be 4 or more, may be 5 or more, may be 6 or more, may be 7 or more, may be 8 or more, may be 9 or more, may be 10 or more, may be 11 or more, may be 12 or more, may be 13 or more, or may be 14 selected from the free fatty acids of the group A2. The free fatty acid to be measured can be arbitrarily selected from the free fatty acids of the group A2.

In a case where the gynecological cancer is ovarian cancer, in the step (A2), one or more types of free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid may be to be detected. Among the free fatty acids, two or more types, three or more types, four or more types, five or more types, six or more types, seven or more types, eight or more types, nine or more types, or ten types of the free fatty acids can be measured.

The free fatty acid to be measured can be arbitrarily selected from these free fatty acids.

In a case where the gynecological cancer is ovarian cancer, it is preferable that, in the step (A2), the concentrations of the ten types of free fatty acids are measured.

In a case where the gynecological cancer is an early gynecological malignant tumor (for example, Stage I and II), in the step (A2), one or more free fatty acids selected from the group consisting of palmitoleic acid, linoleic acid, α-linolenic acid, oleic acid, vaccenic acid, arachidic acid, docosahexaenoic acid, and lignoceric acid may be to be measured. Among the free fatty acids, two or more types, three or more types, four or more types, five or more types, six or more types, seven or more types, or eight types of the free fatty acids can be measured. The free fatty acid to be measured can be arbitrarily selected from these free fatty acids. In a case of intending to detect an early gynecological malignant tumor, it is preferable that, in the step (A2), concentrations of the eight types of free fatty acids are measured. Examples of the early gynecological malignant tumor include an early ovarian malignant tumor.

In a case where the gynecological cancer is endometrial cancer or cervical cancer, in the step (A2), one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid may be to be measured. Among the free fatty acids, two or more types, three or more types, four or more types, five or more types, six or more types, seven or more types, eight or more types, nine or more types, ten or more types, eleven or more types, or twelve types of the free fatty acids can be measured. The free fatty acid to be measured can be arbitrarily selected from these free fatty acids. In a case where the gynecological cancer is endometrial cancer or cervical cancer, it is preferable that, in the step (A2), the concentrations of the twelve types of free fatty acids are measured.

In a case where the gynecological cancer is endometrial cancer, in the step (A2), one or more free fatty acids selected from the group consisting of palmitic acid, palmitoleic acid, linoleic acid, α-linolenic acid, oleic acid, vaccenic acid, stearic acid, and arachidic acid may be to be measured. Among the free fatty acids, two or more types, three or more types, four or more types, five or more types, six or more types, seven or more types, or eight types of the free fatty acids can be measured. The free fatty acid to be measured can be arbitrarily selected from these free fatty acids. In a case where the gynecological cancer is endometrial cancer, it is preferable that, in the step (A2), the concentrations of the eight types of free fatty acids are measured.

In a case where the gynecological cancer is cervical cancer, in the step (A2), one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid may be to be measured. Among the free fatty acids, two or more types, three or more types, four or more types, five or more types, six or more types, seven or more types, eight or more types, nine or more types, ten or more types, eleven or more types, or twelve types of the free fatty acids can be measured. The free fatty acid to be measured can be arbitrarily selected from these free fatty acids. In a case where the cervical cancer is to be detected, it is preferable that, in the step (A2), the concentrations of the twelve types of free fatty acids are measured.

In the step (B2), in a case corresponding to at least one selected from the group consisting of the following (i2) and (ii2), the possibility that the subject has poor prognosis is evaluated to be high.
(i2) A concentration of at least one free fatty acid selected from the group consisting of palmitoleic acid, linoleic acid, α-linolenic acid, oleic acid, and palmitic acid in the sample is high as compared with a predetermined threshold value.
(ii2) A concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.

In a case where the gynecological cancer is ovarian cancer, the free fatty acid used for the evaluation is preferably one or more selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The free fatty acid used for the evaluation may be three or more types, four or more types, five or more types, six or more types, seven or more types, eight or more types, nine or more types, or ten types of the free fatty acids. The free fatty acid used for the evaluation can be arbitrarily selected from the ten types of free fatty acids. In a case where the gynecological cancer is ovarian cancer, the free fatty acid used in the evaluation is preferably the ten types of the free fatty acids.

In a case where the gynecological cancer is an early gynecological malignant tumor, the free fatty acid used for the evaluation is preferably one or more selected from the group consisting of palmitoleic acid, linoleic acid, α-linolenic acid, oleic acid, vaccenic acid, arachidic acid, docosahexaenoic acid, and lignoceric acid. The free fatty acid used for the evaluation may be three or more types, four or more types, five or more types, six or more types, seven or more types, or eight types of the free fatty acids. The free fatty acid used for the evaluation can be arbitrarily selected from the eight types of free fatty acids. In a case of intending to detect an early gynecological malignant tumor, the free fatty acid used in the evaluation is preferably the eight types of free fatty acids.

In a case where the gynecological cancer is endometrial cancer or cervical cancer, the free fatty acid used for the evaluation is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for the evaluation may be three or more types, four or more types, five or more types, six or more types, seven or more types, eight or more types, nine or more types, ten or more types, eleven or more types, or twelve types of the free fatty acids. The free fatty acid used for the evaluation can be arbitrarily selected from the twelve types of free fatty acids.

In a case where the gynecological cancer is endometrial cancer or cervical cancer, the free fatty acid used in the evaluation is preferably the twelve types of the free fatty acids.

In a case where the gynecological cancer is endometrial cancer, the free fatty acid used for the evaluation is preferably one or more selected from the group consisting of palmitic acid, palmitoleic acid, linoleic acid, α-linolenic acid, oleic acid, vaccenic acid, stearic acid, and arachidic acid. The free fatty acid used for the evaluation may be three or more types, four or more types, five or more types, six or more types, seven or more types, or eight types of the free fatty acids. The free fatty acid used for the evaluation can be arbitrarily selected from these free fatty acids.

In a case where the gynecological cancer is endometrial cancer, the free fatty acid used in the evaluation is preferably the twelve types of the free fatty acids.

In a case where the gynecological cancer is cervical cancer, the free fatty acid used for the evaluation is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for the evaluation may be three or more types, four or more types, five or more types, six or more types, seven or more types, eight or more types, nine or more types, ten or more types, eleven or more types, or twelve types of the free fatty acids. The free fatty acid used for the evaluation can be arbitrarily selected from these free fatty acids. In a case where the gynecological cancer is cervical cancer, the free fatty acid used in the evaluation is preferably the twelve types of the free fatty acids.

The "predetermined threshold value" in the (i2) and (ii2) is a value set for each free fatty acid. The predetermined threshold value of the concentration of each free fatty acid may be a cut-off value obtained by acquiring the concentration of each free fatty acid from a plurality of samples derived from gynecological cancer patients which have had good prognosis and a plurality of samples derived from gynecological cancer patients which have had poor prognosis, and statistically processing the concentrations of each free fatty acid thereof.

The step (B2) may be a step of calculating an FFA score, and evaluating the possibility that the subject has poor prognosis of the gynecological cancer to be high in a case where the FFA score is higher than a predetermined threshold value. The FFA score is calculated by the same method as the method described in the first aspect. That is, the FFA score is calculated as a total value of the scores of free fatty acids set in the same manner as in the (i-1) and (ii-1).

In the step (B2), the predetermined threshold value set in the same manner as in the (i-1) and (ii-1) may be acquired as described above, or the same threshold value as that used in the test method according to the first aspect may be used.

The predetermined threshold value for the FFA score may be a cut-off value obtained by calculating the FFA score for a plurality of samples derived from gynecological cancer patients which have had good prognosis and a plurality of samples derived from gynecological cancer patients which have had poor prognosis, and statistically processing the FFA scores. Alternatively, the FFA score may be calculated for the plurality of samples derived from gynecological cancer patients, and the median value of the FFA scores may be used as the threshold value of the FFA score.

The predetermined threshold value for the FFA score depends on the number of types of free fatty acids used for calculating the FFA score, and examples thereof include 0.4n or more and 0.8n or less, 0.5n or more and 0.7n or less, 0.5n or more and 0.6n or less, and the like in a case where the number of types of free fatty acids is represented by n. Examples of the number of types of free fatty acids used for calculating the FFA score include 3 or more, 4 or more, 5 or more, 6 or more, 7 or more, 8 or more, 9 or more, and 10 of free fatty acids among the free fatty acids of the group A2. "0.4n" means a value obtained by multiplying 0.4 by the number n of types. The same applies to other numerical values. For example, in a case where the number of types of free fatty acids is five, "0.4 n or more and 0.8 n or less" means "2 or more and 4 or less".

In a case where the gynecological cancer is ovarian cancer, the free fatty acid used for calculating the FFA score is preferably one or more selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The free fatty acid used for calculating the FFA score may be three or more types, four or more types, five or more types, six or more types, seven or more types, eight or more types, nine or more types, or ten types of the free fatty acids. The free fatty acid used for calculating the FFA score can be arbitrarily selected from these free fatty acids. In a case where the gynecological cancer is ovarian cancer, it is preferable to use the ten types of free fatty acids for calculating the FFA score.

Examples of the predetermined threshold value for the FFA score include the same ones as the predetermined threshold value for the FFA described above in the step (B2). In a case where the ten types of free fatty acids are used, examples of the predetermined threshold value of the FFA score include a value greater than 4 and less than 6.

In a case where the gynecological cancer is an early gynecological malignant tumor, the free fatty acid used for calculating the FFA score is preferably one or more selected from the group consisting of palmitoleic acid, linoleic acid, α-linolenic acid, oleic acid, vaccenic acid, arachidic acid, docosahexaenoic acid, and lignoceric acid. The free fatty acid used for calculating the FFA score may be three or more types, four or more types, five or more types, six or more types, seven or more types, or eight types of the free fatty acids. The free fatty acid used for calculating the FFA score can be arbitrarily selected from these free fatty acids. In a case of intending to detect early gynecological malignant tumors, it is preferable that the eight types of free fatty acids is used for calculating the FFA score.

Examples of the predetermined threshold value for the FFA score include the same ones as the predetermined threshold value for the FFA described above in the step (B2). In a case where the eight types of free fatty acids are used, examples of the predetermined threshold value of the FFA score include greater than 3 and less than 5.

In a case where the gynecological cancer is endometrial cancer or cervical cancer, the free fatty acid used for calculating the FFA score is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for calculating the FFA score can be arbitrarily selected from these free fatty acids. The number of types of free fatty acids used for calculating the FFA score and the predetermined threshold value of the FFA score can be appropriately set. In a case where the gynecological cancer is endometrial cancer or cervical cancer, it is preferable to use the twelve types of free fatty acids for calculating the FFA score.

Examples of the predetermined threshold value for the FFA score include the same ones as the predetermined threshold value for the FFA described above in the step (B2).

In a case where the gynecological cancer is endometrial cancer, the free fatty acid used for calculating the FFA score is preferably one or more selected from the group consisting of palmitic acid, palmitoleic acid, linoleic acid, α-linolenic acid, oleic acid, vaccenic acid, stearic acid, and arachidic acid. The free fatty acid used for calculating the FFA score may be three or more types, four or more types, five or more types, six or more types, seven or more types, or eight types of the free fatty acids. The free fatty acid used for calculating the FFA score can be arbitrarily selected from these free fatty acids. The number of types of free fatty acids used for calculating the FFA score and the predetermined threshold value of the FFA score can be appropriately set. In a case where the gynecological cancer is endometrial cancer, it is preferable to use the eight types of free fatty acids for calculating the FFA score.

Examples of the predetermined threshold value for the FFA score include the same ones as the predetermined threshold value for the FFA described above in the step (B2).

In a case where the gynecological cancer is cervical cancer, the free fatty acid used for calculating the FFA score is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for calculating the FFA score can be arbitrarily selected from these free fatty acids. The number of types of free fatty acids used for calculating the FFA score and the predetermined threshold value of the FFA score can be appropriately set. In a case where the gynecological cancer is cervical cancer, it is preferable to use the ten types of free fatty acids for calculating the FFA score.

Examples of the predetermined threshold value for the FFA score include the same ones as the predetermined threshold value for the FFA described above in the step (B2).

In the test method according to the second aspect, in a case where the FFA score is used, specific examples of the step (A2) and the step (B2) include the following.

In the step (A2), the concentrations of ten types of free fatty acids consisting of palmitoleic acid, linoleic acid, α-linolenic acid, oleic acid, vaccenic acid, arachidic acid, docosahexaenoic acid, adrenic acid, nervonic acid, and lignoceric acid in the sample derived from the subject.

In the step (B2), the possibility that the subject has poor prognosis is evaluated to be high in a case where the FFA score of the ten types of free fatty acids consisting of palmitoleic acid, linoleic acid, α-linolenic acid, oleic acid, vaccenic acid, arachidic acid, docosahexaenoic acid, adrenic acid, nervonic acid, and lignoceric acid is higher than a predetermined threshold value.

The predetermined threshold value for the FFA score may be a cut-off value obtained by calculating the FFA score for ten types of the free fatty acids derived from gynecological cancer patients which have had good prognosis and ten types of the free fatty acids derived from gynecological cancer patients which have had poor prognosis, and statistically processing the FFA scores. Alternatively, the FFA score may be calculated for the plurality of samples derived from gynecological cancer patients, and the median value of the FFA scores may be used as the threshold value of the FFA score.

According to the sample test method according to the second aspect described above, it is possible to predict whether or not the subject has poor prognosis. Therefore, it is easy to make an appropriate treatment plan with reference to the test result. In addition, it is easy to predict recurrence after the treatment. In a case where the score is higher than a predetermined threshold value, the possibility that the subject has poor prognosis is evaluate to be high.

For example, for a subject evaluated to have a high possibility of having poor prognosis, a measure such as a change in a treatment method may be taken. In one embodiment, the present invention provides a treatment method for a gynecological cancer, including a step (a2) of specifying a subject having a high possibility of having poor prognosis by the sample test method according to the second aspect, and a step (b2) of changing a treatment method for a gynecological cancer for the subject.

In the step (a2), the sample test method according to the second aspect is performed as described above to specify a subject having a high possibility of having poor prognosis.

In the step (b2), in the subject specified in the step (a2), the treatment method for a gynecological cancer can be reconsidered and the treatment method for a gynecological cancer can be changed. The subject specified in the step (a2) has a possibility of having a poor prognosis even in a case where the previous treatment is continued. Therefore, it is possible to explore a possibility of improving the prognosis by reconsidering the treatment method and changing the treatment method. Examples of the change in the treatment method include a change in the type of anticancer agent, and the like.

The step (B2) may be a step of specifying the sample derived from the subject having a high possibility of having poor prognosis in a case corresponding to at least one selected from the group consisting of the (i2) and (ii2).

### [Sample test method (3)]

The sample test method according to a third aspect is a method of evaluating whether the gynecological cancer of the subject is a gynecological malignant tumor or a gynecological borderline malignant tumor. The test method includes a step (A3) and a step (B3).

The step (A3): a step of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid

The step (B3): a step of evaluating whether the gynecological cancer is a malignant tumor or a borderline malignant tumor based on the concentration of the free fatty acid in the sample, which is obtained in the step (A3)

In the sample test method according to the third aspect, the subject from which the sample to be subjected to the test is derived is a human female having a gynecological cancer. Examples of the gynecological cancer of the subject include those described above in the first aspect. For example, the subject is a human female who has been diagnosed in advance to have a gynecological cancer. Alternatively, the subject may be a subject determined to have a gynecological cancer by the test method according to the first aspect.

The gynecological malignant tumor is preferably at least one selected from the group consisting of an ovarian malignant tumor, a corpus uteri malignant tumor, and a uterine cervix malignant tumor, and more preferably an ovarian malignant tumor. The gynecological borderline malignant tumor is preferably at least one selected from the group consisting of an ovarian borderline malignant tumor, a corpus uteri borderline malignant tumor, and a uterine cervix borderline malignant tumor, and is preferably an ovarian borderline malignant tumor.

The evaluation in the test method according to the third aspect may be, for example, an evaluation of whether the ovarian cancer of the subject is an ovarian malignant tumor or an ovarian borderline malignant tumor. It may be an evaluation of whether the endometrial cancer of the subject is a corpus uteri malignant tumor or a corpus uteri borderline malignant tumor. It may be an evaluation of whether the cervical cancer of the subject is a uterine cervix malignant tumor or a uterine cervix borderline malignant tumor.

Palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid are collectively referred to as "free fatty acids of group A3".

The step (A3) can be performed in the same manner as the step (A1) in the first aspect, except that the sample derived from the subject having a gynecological cancer is used. The measurement may be performed in vitro.

In the step (A3), the number of types of free fatty acids to be measured is only required to be 1 or more selected from the free fatty acids of the group A3, and may be 2 or more, may be 3 or more, may be 4 or more, may be 5 or more, may be 6 or more, may be 7 or more, may be 8 or more, may be 9 or more, may be 10 or more, may be 11 or more, may be 12 or more, may be 13 or more, or may be 14 selected from the free fatty acids of the group A3. The free fatty acid to be measured can be arbitrarily selected from the free fatty acids of the group A3.

In a case where the gynecological cancer is ovarian cancer, in the step (A3), one or more types of free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid may be to be detected. Among the free fatty acids, two or more types, three or more types, four or more types, five or more types, six or more types, seven or more types, eight or more types, nine or more types, or ten types of the free fatty acids can be measured. The free fatty acid to be measured can be arbitrarily selected from these free fatty acids. In a case where the gynecological cancer is ovarian cancer, it is preferable that, in the step (A3), the concentrations of the ten types of free fatty acids are measured.

In a case where the gynecological cancer is endometrial cancer or cervical cancer, in the step (A3), one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid may be to be measured. Among the free fatty acids, two or more types, three or more types, four or more types, five or more types, six or more types, seven or more types, eight or more types, nine or more types, ten or more types, eleven or more types, or twelve types of the free fatty acids can be measured. The free fatty acid to be measured can be arbitrarily selected from these free fatty acids. In a case where the gynecological cancer is endometrial cancer or cervical cancer, it is preferable that, in the step (A3), the concentrations of the twelve types of free fatty acids are measured.

In a case where the gynecological cancer is endometrial cancer, in the step (A3), one or more free fatty acids selected from the group consisting of palmitic acid, palmitoleic acid, linoleic acid, α-linolenic acid, oleic acid, vaccenic acid, stearic acid, and arachidic acid may be to be measured. Among the free fatty acids, two or more types, three or more types, four or more types, five or more types, six or more types, seven or more types, or eight types of the free fatty acids can be measured. The free fatty acid to be measured can be arbitrarily selected from these free fatty acids. In a case where the gynecological cancer is endometrial cancer, it is preferable that, in the step (A3), the concentrations of the eight types of free fatty acids are measured.

In a case where the gynecological cancer is cervical cancer, in the step (A3), one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid may be to be measured. Among the free fatty acids, two or more types, three or more types, four or more types, five or more types, six or more types, seven or more types, eight or more types, nine or more types, ten or more types, eleven or more types, or twelve types of the free fatty acids can be measured. The free fatty acid to be measured can be arbitrarily selected from these free fatty acids. In a case where the cervical cancer is to be detected, it is preferable that, in the step (A3), the concentrations of the twelve types of free fatty acids are measured.

In the step (B3), in a case corresponding to at least one selected from the group consisting of the following (i3) and (ii3), the possibility that the gynecological cancer of the subject is a malignant tumor is evaluated to be high.
(i3) A concentration of at least one free fatty acid selected from the group consisting of palmitoleic acid, linoleic acid, α-linolenic acid, oleic acid, and palmitic acid in the sample is high as compared with a predetermined threshold value.
(ii3) A concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.

The "predetermined threshold value" in the (i3) and (ii3) is a value set for each free fatty acid. The predetermined threshold value of the concentration of each free fatty acid may be a cut-off value obtained by acquiring the concentration of each free fatty acid from a plurality of samples derived from patients having a gynecological malignant tumor and a plurality of samples derived from patients having a gynecological borderline malignant tumor, and statistically processing the concentrations of each free fatty acid thereof.

In a case where the gynecological cancer is ovarian cancer, the free fatty acid used for the evaluation is preferably one or more selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The free fatty acid used for calculating the FFA score may be three or more types, four or more types, five or more types, six or more types, seven or more types, eight or more types, nine or more types, or ten types of the free fatty acids. The free fatty acid used for the evaluation can be arbitrarily selected from these free fatty acids. The free fatty acid used in the evaluation is preferably the ten types of the free fatty acids.

In a case where the gynecological cancer is endometrial cancer or cervical cancer, the free fatty acid used for the evaluation is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for the evaluation may be three or more types, four or more types, five or more types, six or more types, seven or more types, eight or more types, nine or more types, ten or more types, eleven or more types, or twelve types of the free fatty acids. The free fatty acid used for the evaluation can be arbitrarily selected from these free fatty acids. In a case where the gynecological cancer is endometrial cancer or cervical cancer, the free fatty acid used in the evaluation is preferably the twelve types of the free fatty acids.

In a case where the gynecological cancer is endometrial cancer, the free fatty acid used for the evaluation is preferably one or more selected from the group consisting of palmitic acid, palmitoleic acid, linoleic acid, α-linolenic acid, oleic acid, vaccenic acid, stearic acid, and arachidic acid. The free fatty acid used for calculating the FFA score may be three or more types, four or more types, five or more types, six or more types, seven or more types, or eight types of the free fatty acids. The free fatty acid used for the evaluation can be arbitrarily selected from these free fatty acids. The free fatty acid used in the evaluation is preferably the eight types of the free fatty acids.

In a case where the gynecological cancer is cervical cancer, the free fatty acid used for the evaluation is preferably one or more selected from the group consisting of palmitic acid, palmitoleic acid, linoleic acid, α-linolenic acid, oleic acid, vaccenic acid, stearic acid, and arachidic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for the evaluation can be arbitrarily selected from these free fatty acids. The free fatty acid used in the evaluation is preferably the twelve types of the free fatty acids.

The step (B3) may be a step of calculating an FFA score, and evaluating the possibility that the gynecological cancer of the subject is a malignant tumor to be high in a case where the FFA score is higher than a predetermined threshold value. The FFA score is calculated by the same method as the method described in the first aspect. That is, the FFA score is calculated as a total value of the scores of free fatty acids set in the same manner as in the (i-1) and (ii-1).

In the step (B3), the predetermined threshold value set in the same manner as in the (i-1) and (ii-1) may be acquired as described above, or the same threshold value as that used in the test method according to the first aspect may be used.

The predetermined threshold value for the FFA score may be a cut-off value obtained by calculating the FFA score for a plurality of samples derived from patients having a gynecological malignant tumor and a plurality of samples derived from patients having a gynecological borderline malignant tumor, and statistically processing the FFA scores.

The predetermined threshold value for the FFA score depends on the number of types of free fatty acids used for calculating the FFA score, and examples thereof include 0.2n or more and 0.6n or less, 0.3n or more and 0.6n or less, 0.4n or more and 0.6n or less, and the like in a case where the number of types of free fatty acids is represented by n. Examples of the number of types of free fatty acids used for calculating the FFA score include 3 or more, 4 or more, 5 or more, 6 or more, 7 or more, 8 or more, 9 or more, and 10 of free fatty acids among the free fatty acids of the group A3. The free fatty acid used for calculating the FFA score can be arbitrarily selected from the free fatty acids of the group A3.

In a case where the gynecological cancer is ovarian cancer, the free fatty acid used for calculating the FFA score is preferably one or more selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The free fatty acid used for calculating the FFA score may be three or more types, four or more types, five or more types, six or more types, seven or more types, eight or more types, nine or more types, or ten types of the free fatty acids. The free fatty acid used for calculating the FFA score can be arbitrarily selected from these free fatty acids. In a case where the gynecological cancer is ovarian cancer, it is preferable to use the ten types of free fatty acids for calculating the FFA score. In a case where the ten types of free fatty acids are used, examples of the predetermined threshold value of the FFA score include a value greater than 4 and less than 6.

In a case where the gynecological cancer is endometrial cancer or cervical cancer, the free fatty acid used for calculating the FFA score is preferably one or more selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for calculating the FFA score can be arbitrarily selected from these free fatty acids. In a case where the gynecological cancer is endometrial cancer or cervical cancer, it is preferable to use the twelve types of free fatty acids for calculating the FFA score. In a case where the twelve types of free fatty acids are used, examples of the predetermined threshold value of the FFA score include the same as described above.

In a case where the gynecological cancer is endometrial cancer, the free fatty acid used for calculating the FFA score is preferably one or more selected from the group consisting of palmitic acid, palmitoleic acid, linoleic acid, α-linolenic acid, oleic acid, vaccenic acid, stearic acid, and arachidic acid. The free fatty acid used for calculating the FFA score may be three or more types, four or more types, five or more types, six or more types, seven or more types, or eight types of the free fatty acids. The free fatty acid used for calculating the FFA score can be arbitrarily selected from these free fatty acids. In a case where the gynecological cancer is endometrial cancer, it is preferable to use the eight types of free fatty acids for calculating the FFA score. In a case where the eight types of free fatty acids are used, examples of the predetermined threshold value of the FFA score include the same as described above.

In a case where the gynecological cancer is cervical cancer, the free fatty acid used for calculating the FFA score is preferably one or more selected from the group consisting of palmitic acid, palmitoleic acid, linoleic acid, α-linolenic acid, oleic acid, vaccenic acid, stearic acid, and arachidic acid. The free fatty acid used for calculating the FFA score may be three or more types, four or more types, five or more types, six or more types, seven or more types, eight or more types, nine or more types, ten or more types, eleven or more types, twelve types of the free fatty acids. The free fatty acid used for calculating the FFA score can be arbitrarily selected from these free fatty acids. In a case where the gynecological cancer is cervical cancer, it is preferable to use the twelve types of free fatty acids for calculating the FFA score. In a case where the twelve types of free fatty acids are used, examples of the predetermined threshold value of the FFA score include the same as described above.

In the test method according to the third aspect, in a case where the FFA score is used, specific examples of the step (A3) and the step (B3) include the following.

In the step (A3), the concentrations of ten types of free fatty acids consisting of palmitoleic acid, linoleic acid, α-linolenic acid, oleic acid, vaccenic acid, arachidic acid, docosahexaenoic acid, adrenic acid, nervonic acid, and lignoceric acid in the sample derived from the subject.

In the step (B3), the possibility that the gynecological cancer of the subject is a malignant tumor is determined to be high in a case where the FFA score of ten types of free fatty acids consisting of palmitoleic acid, linoleic acid, α-linolenic acid, oleic acid, vaccenic acid, arachidic acid, docosahexaenoic acid, adrenic acid, nervonic acid, and lignoceric acid is higher than a predetermined threshold value.

In this case, the threshold value of the total score is preferably 4 or more and 6 or less and more preferably 4.5 or more and 5 or less.

According to the sample test method according to a third aspect described above, it is possible to evaluate whether the gynecological cancer of the subject is a gynecological malignant tumor or a gynecological borderline malignant tumor.

The borderline malignant tumor is a tumor having an intermediate property between a malignant tumor and a benign tumor. Since the ovarian borderline malignant tumor does not have hematogenous and lymphogenous metastasis like malignant tumors, the prognosis is not as poor as malignant tumors. That is, the ovarian borderline malignant tumor has a clinical feature that, although the lesion may occasionally spread in the abdominal cavity, there is no metastasis. In addition, between the borderline malignant tumor and the malignant tumor, the proliferation speed of the tumor is generally different. Since malignant tumors increase rapidly, and have disseminate and metastasis, a surgical treatment as soon as possible is required. On the other hand, the borderline malignant tumor has a slow tumor proliferation, and there is little need for emergency surgery.

Due to the difference in the above-described features, it is extremely useful to identify whether the tumor is a borderline malignant tumor or a malignant tumor by a blood test in a non-invasive manner before the treatment (at the time of diagnosis) in determining a treatment policy (waiting time until surgery, surgical procedure, degree of surgical invasion, and the like).

Currently, the type of tumor is identified by a pathological diagnosis during surgery, but according to the sample test method according to the third aspect, it is possible to evaluate whether the gynecological cancer of the subject is a borderline malignant tumor or a malignant tumor in a non-invasive manner before surgery.

In one embodiment, the present invention provides a treatment method for a gynecological cancer, including a step (a3) of specifying a subject having a high possibility of having a gynecological borderline malignant tumor or a subject having a high possibility of having a gynecological malignant tumor, by the sample test method according to a third aspect, and a step (b3) of performing a treatment for a gynecological borderline malignant tumor on the subject having a high possibility of having a gynecological borderline malignant tumor and performing a treatment for a gynecological malignant tumor on the subject having a high possibility of having a gynecological malignant tumor.

In the step (a3), the sample test method according to the third aspect is performed as described above to specify a subject having a high possibility of having a gynecological borderline malignant tumor or a subject having a high possibility of having a gynecological malignant tumor.

In the step (b3), in a case where the subject specified in the step (a3) has a high possibility of having a gynecological borderline malignant tumor, treatment for the gynecological borderline malignant tumor is performed. Examples of the treatment for the gynecological borderline malignant tumor include surgical treatment, and it is preferable to perform resection of intraperitoneal organs (that is, uterus, ovary, fallopian tube, and greater omentum), and it is more preferable to perform resection of only intraperitoneal organs.

In addition, in a case where a possibility that the subject has a gynecological malignant tumor is high, treatment of the gynecological malignant tumor is performed. Examples of the treatment of the gynecological malignant tumor include surgical treatment, and resection of intraperitoneal organs and lymphadenectomy are preferable.

The step (B3) may be a step of specifying the sample derived from the subject having a high possibility that the gynecological cancer is a malignant tumor in a case corresponding to at least one selected from the group consisting of the (i3) and (ii3).

### [Kit]

A test kit of a gynecological cancer and precancerous lesions thereof according to the fourth aspect includes a reagent for measuring one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid.

The kit according to the present aspect is used for at least one purpose selected from the group consisting of the following (a) to (d):
(a) evaluation of possibility that the subject has one or more selected from the group consisting of a gynecological cancer and precancerous lesions thereof;
(b) evaluation of a possibility that a subject having a gynecological cancer has poor prognosis;
(c) evaluation of whether a gynecological cancer of a subject is a malignant tumor or a borderline malignant tumor; and
(d) evaluation of whether a subject having a high possibility of having one or more selected from the group consisting of a gynecological cancer and a gynecological precancerous lesion has any of a gynecological cancer and a gynecological precancerous lesion.

The subject in the (a) is a human female. Examples of the gynecological cancer which the subject can have include those described above in the first aspect. The gynecological cancer that the subject can have is preferably a gynecological malignant tumor. As the gynecological malignant tumor, one or more selected from the group consisting of an ovarian malignant tumor, a corpus uteri malignant tumor, and a uterine cervix malignant tumor are preferable, and an ovarian malignant tumor is more preferable. As the gynecological borderline malignant tumor, one or more selected from the group consisting of an ovarian borderline malignant tumor, a corpus uteri borderline malignant tumor, and a uterine cervix borderline malignant tumor are preferable, and an ovarian borderline malignant tumor is more preferable.

Examples of the gynecological precancerous lesion which the subject can have include those described above in the first aspect.

The subject in the (b) is a human female having a gynecological cancer. Examples of the gynecological cancer include the cancer described above in the first aspect. As the gynecological cancer, a gynecological malignant tumor is preferable, one or more selected from the group consisting of an ovarian malignant tumor, a corpus uteri malignant tumor, and a uterine cervix malignant tumor is more preferable, and an ovarian malignant tumor is still more preferable.

The subject in the (c) is a human female having a gynecological cancer. Examples of the gynecological cancer include the cancer described above in the first aspect. The gynecological malignant tumor is preferably at least one selected from the group consisting of an ovarian malignant tumor, a corpus uteri malignant tumor, and a uterine cervix malignant tumor, and more preferably an ovarian malignant tumor. The gynecological borderline malignant tumor is preferably at least one selected from the group consisting of an ovarian borderline malignant tumor, a corpus uteri borderline malignant tumor, and a uterine cervix borderline malignant tumor, and is preferably an ovarian borderline malignant tumor.

The kit according to the present aspect is suitably used for the test method according to the first to third aspects.

The reagent for measuring a free fatty acid is a reagent used for measuring the concentration of a free fatty acid in a sample derived from a subject. The reagent may be a reagent that specifically bonds any one of free fatty acids of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid (free fatty acids of group A1). For example, the reagent for measuring the free fatty acid may be an antibody, an aptamer, or the like that specifically binds to a specific free fatty acid.

In a case where the free fatty acid in the sample is measured using an analysis instrument such as gas chromatography mass spectrometry, liquid chromatography mass spectrometry, or gas chromatography, examples of a reagent for measuring the concentration of the free fatty acid include a standard sample of any of the above-described free fatty acids. The standard sample is used for creating a calibration curve in the measurement of the free fatty acid by the analysis instrument as described above. The concentration of the free fatty acid in the sample can be calculated based on a calibration curve created by the standard sample.

The standard sample of the free fatty acid may be the purified product of the free fatty acid. The purity of the specific free fatty acid in the standard sample of the specific free fatty acid is, for example, 96% by mass or more, 97% by mass or more, 98% by mass or more, or 99% by mass or more.

The test kit according to the present embodiment is only required to contain a reagent for measuring one or more types of any free fatty acids of the group A1, and may contain a reagent for measuring two or more types, three or more types, four or more types, five or more types, six or more types, seven or more types, eight or more types, nine or more types, ten or more types, eleven or more types, twelve or more types, thirteen or more types, or fourteen or more types of any free fatty acids of the group A1. The fatty acid to be measured by the reagent that can be included in the test kit according to the present embodiment, can be arbitrarily selected from the free fatty acids of the group A1. Alternatively, the test kit according to the present embodiment may include a reagent for measuring all of the 15 types of free fatty acids of the group A1.

In a case where the test kit according to the present embodiment is a test kit for a gynecological cancer, it is preferable that the test kit includes a reagent for measuring one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid. The kit according to the present embodiment can include a reagent for measuring three or more, four or more, five or more, six or more, seven or more, eight or more, nine or more, ten or more, eleven or more, twelve or more, fourteen or more, or fourteen types of the free fatty acids. The fatty acid to be measured by the reagent that can be included in the test kit according to the present embodiment, can be arbitrarily selected from these free fatty acids.

In a case where the test kit according to the present embodiment is a test kit for ovarian cancer, it is preferable that the test kit includes a reagent for measuring one or more free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The kit according to the present embodiment can include a reagent for measuring three or more, four or more, five or more, six or more, seven or more, eight or more, nine or more, or ten types of the free fatty acids. The fatty acid to be measured by the reagent that can be included in the test kit according to the present embodiment, can be arbitrarily selected from these free fatty acids.

In a case where the test kit according to the present embodiment is used to detect also an early gynecological cancer (for example, Stage I and Stage II), it is preferable that the test kit includes a reagent for measuring one or more free fatty acids selected from the group consisting of palmitoleic acid, linoleic acid, α-linolenic acid, oleic acid, vaccenic acid, arachidic acid, docosahexaenoic acid, and lignoceric acid. The kit according to the present embodiment can include a reagent for measuring three or more, four or more, five or more, six or more, seven or more, or eight types of the free fatty acids. The fatty acid to be measured by the reagent that can be included in the test kit according to the present embodiment, can be arbitrarily selected from these free fatty acids.

In a case where the test kit according to the present embodiment is a test kit for endometrial cancer or cervical cancer, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The kit according to the present embodiment can include a reagent for measuring three or more, four or more, five or more, six or more, seven or more, eight or more, nine or more, ten or more, eleven or more, or twelve types of the free fatty acids. The fatty acid to be measured by the reagent that can be included in the test kit according to the present embodiment, can be arbitrarily selected from these free fatty acids.

In a case where the test kit according to the present embodiment is a test kit for endometrial cancer, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, and arachidic acid. The kit according to the present embodiment can include a reagent for measuring three or more, four or more, five or more, six or more, seven or more, or eight types of the free fatty acids. The fatty acid to be measured by the reagent that can be included in the test kit according to the present embodiment, can be arbitrarily selected from these free fatty acids.

In a case where the test kit according to the present embodiment is a test kit for a cervical cancer, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The kit according to the present embodiment can include a reagent for measuring three or more, four or more, five or more, six or more, seven or more, eight or more, nine or more, ten or more, eleven or more, or twelve types of the free fatty acids. The fatty acid to be measured by the reagent that can be included in the test kit according to the present embodiment, can be arbitrarily selected from these free fatty acids.

In a case where the test kit according to the present embodiment is a test kit used in the evaluation of the (b), it is preferable that the test kit includes a reagent for measuring one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid. The kit according to the present embodiment can include a reagent for measuring three or more, four or more, five or more, six or more, seven or more, eight or more, nine or more, ten or more, eleven or more, twelve or more, thirteen or more, or fourteen types of the free fatty acids. The fatty acid to be measured by the reagent that can be included in the test kit according to the present embodiment, can be arbitrarily selected from these free fatty acids.

In a case where the test kit according to the present embodiment is an evaluation test kit for a possibility that the subject having ovarian cancer is poor prognosis, it is preferable that the test kit includes a reagent for measuring one or more free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The kit according to the present embodiment can include a reagent for measuring three or more, four or more, five or more, six or more, seven or more, eight or more, nine or more, or ten types of the free fatty acids. The fatty acid to be measured by the reagent that can be included in the test kit according to the present embodiment, can be arbitrarily selected from these free fatty acids.

In a case where the test kit according to the present embodiment is an evaluation test kit for a possibility that the subject having endometrial cancer or cervical cancer is poor prognosis, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The kit according to the present embodiment can include a reagent for measuring three or more, four or more, five or more, six or more, seven or more, eight or more, nine or more, ten or more, eleven or more, or twelve types of the free fatty acids. The fatty acid to be measured by the reagent that can be included in the test kit according to the present embodiment, can be arbitrarily selected from these free fatty acids.

In a case where the test kit according to the present embodiment is an evaluation test kit for a possibility that the subject having endometrial cancer is poor prognosis, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, and arachidic acid. The kit according to the present embodiment can include a reagent for measuring three or more, four or more, five or more, six or more, seven or more, or eight types of the free fatty acids. The fatty acid to be measured by the reagent that can be included in the test kit according to the present embodiment, can be arbitrarily selected from these free fatty acids.

In a case where the test kit according to the present embodiment is an evaluation test kit for a possibility that the subject having cervical cancer is poor prognosis, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The kit according to the present embodiment can include a reagent for measuring three or more, four or more, five or more, six or more, seven or more, eight or more, nine or more, ten or more, eleven or more, or twelve types of the free fatty acids. The fatty acid to be measured by the reagent that can be included in the test kit according to the present embodiment, can be arbitrarily selected from these free fatty acids.

In a case where the test kit according to the present embodiment is a test kit used in the evaluation of the (c), it is preferable that the test kit includes a reagent for measuring one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid. The kit according to the present embodiment can include a reagent for measuring three or more, four or more, five or more, six or more, seven or more, eight or more, nine or more, ten or more, eleven or more, twelve or more, thirteen or more, or fourteen types of the free fatty acids. The fatty acid to be measured by the reagent that can be included in the test kit according to the present embodiment, can be arbitrarily selected from these free fatty acids.

In a case where the test kit according to the present embodiment is an evaluation test kit for an evaluation possibility of whether the ovarian cancer of the subject is a malignant tumor or a borderline malignant tumor, it is preferable that the test kit includes a reagent for measuring one or more free fatty acids selected from the group consisting of palmitoleic acid, linoleic acid, α-linolenic acid, oleic acid, vaccenic acid, arachidic acid, docosahexaenoic acid, adrenic acid, nervonic acid, and lignoceric acid. The kit according to the present embodiment can include a reagent for measuring three or more, four or more, five or more, six or more, seven or more, eight or more, nine or more, or ten types of the free fatty acids. The fatty acid to be measured by the reagent that can be included in the test kit according to the present embodiment, can be arbitrarily selected from these free fatty acids.

In a case where the test kit according to the present embodiment is an evaluation test kit for an evaluation possibility of whether the endometrial cancer or the cervical cancer of the subject is a malignant tumor or a borderline malignant tumor, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The kit according to the present embodiment can include a reagent for measuring three or more, four or more, five or more, six or more, seven or more, eight or more, nine or more, ten or more, eleven or more, or twelve types of the free fatty acids.

The fatty acid to be measured by the reagent that can be included in the test kit according to the present embodiment, can be arbitrarily selected from these free fatty acids.

In a case where the test kit according to the present embodiment is an evaluation test kit for an evaluation possibility of whether the endometrial cancer of the subject is a malignant tumor or a borderline malignant tumor, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, and arachidic acid. The kit according to the present embodiment can include a reagent for measuring three or more, four or more, five or more, six or more, seven or more, or eight types of the free fatty acids. The fatty acid to be measured by the reagent that can be included in the test kit according to the present embodiment, can be arbitrarily selected from these free fatty acids.

In a case where the test kit according to the present embodiment is an evaluation test kit for an evaluation possibility of whether the cervical cancer of the subject is a malignant tumor or a borderline malignant tumor, it is preferable that the test kit includes a reagent for measuring one or more free fatty acids selected from the group consisting of palmitic acid, palmitoleic acid, linoleic acid, α-linolenic acid, oleic acid, vaccenic acid, stearic acid, arachidic acid, dihomo-γ-linolenic acid, docosahexaenoic acid, adrenic acid, and docosapentaenoic acid. The kit according to the present embodiment can include a reagent for measuring three or more, four or more, five or more, six or more, seven or more, eight or more, nine or more, ten or more, eleven or more, or twelve types of the free fatty acids. The fatty acid to be measured by the reagent that can be included in the test kit according to the present embodiment, can be arbitrarily selected from these free fatty acids.

In a case where the kit according to the present embodiment is a test kit used for the evaluation of the (d), it is preferable that the kit includes a reagent for measuring one or more free fatty acids selected from the group consisting of dihomo-γ-linolenic acid, docosahexaenoic acid, adrenic acid, and docosapentaenoic acid. The kit according to the present embodiment can include a reagent for measuring two or more, three or more, or four types of the free fatty acids. The fatty acid to be measured by the reagent that can be included in the test kit according to the present embodiment, can be arbitrarily selected from these free fatty acids.

In a case where the free fatty acid in the sample is measured using an analysis instrument such as gas chromatography mass spectrometry, liquid chromatography mass spectrometry, or gas chromatography, the kit according to the present embodiment may be include an internal standard substance. The internal standard substance is used by being added to a sample derived from a subject. The concentration of the free fatty acid in the sample can be calculated from the addition amount of the internal standard substance added to the sample derived from the subject, and the ratio of the measured value of the free fatty acid to the measured value of the internal standard substance in the sample.

As the internal standard substance, a substance that is not contained in the sample derived from the subject can be used. For example, a free fatty acid known to be absent in the human body can be used as the internal standard substance. The internal standard substance is preferably a substance having the same extraction rate as the free fatty acid to be measured with respect to the solvent used in the extraction of the free fatty acid from the sample derived from the subject. Examples of such an internal standard substance include margaric acid, and the like.

By using the kit according to the present embodiment, it is possible to easily measure the above-described concentration of free fatty acids in the sample derived from the subject. As a result, it is possible to easily obtain the evaluation result of the test method according to the first to third aspects.

### [Medicine]

The medicine according to the present aspect is a medicine for treating or preventing a gynecological cancer, which is administered to a subject evaluated to have a gynecological cancer by the test method according to the first aspect.

Examples of the gynecological cancer include the cancer described above in the first aspect. As the gynecological cancer, a gynecological malignant tumor is preferable, a malignant tumor selected from the group consisting of an ovarian malignant tumor, a corpus uteri malignant tumor, and a uterine cervix malignant tumor is more preferable, and an ovarian malignant tumor is still more preferable.

The medicine according to the present aspect is not particularly limited as long as it is effective against a gynecological cancer. Examples of the medicine for gynecological cancer include a known anticancer agent used for the treatment of the gynecological cancer. Examples of such an anticancer agent include carboplatin, cisplatin, paclitaxel, docetaxel, irinotecan, doxorubicin, topotecan, gemcitabine, etoposide, trabectedin, Abraxane, tamoxifen, ifosfamide, 5-FU, doxorubicin, and the like. Alternatively, the medicine according to the present embodiment may be an antibody medicine, and examples thereof include a known antibody medicine. Examples of the antibody that can be contained in the antibody medicine include an anti-VEGF antibody such as bevacizumab, and the like.

The medicine according to the present aspect may include various substances generally used for formulation, for example, an excipient, a stabilizer, other pharmaceutically acceptable components, other pharmaceutical active components, or the like.

The medicine according to the present aspect may be administered orally or parenterally. The medicine according to the present embodiment can take various formulation forms such as a solid preparation and a liquid preparation, depending on the administration route. For example, the medicine can be an oral preparation such as a solid agent for oral administration or a liquid agent for oral administration, or a non-oral preparation such as an injection agent or a drip agent. Examples of the carrier that can be used in the non-oral preparation include aqueous carriers such as physiological saline, an isotonic solution containing glucose or D-sorbitol, and the like.

The dose of the medicine according to the present aspect varies depending on the age and weight of the subject, the diseases and symptoms to be applied, and the like. For example, the dose can be about 0.1 µg/kg to 1 mg/kg per body weight as a single dose. The administration interval is not particularly limited, and for example, the administration can be performed once a day to once every few months.

### [Other embodiments]

In one aspect, the present disclosure provides a biomarker for evaluating the presence or absence of one or more selected from the group consisting of a gynecological cancer and precancerous lesions thereof, the biomarker being selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid.

In one aspect, the present disclosure provides a biomarker for predicting prognosis of the gynecological cancer patient, which is selected from the group consisting of a gynecological cancer and precancerous lesions thereof, the biomarker being selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid.

In one aspect, the present disclosure provides a biomarker for classifying a gynecological malignant tumor and a gynecological borderline malignant tumor, which is selected from the group consisting of a gynecological cancer and precancerous lesions thereof, the biomarker being selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid.

In one aspect, the present disclosure provides a biomarker for classifying a gynecological cancer and precancerous lesions thereof, the biomarker being selected from the group consisting of dihomo-γ-linolenic acid, docosahexaenoic acid, adrenic acid, and docosapentaenoic acid.

In one aspect, the present disclosure provides a use of a reagent for manufacturing a diagnostic drug for a gynecological cancer and precancerous lesions thereof. The diagnostic drug is used for at least one diagnosis selected from the group consisting of the following (a) to (d):
(a) diagnosis of the possibility that the subject has one or more selected from the group consisting of a gynecological cancer and precancerous lesions thereof,
(b) diagnosis of a possibility that the subject having a gynecological cancer has poor prognosis,
(c) diagnosis of whether a gynecological cancer of the subject is a malignant tumor or a borderline malignant tumor, and
(d) diagnosis of whether the subject having a high possibility of having one or more selected from the group consisting of a gynecological cancer and a gynecological precancerous lesion has any of a gynecological cancer and a gynecological precancerous lesion.

The reagent is a purified product used for measuring a concentration of a free fatty acid in a sample derived from a subject.

The free fatty acid is one or more selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid.

The purified product may be one or more selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid.

The purified product may be two or more types, three or more types, four or more types, five or more types, six or more types, seven or more types, eight or more types, nine or more types, ten or more types, eleven or more types, twelve or more types, thirteen or more types, fourteen or more, or fifteen types of fatty acids among these fatty acids. The purified product is arbitrarily selected from these fatty acids.

Hereinabove, preferable examples of the present invention have been described above, but the present invention is not limited to these examples. Configurations can be added, omitted, and replaced, and other modifications can be made within a range not departing from the gist of the present invention. The present invention is not limited by the above description, but only by the scope of the appended claims.

### Examples

Hereinafter, the present invention will be described with reference to Examples, but the present invention is not limited to the following Examples. In Examples, ovarian cancer, endometrial cancer, and cervical cancer mean ovarian malignant tumor, corpus uteri malignant tumor, and uterine cervix malignant tumor.

### [Experimental Example 1]

### (Expression analysis of fatty acid metabolic enzyme)

The expression levels of the fatty acid metabolic enzymes in the normal ovarian tissue and the ovarian cancer tissue were analyzed by RT-PCR.

Nine examples of normal ovarian tissues were collected for the purpose of treatment (prevention of metastasis and the like) at the time of surgery for a gynecological malignant tumor, and thirty-two examples of the ovarian cancer tissues were collected at the time of surgery for ovarian malignant tumor. Each of the normal ovarian tissues and the ovarian cancer tissues was crushed with a crusher, and total RNA was extracted using an RNeasy Kit (QIAGEN).

cDNA synthesis was performed using ReverTra Ace qPCR RT Master Mix with gDNA Remover (manufactured by Toyobo Co., Ltd.). Specifically, first, total RNA corresponding to 1 µg and RNase free distilled water were mixed to a volume of 12 µL, and the mixture was incubated at 65°C for 5 minutes to denature RNA. Subsequently, 4 µL of 4× DN Master Mix and gDNA Remover were added thereto on ice, and the mixture was reacted at 37°C for 5 minutes to remove the genomic DNA. Subsequently, 4 µL of 5× RT Master Mix II was further mixed therewith, and a reverse transcription reaction (37°C for 15 minutes, 50°C for 5 minutes, and 98°C for 5 minutes) was performed to synthesize cDNA.

Real-time PCR was performed using the cDNA prepared above as a template and using THUNDERBIRD Probe qPCR Mix (manufactured by Toyobo Co., Ltd.).

The real-time PCR was performed according to the 2^{-ΔCt} protocol. That is, a difference in Ct value between the target gene and glyceraldehyde 3-phosphate dehydrogenase (GAPDH) was defined as a ΔCt value, and the relative quantification value was calculated as 2^{-ΔCt}. Each measurement was performed by triplicate.

The target genes were human acetyl-CoA carboxylase 1 (ACACA), fatty acid synthase (FASN), stearoyl-CoA desaturase 1 (SCD1), fatty acid desaturase 1 (FADS1), FADS2, elongation of very long chain fatty acid 1 (ELOVL1), ELOVL2, ELOVL3, ELOVL4, ELOVL5, and ELOVL6.

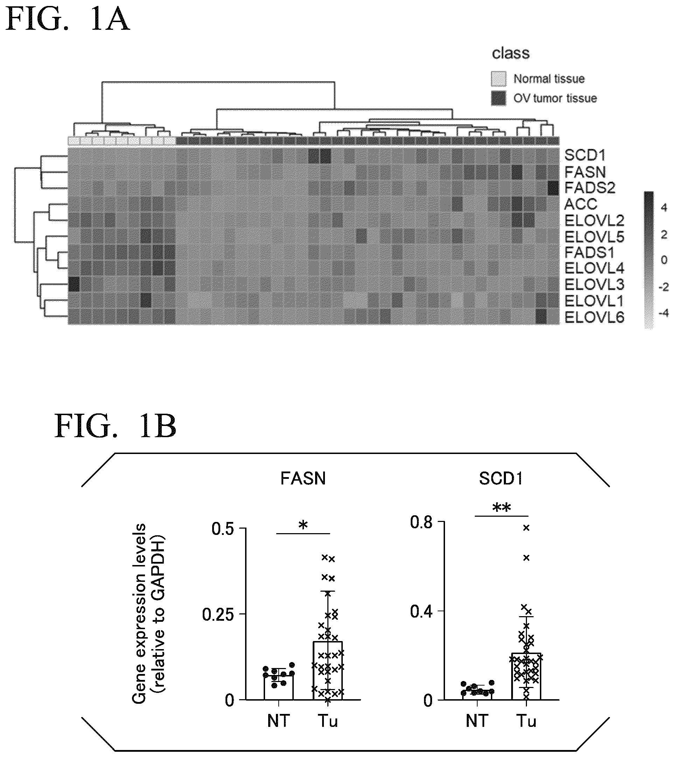

The results are shown in FIGS. 1A to 1D. FIG. 1A is a diagram showing expression levels of free fatty acid metabolic enzymes in the normal ovarian tissue and the ovarian cancer tissue by a heat map. The heat map was prepared using MetaboAnalyst 5.0 software.

FIGS. 1B to 1D are graphs showing the expression levels of each fatty acid metabolic enzyme in the normal ovarian tissue and the ovarian cancer tissue. The comparison between the two groups of the normal ovarian tissue and the ovarian cancer tissue was performed by t-test, and p < 0.05 was considered as a significant difference. "*", "**", "***", and "****" indicate p < 0.05, p < 0.01, p < 0.001, and p < 0.0001.

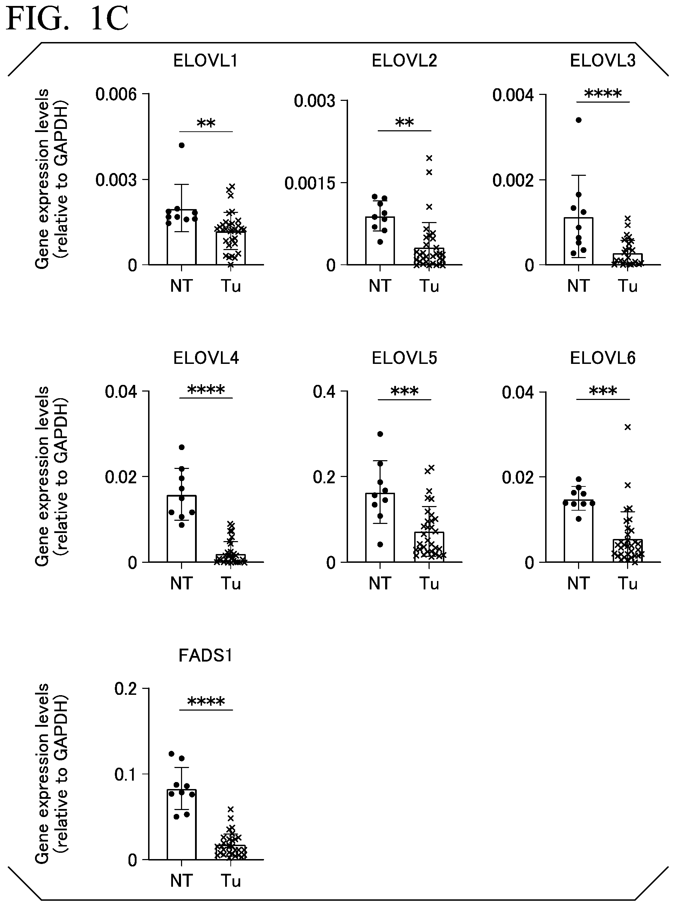

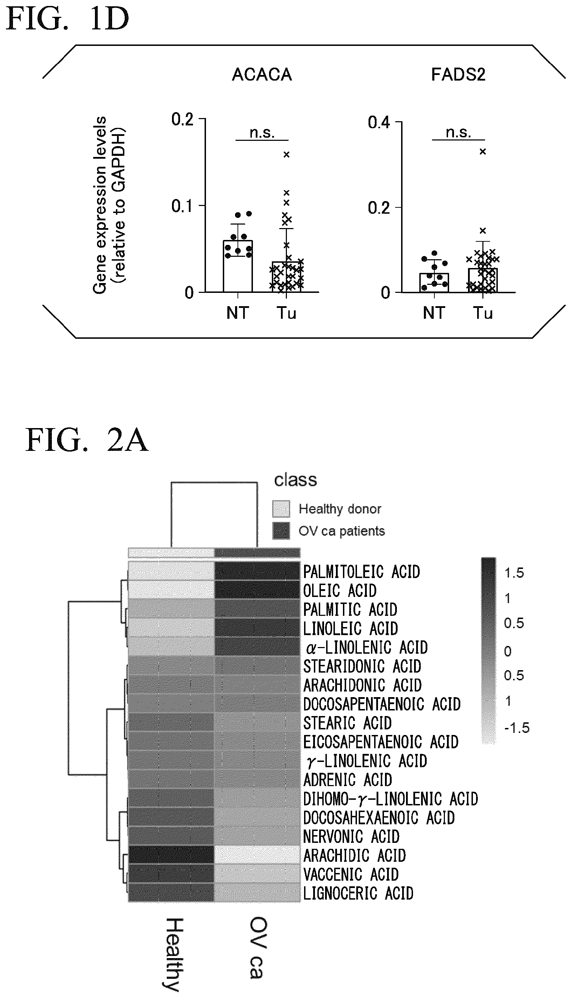

From the results shown in FIG. 1B, it was confirmed that expression levels of FASN and SCD1 in the ovarian cancer tissue were higher than those in the normal ovarian tissue. From the results shown in FIG. 1C, it was confirmed that expression levels of ELOVL1, ELOVL2, ELOVL3, ELOVL4, ELOVL5, ELOVL6, and FADS1 in the ovarian cancer tissue were lower than those in the normal ovarian tissue. From the results shown in FIG. 1D, it was confirmed that expression levels of ACACA and FADS2 in the normal ovarian tissue were same as those in the ovarian cancer tissue. From the above results, it was confirmed that the expression levels of the fatty acid metabolic enzymes were different between the normal ovarian tissue and the ovarian cancer tissue.

### [Experimental Example 2]

### (Concentration of free fatty acid in blood serum of ovarian cancer patient)

The amount of free fatty acids in the blood serum of the healthy female subject and the ovarian cancer patient were analyzed by gas chromatography mass spectrometry (GC-MS).

Blood was collected from 27 healthy adult women and 40 ovarian cancer patients (29 patients with stage I and stage II and 11 patients with stage III and stage IV) to obtain blood serum samples.

First, a calibration curve was created using a standard sample for measuring free fatty acids in blood serum.

Saturated and monosaturated Fatty acid LC-MS mixture (manufactured by Cayman Chemical Co., Inc., #17942), Polyunsaturated Fatty Acid LC-MS Mixture (manufactured by Cayman Chemical Co., Inc., #17941), Sapienic acid (manufactured by Cayman Chemical Co., Inc., #9001845), and vaccenic acid (manufactured by Cayman Chemical Co., Inc., #20023) were used as a standard sample and margaric acid (manufactured by Cayman Chemical Co., Inc., Nacalai Tesque) was used as an internal standard substance.

First, 20 µL of blood serum from a healthy subject and 20 µL of blood serum from an ovarian cancer patient were each diluted with 300 µL of phosphate buffered saline (PBS) containing 100 ng of margaric acid as an internal standard substance. Subsequently, the entire amount of the sample was applied to a diatomite column (product name "SLE+400", manufactured by Biotage AB) and elution was performed with 1.8 mL of dichloromethane.

Subsequently, the eluate was dried and solidified in a nitrogen stream, and the obtained dried product was dissolved in 5 µL of pyridine. 30 µL of BSTFA:TMCS (99:1, Thermo Fisher TS-38831) was added thereto and mixed, and all the OH groups were trimethylsilylated.

Subsequently, 2 µL of the derivatized sample was injected into GC-MS, and the analysis was performed. The quantitative value was obtained by comparing the peak area ratio of the internal standard substance to each of the free fatty acids with a calibration curve created in advance.

The analysis conditions of GC-MS were as follows. As a capillary column, RTx-5MS (length of 30 meters, inner diameter of 0.25 mm, and film thickness of 0.25 µm, manufactured by Restek Corporation) was used. The oven temperature was maintained at 150°C for 1 minute, raised to 250°C at 20°C/min, raised to 280°C at 5°C/min, maintained at 280°C for 3 minutes, raised to 330°C at 20°C/min, and maintained at 330°C for 1 minute. He was used as a carrier gas. The linear velocity was set to 42.0 cm/sec. The ion source temperature, the interface temperature, and the vaporization chamber temperature were set to 200°C, 280°C, and 250°C.

The free fatty acids to be measured were 18 types of palmitoleic acid, palmitic acid, γ-linolenic acid, stearidonic acid, linoleic acid, α-linolenic acid, oleic acid, vaccenic acid, stearic acid, arachidic acid, arachidonic acid, eicosapentaenoic acid, dihomo-γ-linolenic acid, docosahexaenoic acid, adrenic acid, docosapentaenoic acid, nervonic acid, and lignoceric acid.

The measurement results are shown in FIGS. 2A to 2D. FIG. 2A is a diagram showing the average values of the various free fatty acid amounts in the blood serum of healthy subjects and the blood serum of ovarian cancer patients by a heat map. The heat map was prepared using MetaboAnalyst 5.0 software.

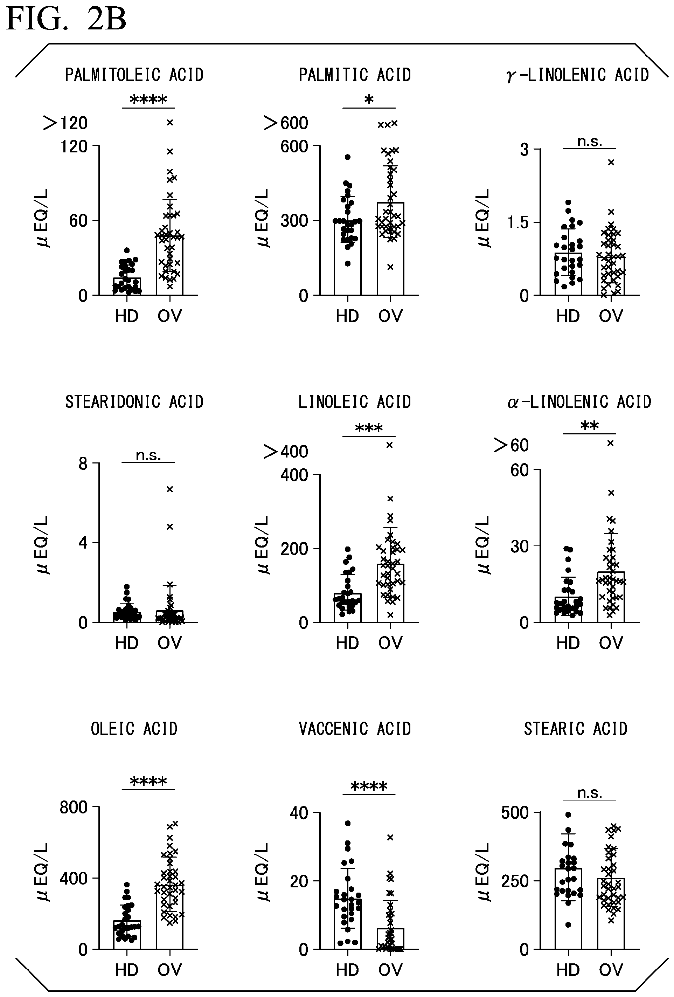

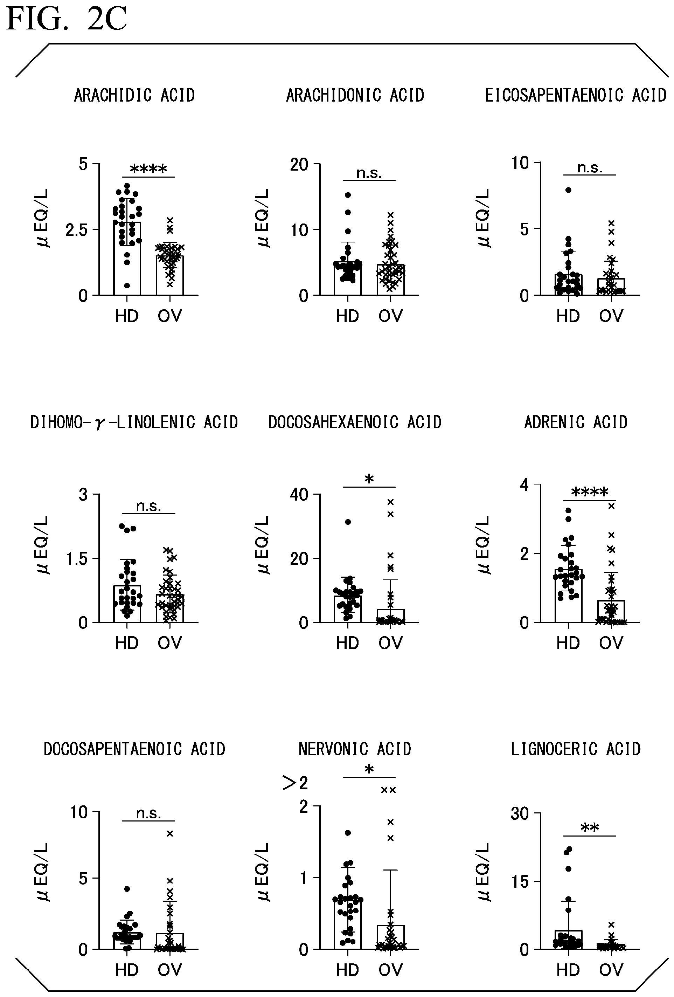

FIGS. 2B and 2C are graphs showing individual numerical values of various free fatty acids in the blood serum of healthy subjects and the blood serum of ovarian cancer patients. The comparison between the two groups was performed by a t-test, and p < 0.05 was considered as a significant difference. "*", "**", "***", and "****" indicate p < 0.05, p < 0.01, p < 0.001, and p < 0.0001.

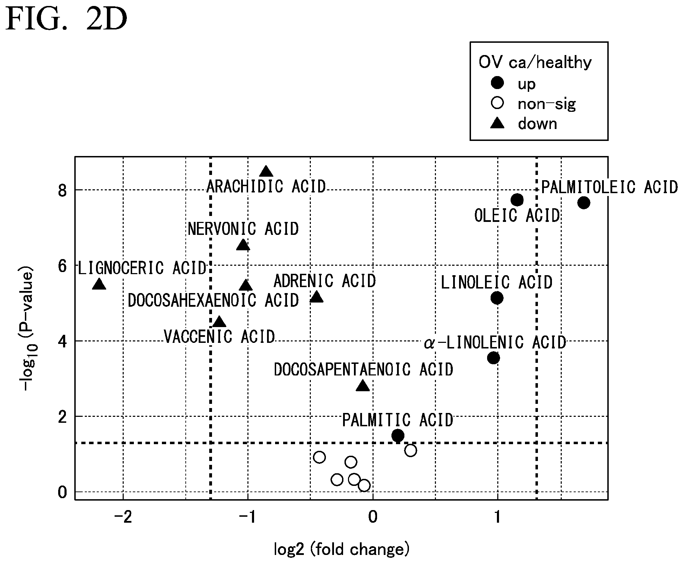

FIG. 2D is a diagram showing the measurement results of each free fatty acid in a volcano plot. In FIG. 2D, "Up" is a free fatty acid in which the concentration in the blood serum of an ovarian cancer patient has been significantly higher than that in the blood serum of a healthy subject. "Down" is a free fatty acid in which the concentration in the blood serum of an ovarian cancer patient has been significantly lower than that in the blood serum of a healthy subject. The "non-sig" is a free fatty acid in which no significant difference in concentration is confirmed between the blood serum of the healthy subject and the blood serum of the ovarian cancer patient. The horizontal axis represents a ratio of the average value of the concentrations of each free fatty acid in the blood serum of the ovarian cancer patient to the average value of the concentrations of each free fatty acid in the blood serum of the healthy subject. The vertical axis represents the P value in a case where there is no difference between the concentration of each free fatty acid in the blood serum of the healthy subject and the concentration of each free fatty acid in the blood serum of the ovarian cancer patient.

From the results shown in FIGS. 2A to 2C, it was confirmed that the concentrations of palmitoleic acid, palmitic acid, linoleic acid, α-linolenic acid, and oleic acid in the blood serum of the ovarian cancer patient were higher than those in the blood serum of the healthy subject. On the contrary, it was confirmed that the concentrations of vaccenic acid, arachidic acid, docosahexaenoic acid, adrenic acid, nervonic acid, and lignoceric acid in the blood serum of the ovarian cancer patient were lower than those in the blood serum of the healthy subject.

Table 1 summarizes the free fatty acids that had a significant difference between the blood serum of the healthy subject and the blood serum of the ovarian cancer patient. In Table 1, the description of "High" in the column of the concentration difference means that the concentration of the free fatty acid in the blood serum of the ovarian cancer patient was significantly higher than that in the blood serum of the healthy subject. In Table 1, the description of "Low" in the column of the concentration difference means that the concentration of the free fatty acid in the blood serum of the ovarian cancer patient was significantly lower than that in the blood serum of the healthy subject.

**[Table 1]**

| Japanese name | English name | Abbreviation | CAS number | Concentration difference |
|---|---|---|---|---|
| Palmitoleic acid | Palmitoleic acid | 16:1 (Δ⁹) | 373-49-9 | High |
| | | FA (16:1) | | |
| Palmitic acid | Palmitic acid | 16:0 | 57-10-3 | High |
| | | FA (16:0) | | |
| Linoleic acid | Linoleic acid | 18:2 (Δ^{9, 12}) | 60-33-3 | High |
| | | FA (18:2) | | |
| α-Linolenic acid | α-linolenic acid | 18:3 (Δ^{9, 12, 15}) | 463-40-1 | High |
| | | FA (18:3) α | | |
| Oleic acid | Oleic acid | 18:1 (Δ⁹-cis) | 112-80-1 | High |
| | | FA (18:1) | | |
| Vaccenic acid | Vaccenic acid | 18:1 (Δ⁷-trans) | 693-72-1 | Low |
| | | FA (18:1) A | | |
| Arachidic acid | Arachidic acid | 20:0 | 506-30-9 | Low |
| | | FA (20:0) | | |
| Docosahexaenoic acid | Docosahexaenoic acid | 22:6 (Δ^{4, 7, 10, 13, 16, 19}) | 6217-54-5 | Low |
| | | FA (22:6) | | |
| Adrenic acid | Adrenic acid | 22:4 (Δ^{7, 10, 13, 16}) | 28874-58-0 | Low |
| | | FA (22:4) | | |
| Nervonic acid | Nervonic acid | 24:1 (Δ¹⁵) | 506-37-6 | Low |
| | | FA (24:1) | | |
| Lignoceric acid | Lignoceric acid | 24:0 | 557-59-5 | Low |
| | | FA (24:0) | | |

### [Experimental Example 3]

### (Correlation analysis between expression level of fatty acid metabolic enzyme and concentration of free fatty acid)

The correlation between the expression level of the fatty acid metabolic enzyme in the tissue and the free fatty acid concentration in the blood serum was analyzed.

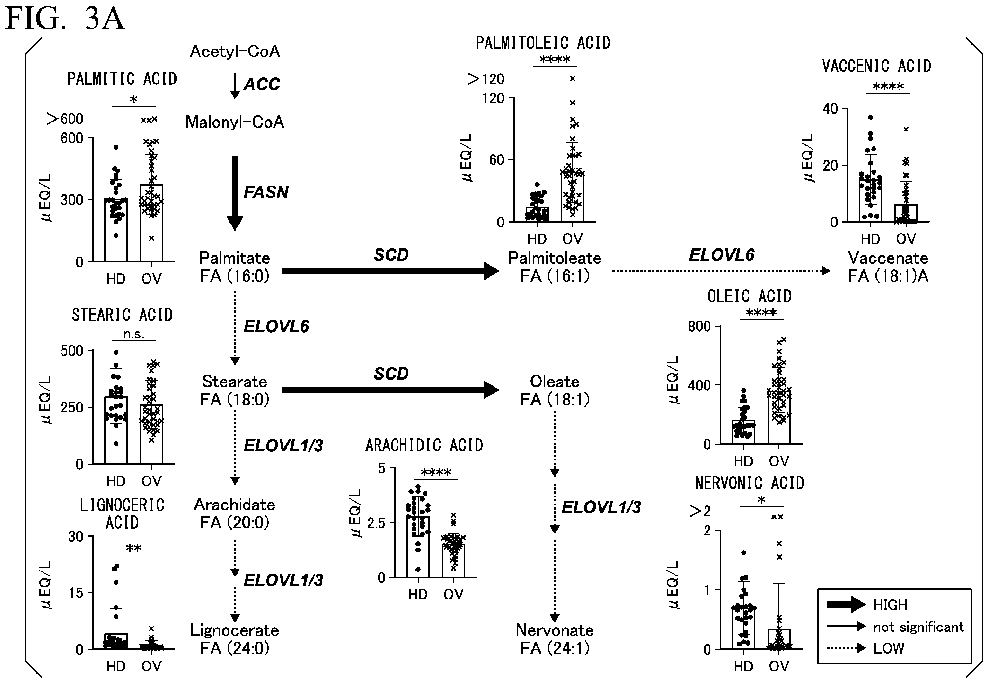

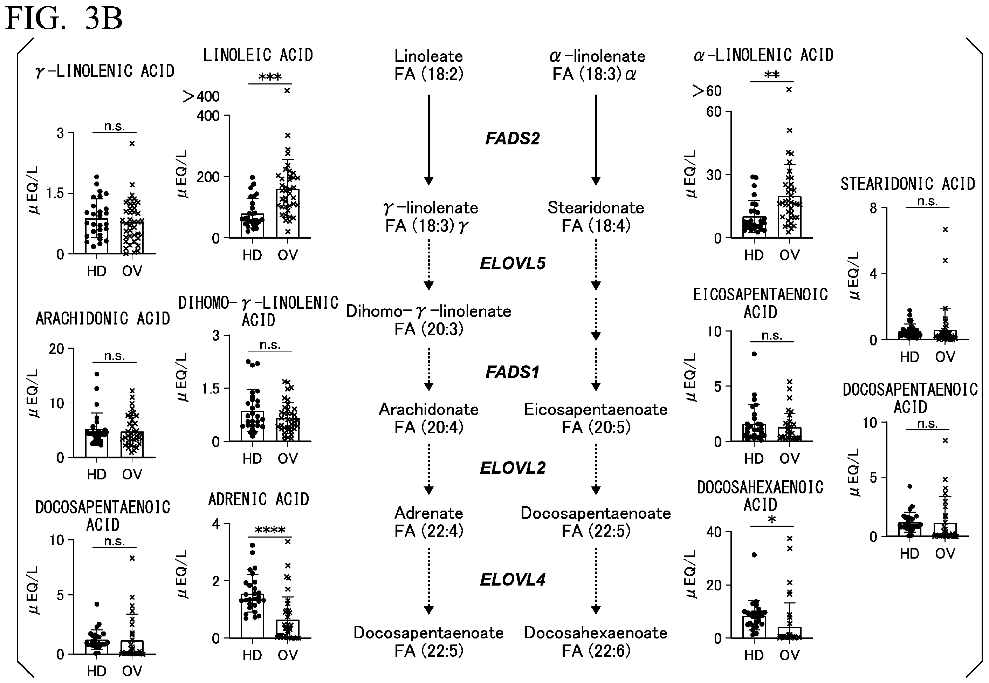

FIGS. 3A and 3B are diagrams showing a correlation between a change in expression of the fatty acid metabolic enzyme in the tissues shown in FIGS. 1B to 1D and a change in the free fatty acid composition in the blood serum shown in FIGS. 2B and 2C. "HIGH" is a metabolic enzyme in which the expression in the ovarian cancer tissue has been significantly higher than that in the normal ovarian tissue in FIGS. 1B to 1D. "LOW" is a metabolic enzyme in which the expression in the ovarian cancer tissue has been significantly lower than that in the normal ovarian tissue in FIGS. 1B to 1D. "not significant" is a metabolic enzyme in which no significant difference in expression is confirmed between the normal ovarian tissue and the ovarian cancer tissue in FIGS. 1B to 1D.

Samples of ovarian cancer tissue and blood serum were collected in pairs from 20 ovarian cancer patients at the same time. In this pair sample, a correlation between the expression level of SCD1 in the ovarian cancer tissue and the amount ratio of the free fatty acid, which is a substrate of SCD1, and the free fatty acid generated by the action of SCD1 was analyzed.

SCD1 acts on palmitic acid, which is a substrate, to produce palmitoleic acid. In addition, SCD1 acts on stearic acid to produce oleic acid.

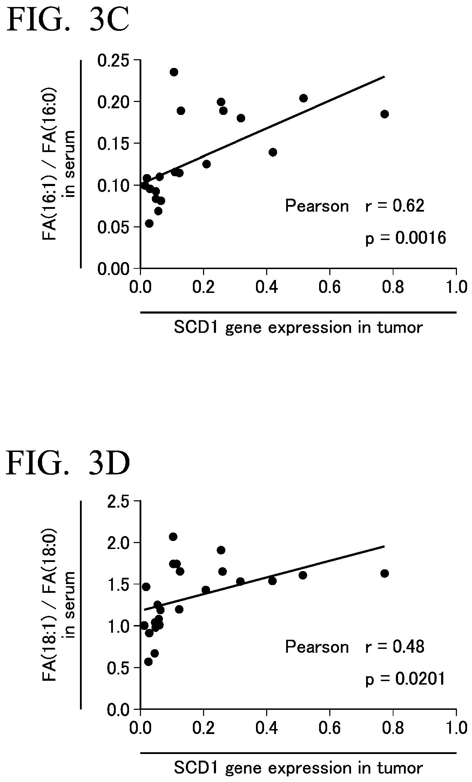

FIG. 3C shows a graph showing a correlation between the expression level of the SCD1 gene in the ovarian cancer tissue and the free fatty acid ratio (ratio of produced free fatty acid/substrate free fatty acid (ratio of palmitoleic acid/palmitic acid)) corresponding to the SCD1 activity in the blood serum of the ovarian cancer patient. FIG. 3D shows a graph showing a correlation between the expression level of the SCD1 gene in the ovarian cancer tissue and the free fatty acid ratio (ratio of produced free fatty acid/substrate free fatty acid (ratio of oleic acid/stearic acid)) corresponding to the SCD1 activity in the blood serum of the ovarian cancer patient. The correlation analysis was performed by Pearson's correlation test using GraphPad Software version 9.0, and the r value and the p value were calculated.

From the results shown in FIGS. 3A to 3D, it was shown that the change in expression of the fatty acid metabolic enzyme in the tissue and the change in concentration of the free fatty acid in the blood serum were linked to each other.

### [Experimental Example 4]

### (Detection of ovarian cancer patients by ROC analysis based on free fatty acid concentration)

In Experimental Example 2, Receiver operating characteristic analysis (ROC analysis) was performed using eleven types of free fatty acids confirmed to have a significant difference in concentrations in the blood serum between healthy female subjects and ovarian cancer patients, as indicators.

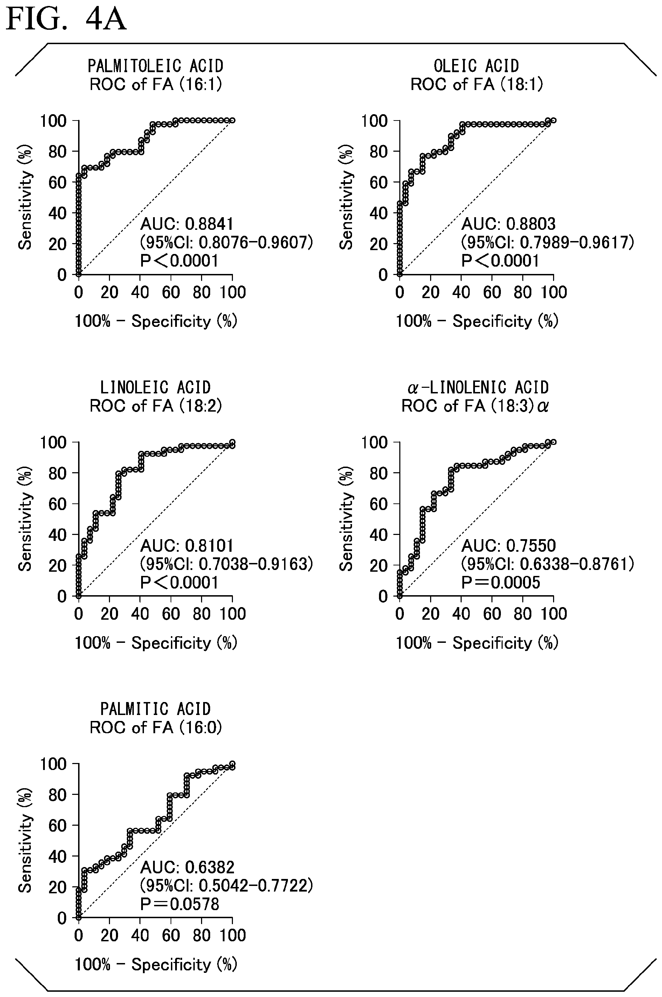

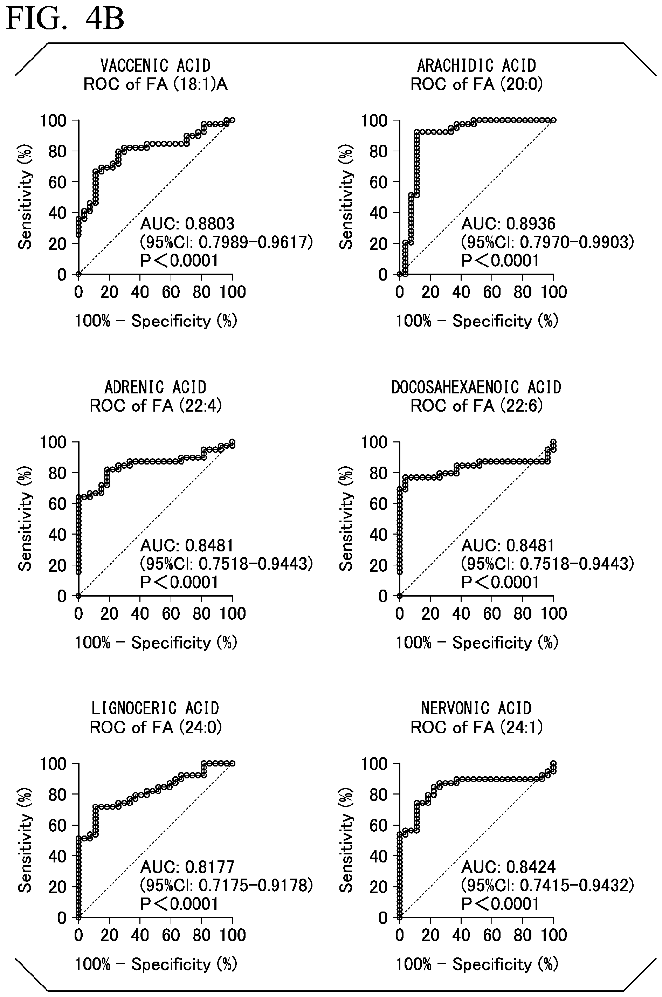

ROC analysis was performed on healthy subjects and ovarian cancer patients using the concentration of each free fatty acid. FIG. 4A shows the results of the ROC analysis using the concentrations of palmitoleic acid, palmitic acid, linoleic acid, α-linolenic acid, and oleic acid. FIG. 4B shows the results of the ROC analysis using the concentrations of vaccenic acid, arachidic acid, docosahexaenoic acid, adrenic acid, nervonic acid, and lignoceric acid. In FIGS. 4A and 4B, AUC and CI mean Area Under Curve and Confidence Interval.

It was revealed that whether the subject of the test is a healthy subject or an ovarian cancer patient can be distinguished based on the concentrations of 10 types of free fatty acids of palmitoleic acid, linoleic acid, α-linolenic acid, oleic acid, vaccenic acid, arachidic acid, docosahexaenoic acid, adrenic acid, nervonic acid, and lignoceric acid among these 11 types.

### [Experimental Example 5]

### (Detection of ovarian cancer patient by ROC analysis with FFA score)

For the ten types of free fatty acids having good results in the ROC analysis of Experimental Example 4, the threshold value was set such that the positive likelihood ratio of the ROC analysis in Experimental Example 4 was maximized. The threshold values of each free fatty acid were 29.06 µEQ/L for palmitoleic acid, 326.0 µEQ/L for oleic acid, 183.1 µEQ/L for linoleic acid, 28.62 µEQ/L for α-linolenic acid, 1.926 µEQ/L for vaccenic acid, 1.924 µEQ/L for arachidic acid, 0.7085 µEQ/L for adrenic acid, 1.847 µEQ/L for docosahexaenoic acid, 0.5773 µEQ/L for lignoceric acid, and 0.0891 µEQ/L for nervonic acid.

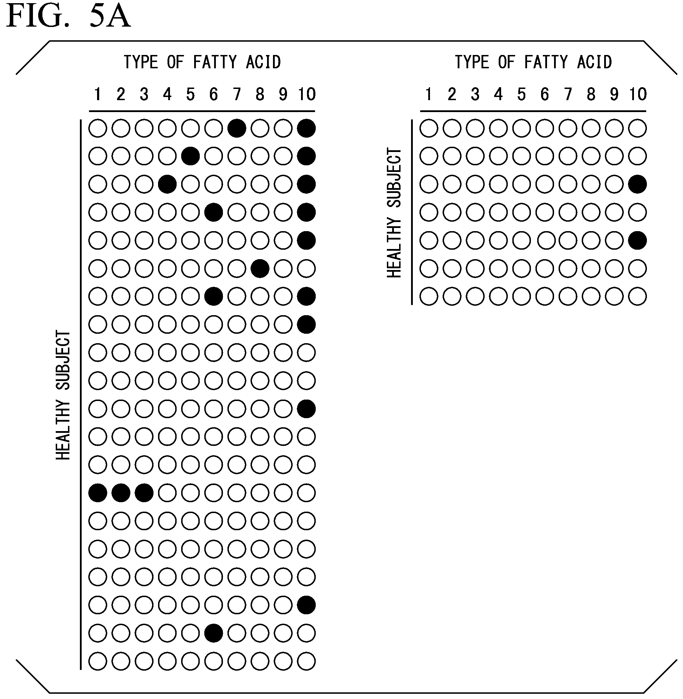

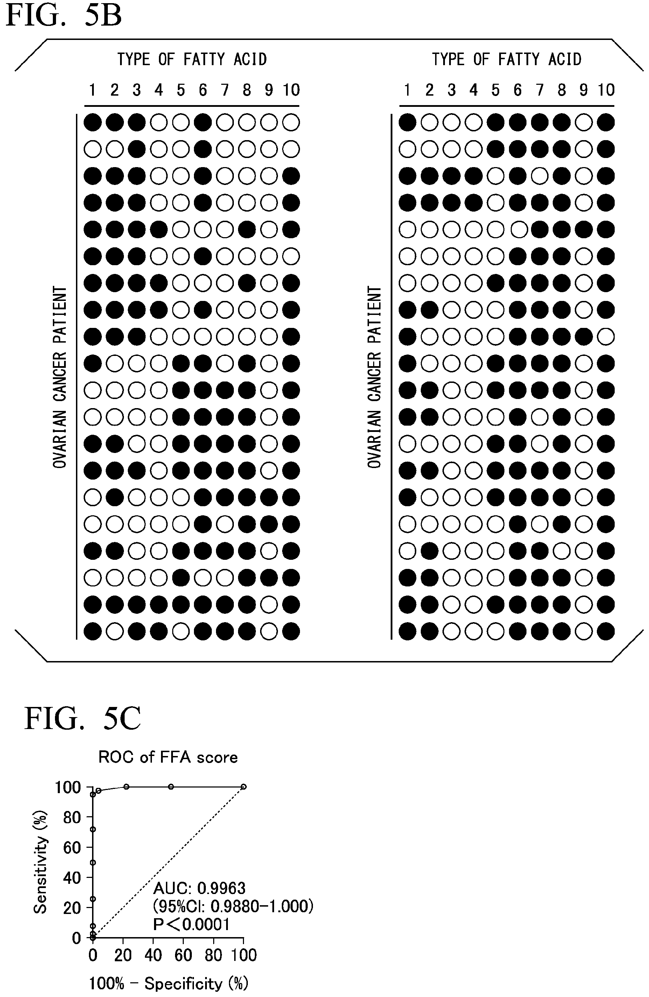

For the test subjects analyzed in Experimental Example 2, whether or not the concentration of each free fatty acid in the blood serum is an abnormal value was determined based on the threshold value of each of the ten types of free fatty acids set as above. FIG. 5A shows the results in healthy subjects. FIG. 5B shows the results in ovarian cancer patients. In FIGS. 5A and 5B, "1" indicates palmitoleic acid, "2" indicates oleic acid, "3" indicates linoleic acid, "4" indicates α-linolenic acid, "5" indicates vaccenic acid, "6" indicates arachidic acid, "7" indicates adrenic acid, "8" indicates docosahexaenoic acid, "9" indicates lignoceric acid, and "10" indicates nervonic acid. In a case where the measured concentration was higher than the threshold value, the free fatty acids of "1" to "4" each were determined in that the concentration was an abnormal value and were indicated by a black circle, and in a case where the measured concentration was lower than the threshold value, it was determined that the concentration was a normal value and was indicated by a white circle. In a case where the measured concentration was lower than the threshold value, the free fatty acids of "5" to "10" each were determined in that the concentration was an abnormal value and were indicated by a black circle, and in a case where the measured concentration was higher than the threshold value, it was determined that the concentration was a normal value and was indicated by a white circle.

From the determination results shown in FIGS. 5A and 5B, the number of black circles in each test subject was calculated as the FFA score of each subject. Using this FFA score, ROC analysis was performed on healthy female subjects and ovarian cancer patients. The results of the ROC analysis are shown in FIG. 5C. As a result of the ROC analysis, the AUC value was 0.9963. The CI value was 0.9880 to 1.000. The p-value was P < 0.0001. From this result, it was confirmed that the ovarian cancer patient can be detected with high sensitivity by using the FFA score.

### [Experimental Example 6]

### (Free fatty acid concentration in blood serum of early ovarian cancer patient)

The concentrations in the blood serum, of ten types of free fatty acids with good results of the ROC analysis in Experimental Example 4 were compared for 27 healthy female subjects, 29 ovarian cancer patients with Stage I or Stage II, and 11 ovarian cancer patients with Stage III or Stage IV.

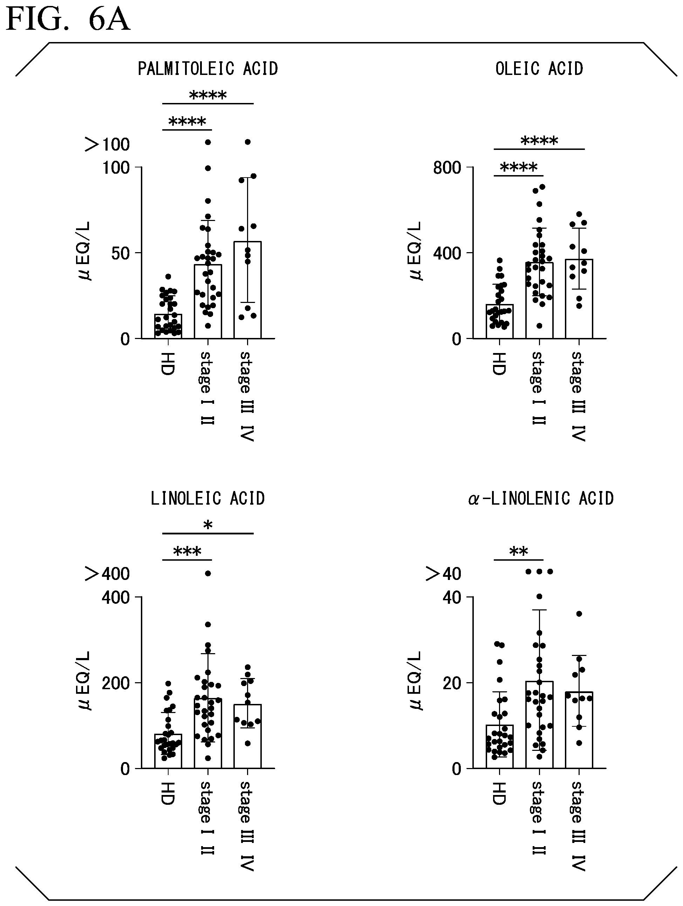

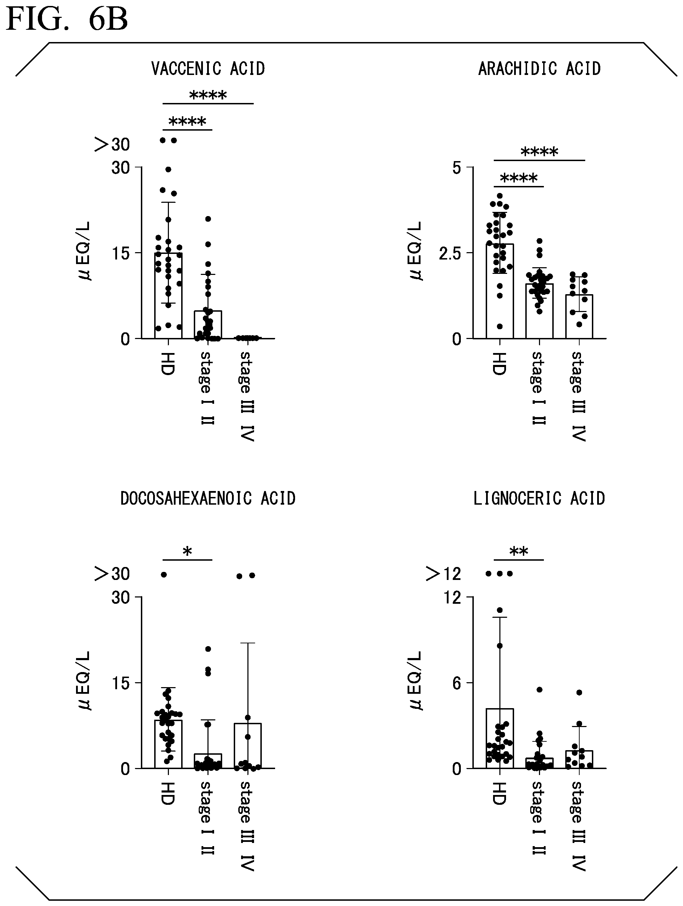

The results of the comparison between the three groups are shown in FIG. 6A and FIG. 6B. In the figures, "HD" means a healthy female subject. The comparison between the three groups was performed by one-way ANOVA and Bonferroni's multiple comparisons tests, and p < 0.05 was considered as a significant difference. "*", "**", "***", and "****" indicate p < 0.05, p < 0.01, p < 0.001, and p < 0.0001.

From the results shown in FIG. 6A, it was confirmed that the concentrations of four types of free fatty acids, palmitoleic acid, linoleic acid, α-linolenic acid, and oleic acid, in the blood serum of ovarian cancer patients with Stage I or Stage II were significantly higher than those in the blood serum of healthy subjects.

From the results shown in FIG. 6B, it was confirmed that the concentrations of four types of free fatty acids, vaccenic acid, arachidic acid, docosahexaenoic acid, and lignoceric acid, in the blood serum of ovarian cancer patients with Stage I or Stage II were significantly lower than those in the blood serum of healthy subjects.

It was confirmed that the concentrations of the eight types of free fatty acids in the blood serum could be an indicator for distinguishing between healthy subjects and ovarian cancer patients with Stage I or Stage II.

It is noted that in the ovarian cancer patients with Stage III or Stage IV, there was no significant difference in the concentrations of α-linolenic acid, docosahexaenoic acid, and lignoceric acid in the blood serum between the healthy subjects.

### [Experimental Example 7]

### (Detection of early ovarian cancer patients by ROC analysis based on free fatty acid concentration)

In Experimental Example 6, Receiver operating characteristic analysis (ROC analysis) was performed using the concentrations in the blood serum, of the eight types of free fatty acids confirmed to have a significant difference in concentrations in the blood serum between healthy female subjects and ovarian cancer patients with Stage I or Stage II, as indicators.

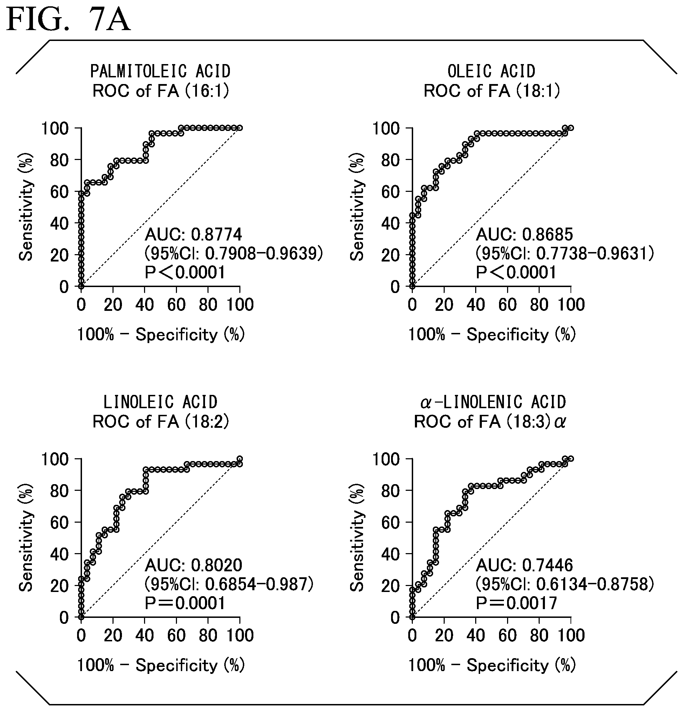

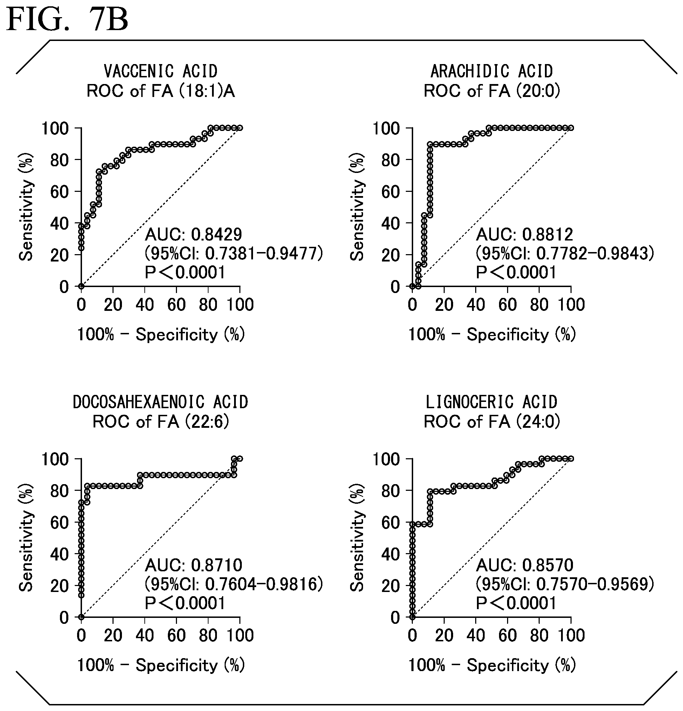

ROC analysis was performed on healthy subjects and ovarian cancer patients with Stage I or Stage II using the concentration of each free fatty acid. FIG. 7A shows the results of the ROC analysis using the concentrations of palmitoleic acid, linoleic acid, α-linolenic acid, and oleic acid. FIG. 7B shows the results of the ROC analysis using the concentrations of vaccenic acid, arachidic acid, docosahexaenoic acid, and lignoceric acid. In FIGS. 7A and 7B, AUC and CI mean Area Under Curve and Confidence Interval.

It was revealed that it is possible to distinguish whether the subject of the test is a healthy subject or an ovarian cancer patient of Stage I or Stage II based on the concentrations of these eight types of free fatty acids.

### [Experimental Example 8]

### (Detection of early ovarian cancer patient by ROC analysis with FFA score)

For the eight types of free fatty acids subjected to the ROC analysis in Experimental Example 7, the threshold value was set such that the positive likelihood ratio of the ROC analysis in Experimental Example 7 was maximized. The threshold values of each free fatty acid were 29.06 µEQ/L for palmitoleic acid, 326.0 µEQ/L for oleic acid, 183.1 µEQ/L for linoleic acid, 28.62 µEQ/L for α-linolenic acid, 1.930 µEQ/L for vaccenic acid, 1.953 µEQ/L for arachidic acid, 1.847 µEQ/L for docosahexaenoic acid, and 0.5773 µEQ/L for lignoceric acid.

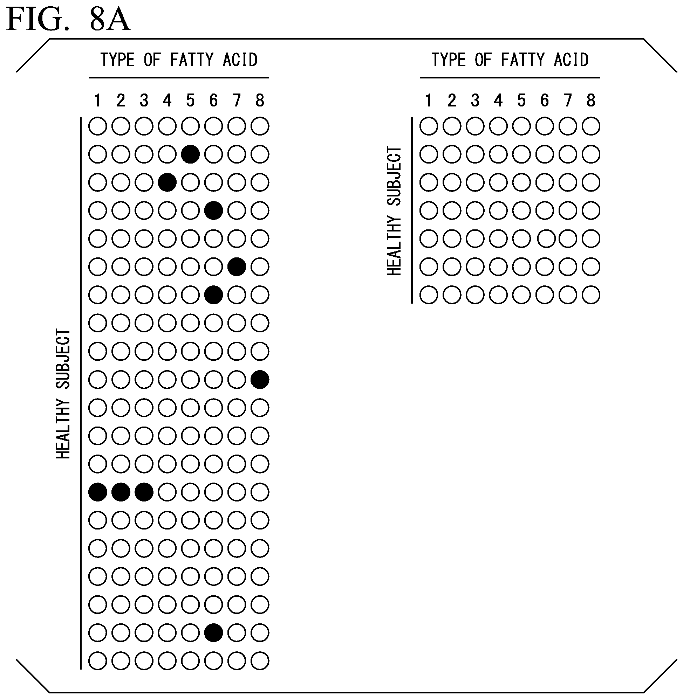

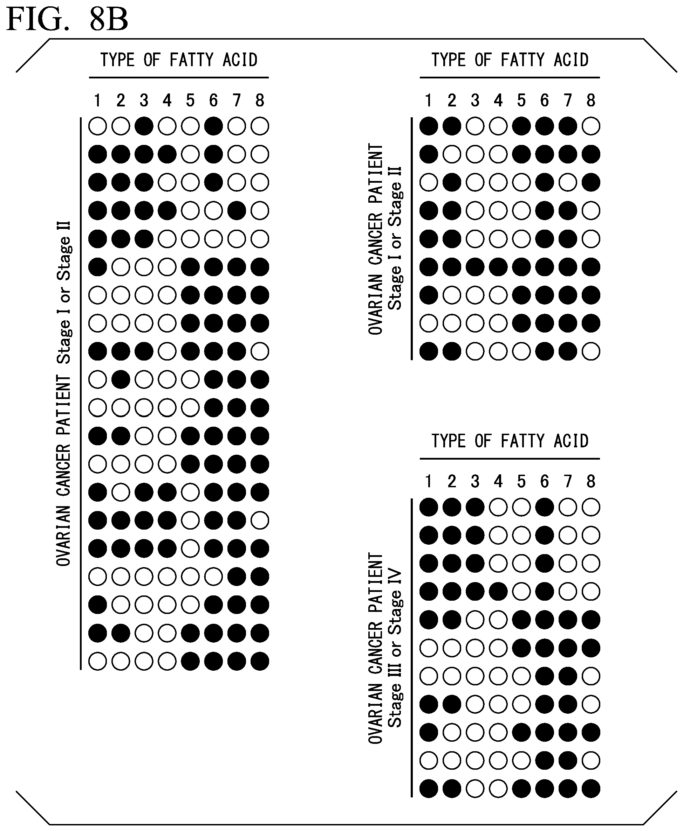

For the test subjects analyzed in Experimental Example 6, whether or not the concentration of each free fatty acid in the blood serum is an abnormal value was determined based on the threshold value of each of the eight types of free fatty acids set as above. FIG. 8A shows the results in healthy subjects. FIG. 8B shows the results in ovarian cancer patients with Stage I or Stage II. In FIGS. 8A and 8B, "1" indicates palmitoleic acid, "2" indicates oleic acid, "3" indicates linoleic acid, "4" indicates α-linolenic acid, "5" indicates vaccenic acid, "6" indicates arachidic acid, "7" indicates docosahexaenoic acid, and "8" indicates lignoceric acid. In a case where the measured concentration was higher than the threshold value, the free fatty acids of "1" to "4" each were determined in that the concentration was an abnormal value and were indicated by a black circle, and in a case where the measured concentration was lower than the threshold value, it was determined that the concentration was a normal value and was indicated by a white circle. In a case where the measured concentration was lower than the threshold value, the free fatty acids of "5" to "8" each were determined in that the concentration was an abnormal value and were indicated by a black circle, and in a case where the measured concentration was higher than the threshold value, it was determined that the concentration was a normal value and was indicated by a white circle.

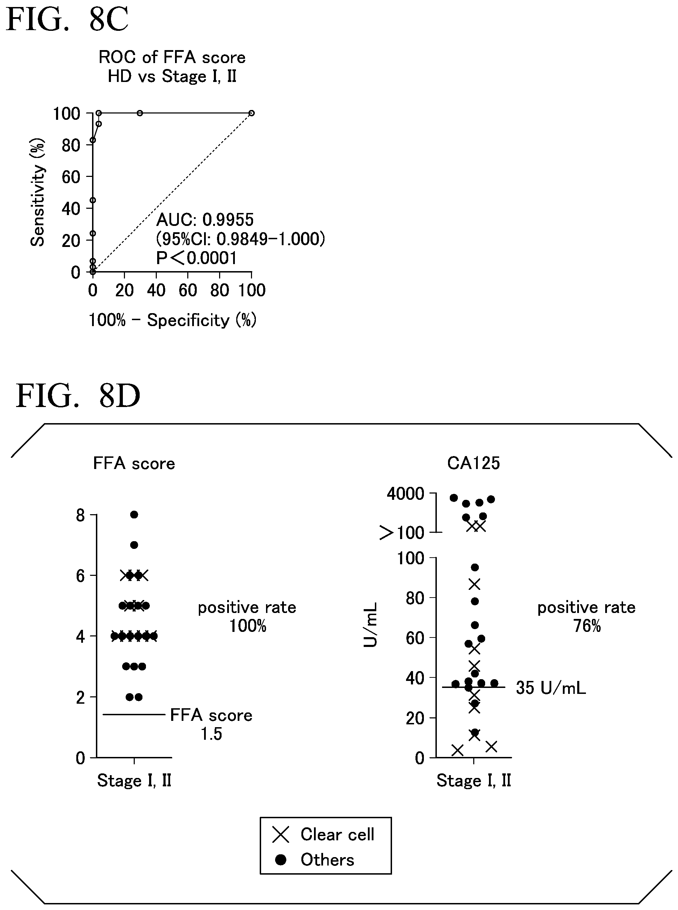

From the determination results shown in FIGS. 8A and 8B, the number of black circles in each test subject was calculated as the FFA score of each subject. Using this FFA score, ROC analysis was performed on healthy female subjects and ovarian cancer patients with Stage I and Stage II. The results are shown in FIG. 8C. As a result of the ROC analysis, the AUC value was 0.9955. The 95% CI value was 0.9849 to 1.000. The p-value was P < 0.0001. From this result, it was confirmed that the ovarian cancer patient with Stage I and Stage II can be detected with high sensitivity by using the FFA score.

The threshold value of the FFA score was set such that the positive likelihood ratio of the ROC analysis in FIG. 8C was maximized. The threshold value of the FFA score was 1.5.

Using the threshold value of the FFA score, for the ovarian cancer of Stage I or II, the detection sensitivity based on the FFA score was compared with the detection sensitivity based on the blood concentration of CA125. FIG. 8D shows the FFA score (left figure) and the blood concentration of CA125 (right figure) in ovarian cancer patients with Stage I or Stage II. In FIG. 8D, "×" indicates clear cell type ovarian cancer, and "•" indicates ovarian cancer other than clear cell cancer type.

From the analysis result of FIG. 8D, in a case where the threshold value of the FFA score was set to 1.5, the detection sensitivity of the ovarian cancer patient with Stage I or Stage II was 100%. In a case where the threshold value of the blood concentration of CA125 is set to 35 U/mL used in actual clinical practice, the detection sensitivity of Stage I or Stage II ovarian cancer patients is 76%. From these results, it was confirmed that, by using the FFA score calculated from the values of the eight types of free fatty acids, it is possible to detect with higher sensitivity, the ovarian cancer patients with Stage I or Stage II than by performing the test using the blood concentration of CA125. Furthermore, it was confirmed that based on the FFA score, an ovarian cancer of a clear cell type, which is a tissue type that is difficult to detect by CA125, can be detected with high sensitivity.

### [Experimental Example 9]

### (Prediction of prognosis of ovarian cancer patient)

For the 40 ovarian cancer patients, which were to be analyzed in Experimental Example 5, the median value of the FFA scores of these ovarian cancer patients was calculated from the FFA scores calculated in Experimental Example 5. Next, for the 40 ovarian cancer patients, patients having a FFA score higher than the median value were classified into a group of "FFA score high", and patients having a FFA score equal to or lower than the median value were classified into a group of "FFA score low".

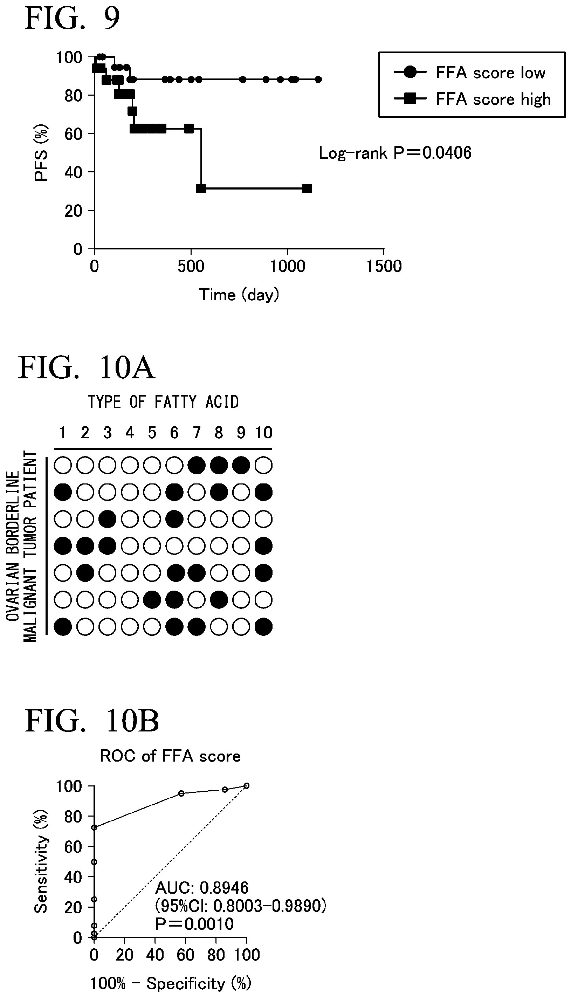

It is noted that the blood serum sample used for calculating the FFA score in Experimental Example 5 was obtained by blood collection before the start of the treatment. The prognosis of each of the patients in the FFA score high group and the patients in the FFA score low group was followed up, and the transition of the progression-free survival rate in each patient group was examined. The results are shown in FIG. 9. In FIG. 9, the X-axis represents the number of days from the start of the treatment, and the Y-axis represents the progression-free survival rate. A Log-rank test was used for the significance difference test.

It was confirmed that the prognosis of the treatment of the ovarian cancer patient can be predicted using the FFA score as an indicator.

### [Experimental Example 10]

### (Detection of ovarian borderline malignant tumor by ROC analysis with FFA score)

Blood was collected from 7 ovarian borderline malignant tumor patients to obtain blood serum samples. Next, the concentration of each free fatty acid in the blood serum was measured by GC-MS in the same manner as in Experimental Example 2. The free fatty acids to be measured were the ten types of free fatty acids analyzed in Experimental Example 5. Next, the FFA score of each ovarian borderline malignant tumor patient was calculated by the same method as in Experimental Example 5.

The threshold value of each free fatty acid used for calculating the FFA score was the same as the threshold value set in Experimental Example 5. That is, the threshold values were 29.06 µEQ/L for palmitoleic acid, 326.0 µEQ/L for oleic acid, 183.1 µEQ/L for linoleic acid, 28.62 µEQ/L for α-linolenic acid, 1.926 µEQ/L for vaccenic acid, 1.924 µEQ/L for arachidic acid, 0.7085 µEQ/L for adrenic acid, 1.847 µEQ/L for docosahexaenoic acid, 0.5773 µEQ/L for lignoceric acid, and 0.0891 µEQ/L for nervonic acid.

FIG. 10A shows a result of determining whether or not the concentration of each free fatty acid in the blood serum is an abnormal value for the borderline malignant tumor patients based on the threshold value.

ROC analysis was performed using the FFA score of the ovarian borderline malignant tumor patient calculated from the result of FIG. 10A and the FFA score of the ovarian cancer patient (ovarian malignant tumor patient) calculated from the result of FIG. 5B. The results are shown in FIG. 10B. As a result of the ROC analysis, the AUC value was 0.8946. The 95% CI value was 0.8003 to 1.000. The p-value was P < 0.0001. From this result, it was confirmed that the ovarian borderline malignant tumor patient and the ovarian cancer patient (ovarian malignant tumor patient) can be distinguished with high sensitivity by using the FFA score.

### [Experimental Example 11]

### (Analysis of endometrial cancer and cervical cancer)

Blood was collected from 27 healthy adult women, 66 endometrial cancer patients, and 45 cervical cancer patients to obtain blood serum samples. Next, the concentration of each free fatty acid in the blood serum was measured by GC-MS in the same manner as in Experimental Example 2.

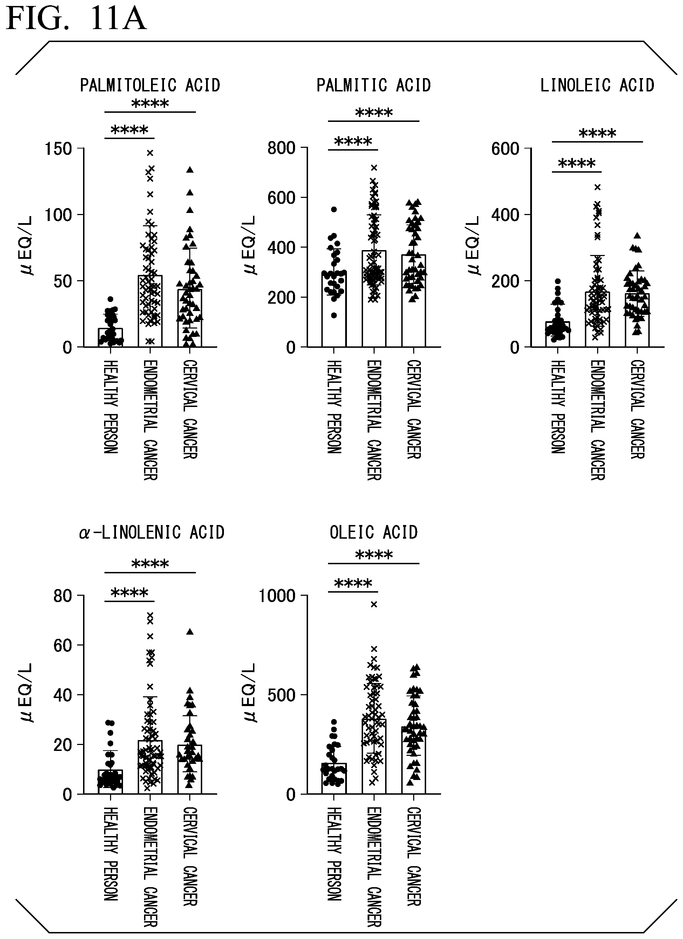

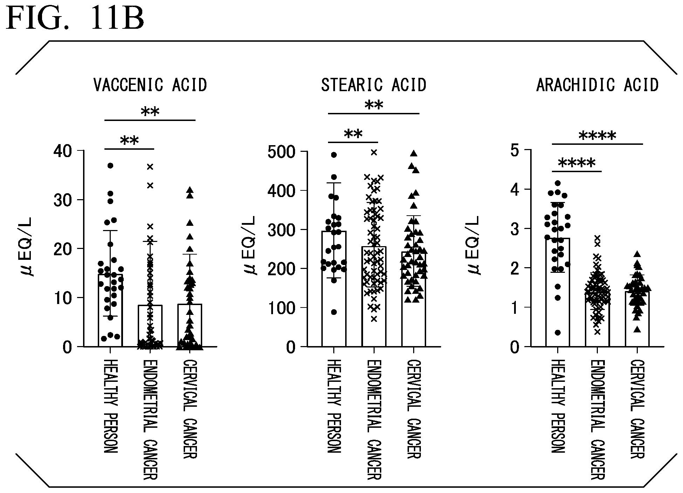

The measurement results are shown in FIGS. 11A and 11B. The comparison between the three groups was performed by one-way ANOVA and Bonferroni's multiple comparisons tests, and p < 0.05 was considered as a significant difference. "**" and "****" each indicate p < 0.01 and p < 0.0001.

It was confirmed that the concentrations of palmitoleic acid, palmitic acid, linoleic acid, α-linolenic acid, and oleic acid in the blood serum of the endometrial cancer patient and the blood serum of the cervical cancer patient were higher than those in the blood serum of the healthy subject. It was confirmed that the concentrations of vaccenic acid, arachidic acid, and stearic acid in the blood serum of the endometrial cancer patient and the blood serum of the cervical cancer patient were lower than those in the serum of the healthy subjects. From these results, it has been revealed that it is possible to distinguish whether the subject of the test is a healthy subject, or an endometrial cancer patient or a cervical cancer patient, using the concentrations of these free fatty acids in the blood serum as an indicator.

### [Experimental Example 12]

### (Free fatty acid concentration in blood serum of early cervical cancer patient)

Blood was collected from 27 healthy adult women, 29 Stage I cervical cancer patients, and 23 Stage II or Stage III cervical cancer patients to obtain blood serum samples. Next, the concentration of each free fatty acid in the blood serum was measured by GC-MS in the same manner as in Experimental Example 2.

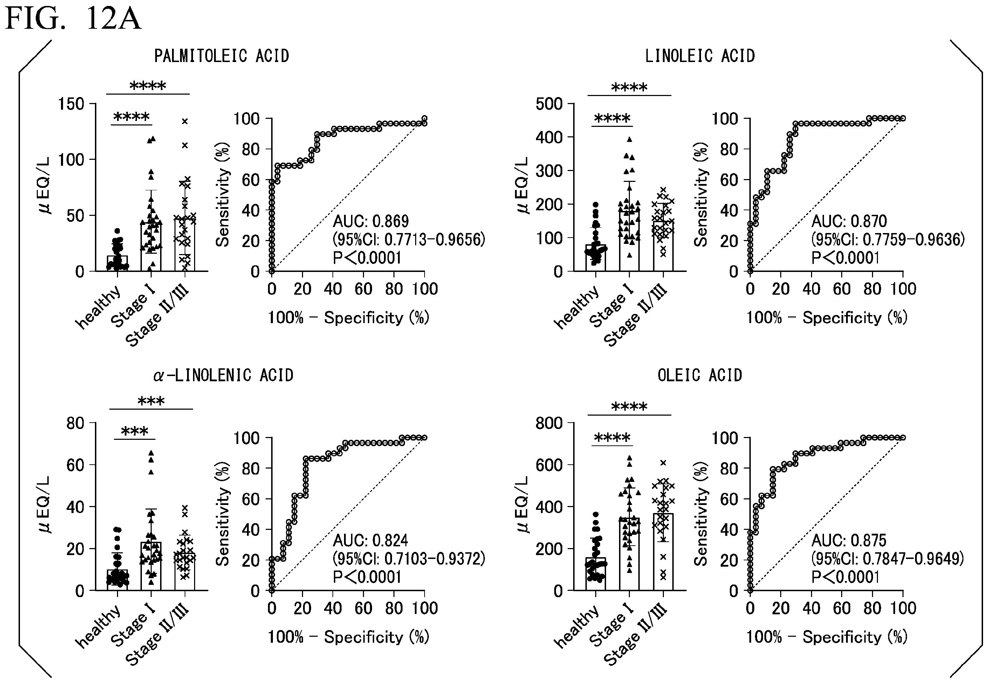

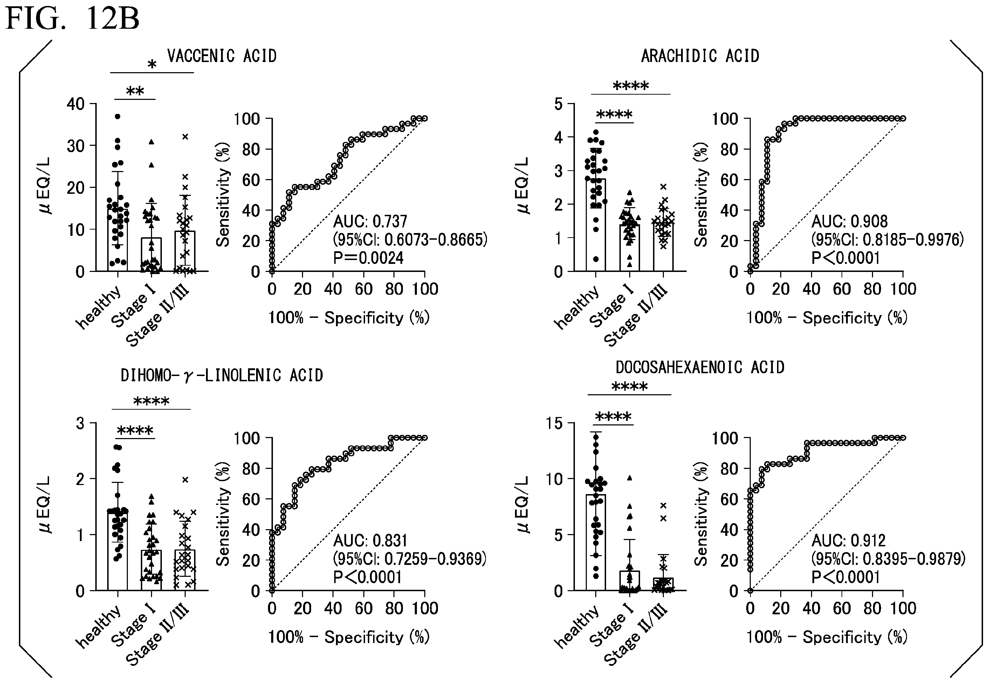

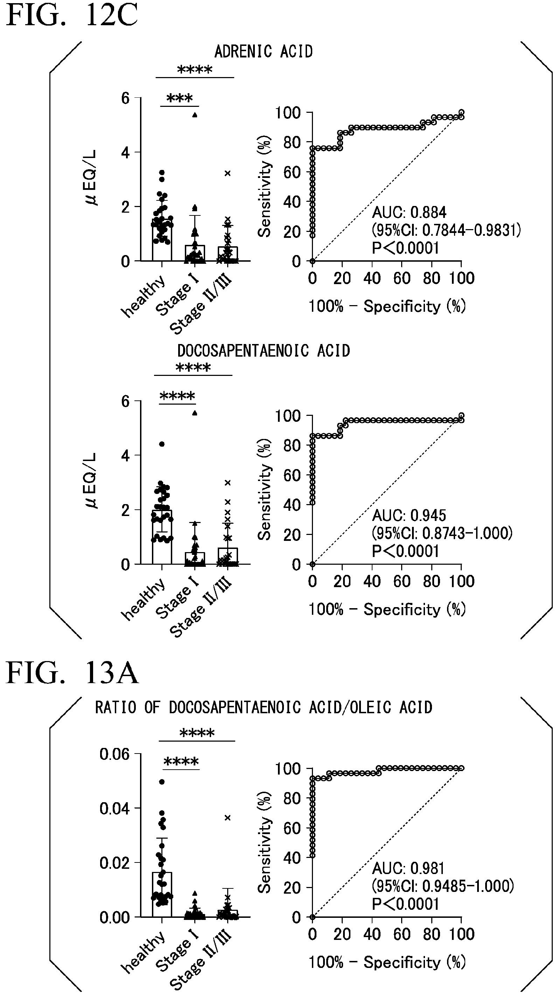

The measurement results are shown in FIGS. 12A and 12C. The comparison between the three groups was performed by one-way ANOVA and Bonferroni's multiple comparisons tests, and p < 0.05 was considered as a significant difference. "*", "**", "***", and "****" indicate p < 0.05, p < 0.01, p < 0.001, and p < 0.0001.

It was confirmed that the concentrations of palmitoleic acid, linoleic acid, α-linolenic acid, and oleic acid, in the blood serum of Stage I cervical cancer patients, or Stage II or Stage III cervical cancer patients were significantly higher than those in the blood serum of healthy subjects. On the other hand, it was confirmed that the concentrations of vaccenic acid, arachidic acid, dihomo-γ-linolenic acid, docosahexaenoic acid, adrenic acid, and docosapentaenoic acid were significantly lower in the blood serum of Stage I cervical cancer patient, or Stage II or Stage III cervical cancer patient than those in the blood serum of healthy subjects.

It was confirmed that the concentrations of these free fatty acids in the blood serum could be an indicator for distinguishing between healthy subjects and Stage I, Stage II, or Stage III cervical cancer patients.

Furthermore, the threshold value of the concentration of each free fatty acid was set to the value shown in Table 2, and the ROC analysis was performed on healthy subjects and Stage I cervical cancer patients using the concentration of each free fatty acid by ROC analysis. The results are shown in FIGS. 12A to 12C. The AUC of any of the free fatty acids was 0.7 or more. It has been revealed that it is possible to distinguish whether the subject of the test is a healthy subject or a Stage I cervical cancer patient, using the concentrations of these free fatty acids in the blood serum as an indicator.

**[Table 2]**

| Free fatty acid | Normal (µEQ/L) | Abnormal (µEQ/L) |
|---|---|---|
| Palmitoleic acid | ≤29.12 | >29.12 |
| Linoleic acid | ≤84.63 | >84.63 |
| α-Linolenic acid | ≤13.13 | >13.13 |
| Oleic acid | ≤251.5 | >251.5 |
| Vaccenic acid | ≥5.690 | <5.690 |
| Arachidic acid | ≥1.875 | <1.875 |
| Dihom-γ-linolenic acid | ≥0.9315 | <0.9315 |
| Docosahexaenoic acid | ≥2.670 | <2.670 |
| Adrenic acid | ≥0.6688 | <0.6688 |
| Docosapentaenoic acid | ≥0.7814 | <0.7814 |
| | | |

| Concentration ratio | Normal | Abnormal |
|---|---|---|
| Docosapentaenoic acid/Oleic acid | ≥0.004433 | <0.004433 |

### [Experimental Example 13]

### (Analysis of ratio of free fatty acid concentrations in blood serum of early cervical cancer patient)

It was verified whether or not it is possible to distinguish whether the subject of the test is a healthy subject or a cervical cancer patient using the concentration ratios of the ten types of free fatty acids as an indicator.

For the blood serum sample of Experimental Example 12, the ratio of docosapentaenoic acid to oleic acid (docosapentaenoic acid/oleic acid) was calculated. The calculation results are shown in FIG. 13A. The comparison between the three groups was performed by one-way ANOVA and Bonferroni's multiple comparisons tests, and p < 0.05 was considered as a significant difference. "*", "**", "***", and "****" indicate p < 0.05, p < 0.01, p < 0.001, and p < 0.0001.

It was confirmed that the ratio (docosapentaenoic acid/oleic acid) in the blood serum of the Stage I cervical cancer patient or the Stage II or Stage III cervical cancer patient was significantly lower than that in the blood serum of the healthy subject.

It was confirmed that the ratio (docosapentaenoic acid/oleic acid) could be an indicator for distinguishing between healthy subjects and Stage I, Stage II or Stage III cervical cancer patients.

Furthermore, the threshold value of the ratio (docosapentaenoic acid/oleic acid) was set to the value shown in Table 2, and the ROC analysis was performed on healthy subjects and Stage I cervical cancer patients using the ratio (docosapentaenoic acid/oleic acid) by ROC analysis. The results are shown in FIG. 13A. The ROC analysis result was AUC = 0.981 and P < 0.0001. That is, it was confirmed that, by using the ratio (docosapentaenoic acid/oleic acid) as an indicator, it is possible to distinguish whether the subject of the test is a healthy subject or a Stage I cervical cancer patient with higher accuracy than by using any one of docosapentaenoic acid or oleic acid as an indicator.

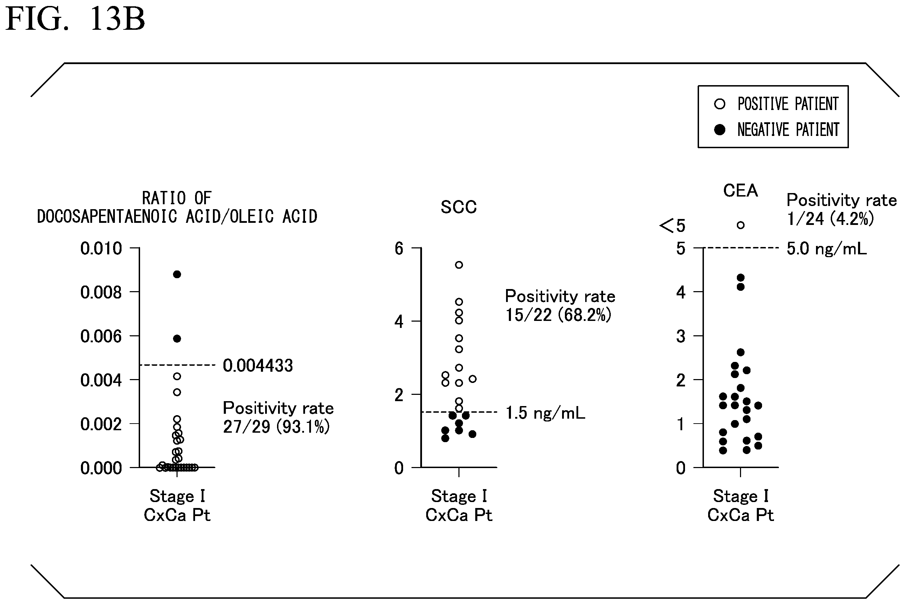

Next, the detection sensitivity by the ratio (docosapentaenoic acid/oleic acid) was calculated for 29 Stage I cervical cancer patients as the subject of the test in Experimental Example 12, and compared with the detection sensitivity by the existing diagnostic marker. The results are shown in FIG. 13B.

In a case where the threshold value of the ratio (docosapentaenoic acid/oleic acid) was set to 0.004433, the detection sensitivity of the Stage I cervical cancer patient was 93.1%. In a case where the threshold value of the blood concentration of SCC was set to 1.5 ng/mL used in actual clinical practice, the detection sensitivity of Stage I cervical cancer patients was 68.2%. In a case where the threshold value of the blood concentration of CEA was set to 5.0 ng/mL used in actual clinical practice, the detection sensitivity of Stage I cervical cancer patients was 4.2%.

From these results, it was confirmed that, by using the ratio (docosapentaenoic acid/oleic acid), it is possible to detect Stage I cervical cancer patients with higher sensitivity than by performing the test using the blood concentration of SCC or CEA, which is an existing diagnostic marker.

### [Experimental Example 14]

### (Analysis of patients with cervical precancerous lesion)

Blood was collected from 27 healthy adult women and 17 cervical precancerous lesion (cervical intraepithelial neoplasia: CIN) patients to obtain blood serum samples. Next, the concentration of each free fatty acid in the blood serum was measured by GC-MS in the same manner as in Experimental Example 2.

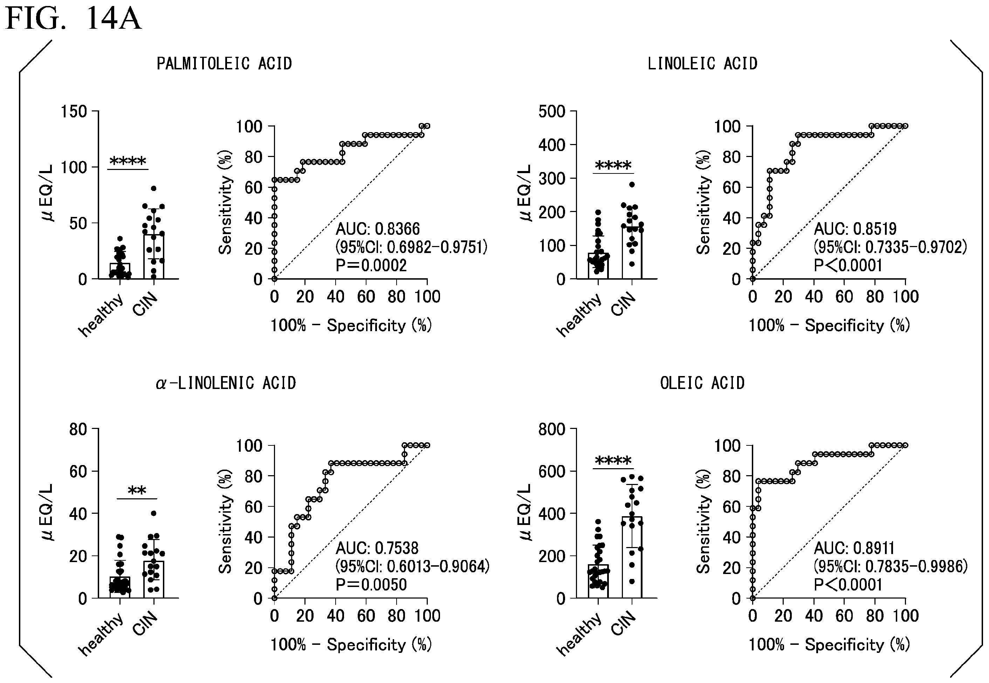

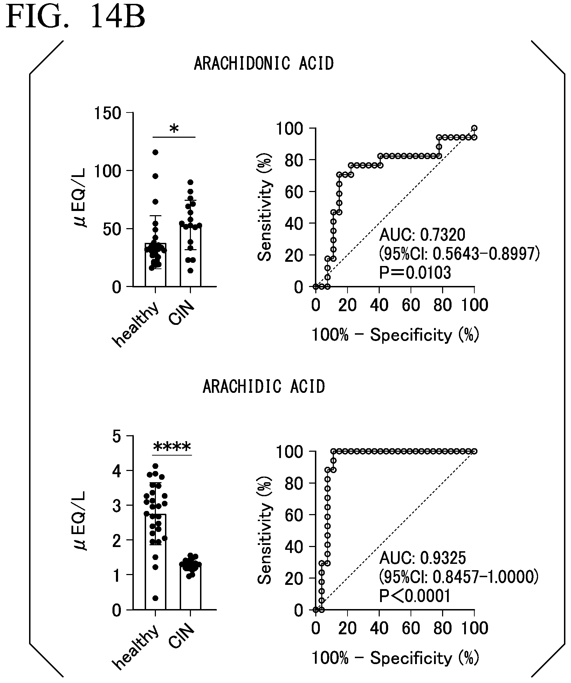

The measurement results are shown in FIGS. 14A and 14B. The comparison between the three groups was performed by one-way ANOVA and Bonferroni's multiple comparisons tests, and p < 0.05 was considered as a significant difference. "*", "**", "***", and "****" indicate p < 0.05, p < 0.01, p < 0.001, and p < 0.0001.

It was confirmed that the concentrations of palmitoleic acid, linoleic acid, α-linolenic acid, oleic acid, and arachidonic acid in the blood serum of the CIN patient were significantly higher than those in the blood serum of the healthy subject. On the other hand, it was confirmed that the concentration of the arachidic acid in the blood serum of the CIN patient was significantly lower than that in the blood serum of the healthy subject.

It was confirmed that the concentrations of these free fatty acids in the blood serum could be an indicator for distinguishing between healthy subjects and CIN patients.

Furthermore, the threshold value of the concentration of each free fatty acid was set to the value shown in Table 3, and the ROC analysis was performed on healthy subjects and CIN patients using the concentration of each free fatty acid by ROC analysis. The results are shown in FIGS. 14A and 14B. The AUC of any of the free fatty acids was 0.7 or more. It has been revealed that it is possible to distinguish whether the subject of the test is a healthy subject or a CIN patient, using the concentrations of these free fatty acids in the blood serum as an indicator.

**[Table 3]**

| Free fatty acid | Normal (µEQ/L) | Abnormal (µEQ/L) |
|---|---|---|
| Palmitoleic acid | ≤36.63 | >36.63 |
| Linoleic acid | ≤83.34 | >83.34 |
| α-Linolenic acid | ≤8.334 | >8.334 |
| Oleic acid | ≤333.4 | >333.4 |
| Arachidonic acid | ≤6.655 | >6.655 |
| Arachidic acid | ≥1.781 | <1.781 |

### [Experimental Example 15]

### (Analysis for CIN patients and cervical cancer patients)

It was verified that it is possible to distinguish whether the subject of the test is a CIN patient or a cervical cancer patient for the blood serum samples of 17 CIN patients collected in Experimental Example 14 and the blood serum samples of 52 cervical cancer patients collected in Experimental Example 12.

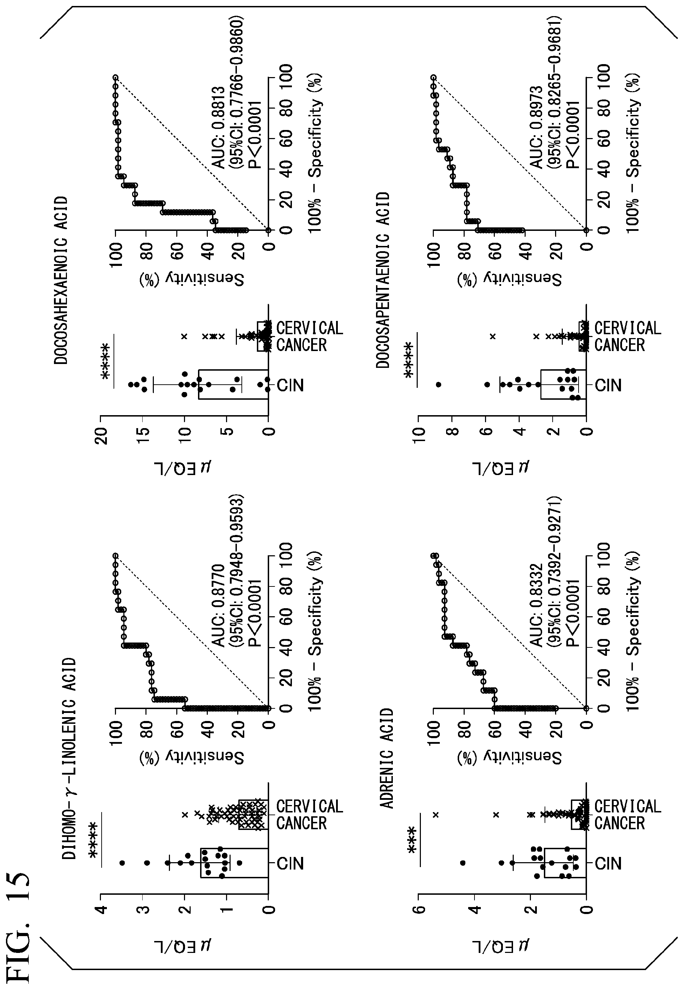

The analysis results are shown in FIG. 15. The comparison between the three groups was performed by one-way ANOVA and Bonferroni's multiple comparisons tests, and p < 0.05 was considered as a significant difference. "***" and "****" each indicate p < 0.001 and p < 0.0001.

It was confirmed that the concentrations of dihomo-γ-linolenic acid, docosahexaenoic acid, adrenic acid, and docosapentaenoic acid in the blood serum of the cervical cancer patient were significantly lower than those in the blood serum of the CIN patient.

It was confirmed that the concentrations of these free fatty acids in the blood serum could be an indicator for distinguishing between CIN patients or cervical cancer patients.

Furthermore, the threshold value of the concentration of each free fatty acid was set to the value shown in Table 4, and the ROC analysis was performed on CIN patients and cervical cancer patients using the concentration of each free fatty acid by ROC analysis. The results are shown in FIG. 15. The AUC of any of the free fatty acids was 0.7 or more. It has been revealed that it is possible to distinguish whether the subject of the test is a CIN patient or a cervical cancer patient, using the concentrations of these free fatty acids in the blood serum as an indicator.

**[Table 4]**

| Free fatty acid | Normal (µEQ/L) | Abnormal (µEQ/L) |
|---|---|---|
| Dihom-γ-linolenic acid | ≥1.012 | <1.012 |
| Docosahexaenoic acid | ≥3.507 | <3.507 |
| Adrenic acid | ≥0.312 | <0.312 |
| Docosapentaenoic acid | ≥0.7231 | <0.7231 |

### INDUSTRIAL APPLICABILITY

According to the present invention, it is possible to provide a simple and highly accurate test method for detecting one or more types selected from the group consisting of gynecological cancer and a precancerous lesion thereof, predicting a prognosis of gynecological cancer, and classifying gynecological malignant tumors and gynecological borderline malignant tumors, a medicine for treating a subject determined to have the gynecological cancer by the test method, and a kit. The present invention is useful for early diagnosis of a gynecological cancer or a precancerous lesion thereof.

## Claims

1. A sample test method comprising:
a step (A1) of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid, in a sample derived from a subject; and
a step (B1) of evaluating a possibility that the subject has one or more selected from the group consisting of a gynecological cancer and a precancerous lesion of the gynecological cancer, based on the concentration of the free fatty acids in the sample, which is obtained in the step (A1),
wherein in the step (B 1), the possibility that the subject has one or more selected from the group consisting of a gynecological cancer and a precancerous lesion of the gynecological cancer is evaluated to be high in a case corresponding to at least one selected from the group consisting of the following (i1), (ii1), and (iii1),
(i1) a concentration of at least one free fatty acid selected from the group consisting of palmitoleic acid, linoleic acid, α-linolenic acid, oleic acid, palmitic acid, and arachidonic acid in the sample is high as compared with a predetermined threshold value,
(ii1) a concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value, and
(iii1) a concentration ratio of docosapentaenoic acid to oleic acid is low as compared with a predetermined threshold value.

2. A sample test method comprising:
a step (A2) of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid, in a sample derived from a subject having a gynecological cancer; and
a step (B2) of evaluating a possibility that the subject has poor prognosis based on the concentration of the free fatty acids in the sample, which is obtained in the step (A2),
wherein in the step (B2), the possibility that the subject has poor prognosis is evaluated to be high in a case corresponding to at least one selected from the group consisting of the following (i2) and (ii2),
(i2) a concentration of at least one free fatty acid selected from the group consisting of palmitoleic acid, linoleic acid, α-linolenic acid, oleic acid, and palmitic acid in the sample is high as compared with a predetermined threshold value, and
(ii2) a concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.

3. A sample test method comprising:
a step (A3) of measuring a concentration of one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, and docosapentaenoic acid, in a sample derived from a subject having a gynecological cancer; and
a step (B3) of evaluating whether the gynecological cancer of the subject is a malignant tumor or a borderline malignant tumor based on the concentration of the free fatty acids in the sample, which is obtained in the step (A3),
wherein in the step (B3), a possibility that the gynecological cancer is a malignant tumor is evaluated to be high in a case corresponding to at least one selected from the group consisting of the following (i3) and (ii3),
(i3) a concentration of at least one free fatty acid selected from the group consisting of palmitoleic acid, linoleic acid, α-linolenic acid, oleic acid, and palmitic acid in the sample is high as compared with a predetermined threshold value, and
(ii3) a concentration of at least one free fatty acid selected from the group consisting of vaccenic acid, arachidic acid, docosahexaenoic acid, adrenic acid, nervonic acid, lignoceric acid, stearic acid, dihomo-γ-linolenic acid, and docosapentaenoic acid in the sample is low as compared with a predetermined threshold value.

4. The sample test method according to Claim 1, further comprising:
a step (C1) of evaluating whether the subject determined to have a high possibility of having one or more selected from the group consisting of a gynecological cancer and a gynecological precancerous lesion by the step (B 1) has any of a gynecological cancer or a gynecological precancerous lesion based on the concentration of the free fatty acid in the sample, which is obtained in the step (A1),
wherein in the step (C1), a possibility that the subject has a gynecological cancer is evaluated to be high in a case corresponding to the following (Ci),
(Ci) a concentration of at least one free fatty acid selected from the group consisting of dihomo-γ-linolenic acid, docosahexaenoic acid, adrenic acid, and docosapentaenoic acid in the sample is low as compared with a predetermined threshold value.

5. The sample test method according to any one of Claims 1 to 4,
wherein the gynecological cancer includes at least one selected from the group consisting of ovarian cancer, endometrial cancer, and cervical cancer.

6. The sample test method according to Claim 1,
wherein the gynecological cancer is a Stage I or Stage II gynecological malignant tumor.

7. The sample test method according to Claim 6,
wherein the step (A1) is a step of measuring a concentration of one or more free fatty acids selected from the group consisting of palmitoleic acid, linoleic acid, α-linolenic acid, oleic acid, palmitic acid, vaccenic acid, arachidic acid, and stearic acid, and
the free fatty acid in (i1) of the step (B1) is at least one selected from the group consisting of palmitoleic acid, linoleic acid, α-linolenic acid, oleic acid, and palmitic acid, and the free fatty acid in (ii1) of the step (B 1) is at least one selected from the group consisting of vaccenic acid, arachidic acid, and stearic acid.

8. The sample test method according to any one of Claims 1 to 4,
wherein the sample is a blood sample.

9. A test kit for a gynecological cancer and a precancerous lesion of the gynecological cancer, the test kit comprising:
a reagent for measuring one or more free fatty acids selected from the group consisting of palmitoleic acid, linoleic acid, α-linolenic acid, oleic acid, palmitic acid, vaccenic acid, arachidic acid, docosahexaenoic acid, adrenic acid, nervonic acid, lignoceric acid, stearic acid, dihomo-γ-linolenic acid, docosapentaenoic acid, and arachidonic acid,
wherein the test kit is used for at least one purpose selected from the group consisting of the following (a) to (d),
(a) evaluation of a possibility that a subject has one or more selected from the consisting of a gynecological cancer and a precancerous lesion of the gynecological cancer,
(b) evaluation of a possibility that a subject having a gynecological cancer has poor prognosis,
(c) evaluation of whether a gynecological cancer of a subject is a malignant tumor or a borderline malignant tumor, and
(d) evaluation of whether a subject having a high possibility of having one or more selected from the group consisting of a gynecological cancer and a gynecological precancerous lesion has any of a gynecological cancer and a gynecological precancerous lesion.

10. The test kit for a gynecological cancer according to Claim 9,
wherein the test kit is used in the sample test method according to any one of Claims 1 to 4.

11. A medicine for treating or preventing a gynecological cancer, which is administered to a subject evaluated to have a gynecological cancer by the test method according to Claim 1.
